# EUROPEAN PATENT SPECIFICATION

(11) **EP 0 416 814 B1**
(45) Date of publication and mention of the grant of the patent: **24.04.2002**
(21) Application number: 90309493.6
(22) Date of filing: 30.08.1990
(51) Int. Cl.: C07D 501/36, A61K 31/545, C07D 213/70, C07D 221/04, C07D 213/77, C07D 401/04, C07D 417/12, C07D 401/12

(54) **Antibacterial cephalosporin derivatives**
Antibakterielle Cephalosporin-Derivate
Dérivés de céphalosporine antibactériens

(30) Priority: 04.09.1989 GB 8919945; 04.09.1989 GB 8919946; 08.05.1990 GB 9010265; 08.05.1990 GB 9010299; 26.03.1990 GB 9006728
(43) Date of publication of application: 13.03.1991
(73) Proprietor: PFIZER INC., New York, N.Y. 10017-5755 (US)
(72) Inventor: Branch, Clive Leslie, Betchworth, Surrey RH3 7AJ (GB); Guest, Angela Wendy, Betchworth, Surrey RH3 7AJ (GB); Adams, Richard George, Betchworth, Surrey RH3 7AJ (GB)
(74) Representative: Watkins, David

(56) References cited:
- EP-A- 0 153 709
- EP-A- 0 248 645
- JOURNAL OF HETEROCYCLIC CHEMISTRY, vol. 11, no. 4, August 1974, pages 487-490, HeteroCorporation, Provo, Utah, US; Ibrahim El-Sayed El-Kholy et al.: "Action of amines and carbonyl reagents on 3,5-diphenyl-4-pyrone and its thio analogue"
- BULLETIN OF THE CHEMICAL SOCIETY OF JAPAN, vol. 51, no. 1, January 1978, pages 179-181, The Chemical Society of Japan, Tokyo, JP; H. Besso et al.: "Reactions of 4H-pyran-4-thiones with ammonia, hydrazine, and guanidine"
- JOURNAL OF THE CHEMICAL SOCIETY, Perkin Transactions I, vol. B4, no. 12, 1977, pages 1365-1504, Royal Society of Chemistry, Cambridge, GB; A.S. AFRIDI et al.: "Preparation of NN'-linked Bi(heteroaryls) from dehydroacetic acid and 2,6-dimethyl-4-pyrone"

## Description

This invention relates to novel β-lactam containing compounds, in particular to a novel class of cephalosporin derivatives, their preparation and their use. These compounds have antibacterial properties and are therefore of use in the treatment of bacterial infections in humans and animals caused by a wide range of organisms.

7β-[2-(2-amino-4-thiazolyl)-2-oxyiminoacetamido]-cephalosporin derivatives have been reported, of which a particular subgroup are those comprising at the 3-position a substituent -CH₂S-Het in which 'Het' is an optionally substituted heterocyclic group. EP-A-0 248 645 (Tanabe Seiyaku) discloses inter alia a class of such compounds in which 'Het' is a pyridinium ring (bonded through a ring carbon atom), optionally substituted on the pyridinium nitrogen by acylamino and exemplified by an N-acetyl derivative, in combination with a [2-oxopyrrolidin-3-yl]-oxyimino substituent.

In addition, EP-A-0 153 709 (Meiji Seika Kaisha) discloses generically another class of such compounds wherein 'Het' is a bicyclic heterocyclic system comprising a pyridinium ring (bonded through a ring carbon atom) fused to a carbocyclic ring and which may be substituted on the pyridinium nitrogen by an amino group, optionally substituted by alkyl. No exemplification thereof is however provided.

It has now been found that the range of substituents that can be accommodated on the pyridinium nitrogen may be substantially expanded, to give a class of cephalosporin derivatives that possess high antibacterial activity.

Accordingly, the present invention provides a compound of formula (I) or a salt thereof: 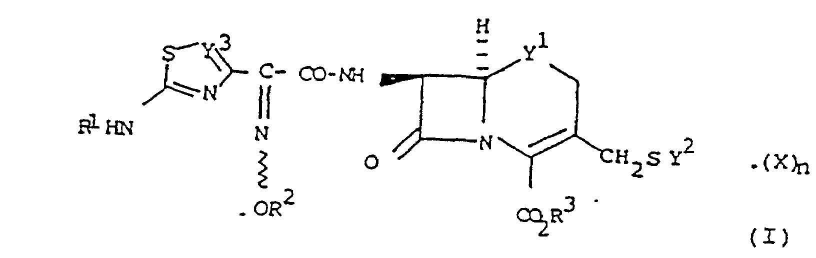 wherein
- Y¹: is sulphur, -SO-, or SO₂-;
- R¹: is hydrogen or an amino protecting group;
- R²: is (C₁-C₁₂)alkyl, (C₂-C₁₂)alkenyl, (C₂-C₁₂)alkynyl, (C₃₋₇)cycloalkyl, (C₅₋₈)cycloalkenyl, each of which is substituted with a phenyl group which may be unsubstituted or substituted up to 3 times with (C₁₋₆)alkoxy or hydroxy; and wherein each of the R² substituents hereinbefore defined is optionally further substituted with carboxyl; or R² is (methylenedioxy)-benzyl;
- CO₂R³: is carboxy or a carboxylate anion, or the group R³ is a readily removable carboxy protecting group;
- Y²: is a group of the formula: 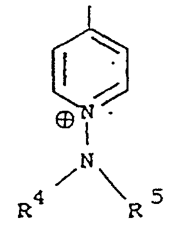 wherein the pyridinium group is unsubstituted or substituted at a ring carbon atom available for substitution by up to four substituents selected from (C₁₋₆)alkyl, and (C₁₋₆)alkoxy;
- R⁴ and R⁵: which may be the same or different are selected from hydrogen, a group R⁶;
- R⁶: is (C₁₋₆)alkyl, (C₃₋₇)cycloalkyl, (C₂₋₆)alkenyl, (C₅₋₈)cycloalkenyl, (C₂₋₆)alkynyl, each of which may be unsubstituted or substituted by halogen, cyano, azido, nitro, carboxy, (C₁₋₆)alkoxycarbonyl, carbamoyl, mono- or di-(C₁₋₆)alkylcarbamoyl, sulphono, sulphamoyl, mono- and di-(C₁₋₆)alkylsuphamoyl, amino, mono- and di-(C₁₋₆)alkylamino, acylamino, (C₁₋₆)alkoxycarbonyl amino, hydroxy, (C₁₋₆)alkoxy, acyloxy, oxo, phenylcarbonyl, naphthylcarbonyl, heterocyclylcarbonyl, (C₁₋₆)alkylthio, (C₁₋₆) alkanesulphinyl, (C₁₋₆) alkanesulphonyl, phenyl, naphthyl or heterocyclyl; wherein the phenyl or naphthyl may be unsubstituted or substituted up to 5 times by halogen, (C₁₋₆)alkyl, phenyl, (C₁₋₆)alkoxy, hydroxy(C₁₋₆)alkyl, mercapto(C₁₋ ₆)alkyl, halo(C₁₋₆)alkyl, mercapto, hydroxy, amino, mono- or di-(C₁₋₆)alkylamino, nitro, carboxy, (C₁₋ ₆) alkoxycarbonyl, (C₁₋₆)alkoxycarbonyl-(C₁₋₆)alkyl, (C₁₋ ₆) alkylcarbonyloxy, formyl, or (C₁₋₆) alkylcarbonyl; wherein the heterocyclyl moiety may be unsubstituted or substituted up to 3 times by (C₁₋₆)alkyl, (C₁₋₆)alkoxy, (C₁₋ ₆)alkoxy(C₁₋₆)alkyl, halo(C₁₋₆)alkyl, hydroxy, amino, mono- or di-(C₁₋₆) alkylamino, carboxy, carbamoyl, (C₁₋₆)alkoxycarbonyl, (C₁₋₆)alkoxycarbonyl(C₁₋₆)alkyl, oxo; and the heterocyclyl moiety is aromatic or non-aromatic, single or bicyclic rings containing up to four heteroatoms in each ring selected from oxygen, nitrogen and sulphur; or R⁶ is Pyridyl; isoxazolylmethyl; thiazolymethyl; chloropyridinyl; pyrazinyl; imidazolinyl; benzopyridinyl; benzoyl; 3,4-dihydroxybenzoyl; 4-nitrobenzoyl; 4-methoxybenzoyl ; 4-carboxybenzoyl; 4-aminobenzoyl; 2-furanoyl; 3,4-dihydroxycinnamoyl; carbamoyl; and N-methylcarbamoyl.
- Y³: is nitrogen or -CH;
- x: is an inorganic or organic anion, and
- n: is o or 1,
with the proviso that:
(i) when CO₂R³ is carboxylate, n is 0, and
(ii) when CO₂R³ is carboxy or the group R³ is a readily removable carboxy protecting group, then n is 1,
and the anion X is present in the appropriate stoichiometric proportion to balance the positive charge on the pyridinium group.

It will be appreciated that the compounds of formula (I) are quaternary salts and the positive charge on the pyridinium group must always be balanced by a counter anion. The counter anion may be present on a negatively charged group within the molecule, such as the carboxylate anion CO₂R³ (when n is O), or the counter anion may be present as an external anion X (when n is 1).

It will be further appreciated that one or more chiral centres may be present in the compound of formula (I), for example in the oxime etherifying group. The invention includes within its scope the individual R and S forms at each chiral centre as well as mixtures thereof.

Suitable (C₁₋₁₂)alkyl groups include straight and branched chain alkyl groups containing 1 to 12 carbon atoms. Preferred alkyl groups contain 1 to 6 carbon atoms, such as methyl, or ethyl.

Suitable (C₂₋₁₂)alkenyl groups include straight and branched chain alkenyl groups containing 2 to 12 carbon atoms. Preferred alkenyl groups contain 2 to 6 carbon atoms, such as propenyl and butenyl.

Suitable (C₂₋₁₂)alkynyl groups include straight and branched chain alkynyl groups containing 2 to 12 carbon atoms. Preferred alkynyl groups contain 2 to 6 carbon atoms such as propynyl and butynyl.

Suitable (C₃₋₇)cycloalkyl groups include cyclopropyl, cyclopentyl, and cyclohexyl.

Suitable (C₅₋₈)cycloalkenyl groups include cyclopentenyl and cyclohexenyl.

When used herein, the term 'halogen' refers to fluorine, chlorine, bromine and iodine.

Suitable amino protecting groups R¹ are those well known in the art and which may be removed under conventional conditions without disruption of the remainder of the molecule. A comprehensive discussion of the ways in which amino groups may be protected and methods for cleaving the resulting protected derivatives is given in, for example, 'Protective Groups in Organic Synthesis' by T. W. Greene (Wiley-Interscience, New York, 1981). Particularly suitable protecting groups include, for example, amides and carbamates.

Examples of such amino protecting groups include (C₁₋₆) alkanoyl; benzoyl; benzyl optionally substituted in the phenyl ring by one or two substituents selected from (C₁₋₄)alkyl, (C₁₋₄)alkoxy, trifluoromethyl, halogen, or nitro; (C₁₋₄)alkoxycarbonyl; benzyloxycarbonyl or trityl substituted as for benzyl above; allyloxycarbonyl, trichloroethoxycarbonyl or chloroacetyl.

Suitable groups for R² include, for example, (C₁₋₆) alkyl or (C₃₋₇) cycloalkyl, each of which is substituted with a phenyl group which may be unsubstituted or substituted up to 3 times with (C₁₋₆) alkoxy or hydroxy.

Preferably the group for R² is methyl, optionally further substituted by carboxy. A suitable R² group is 3,4-dihydroxyphenyl methyl, optionally further substituted by carboxy.

Specific examples of the group R² include carboxy (3,4-dihydroxy-phenyl) methyl and (methylenedioxy) benzyl; of which (carboxy 3,4-dihydroxyphenyl)methyl is particularly preferred.

Specific examples of the groups R⁴ and R⁵ include hydrogen, methyl, ethyl, carboxymethyl, methoxyethyl, cyanomethyl, propargyl, 4-carboxy-butan-1-yl, 2-amino-2-(methoxycarbonyl)ethyl, cyclopropylmethyl, propyl, cyclopentyl, prop-2-en-1-yl, butyl, hexyl, isopropyl, 2-hydroxyethyl, pyridyl, isoxazolylmethyl, thiazolylmethyl, chloropyridinyl, pyrazinyl, imidazolinyl, benzopyrazidinyl, benzoyl, 3,4-dihydroxybenzoyl, 4-nitrobenzoyl, 4-methoxybenzoyl, 4-carboxybenzoyl, 4-aminobenzoyl, 2-furanoyl, 3,4-dihydroxycinnamoyl, carbamoyl and N-methylcarbamoyl.

Included within the scope of readily removable carboxy protecting groups for R³ are, for example, ester groups including pharmaceutically acceptable in vivo hydrolysable ester groups.

Compounds of the invention may exist in two or more tautomeric forms, e.g. those having the partial structures below:

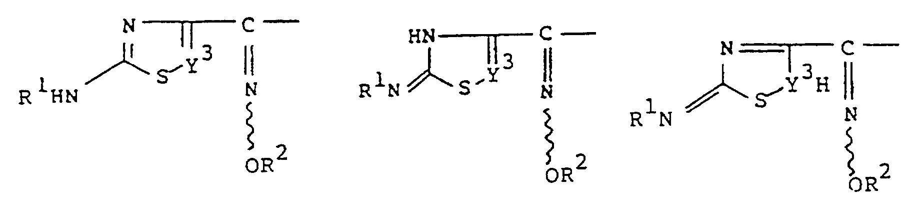

Compounds of the present invention may exist as either syn or anti isomers, or may exist as mixtures of syn and anti isomers containing at least 75% of one such isomer, or preferably at least 90% of one such isomer.

When used herein, the terms syn and anti refer to the configuration of the group OR² with respect to the carboxamido group, the syn-configuration (sometimes called the Z-configuration) being denoted thus: 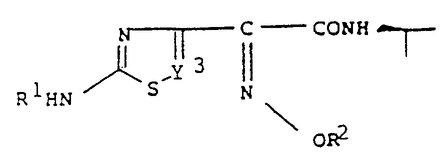 and the anti configuration (sometimes called the E-configuration) being denoted thus:

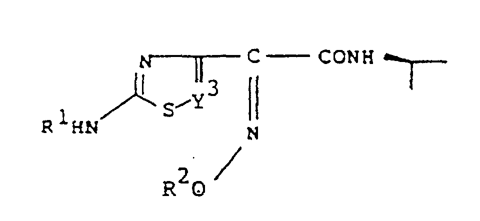

Preferred compounds of the present invention are the syn-isomers, the compounds of formula (II): 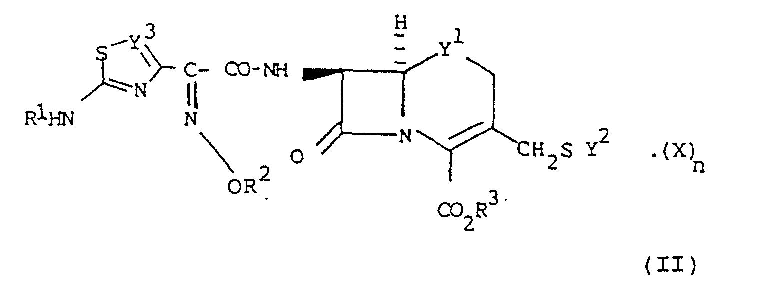 wherein R¹, R², R³, X, Y¹, Y², Y³ and n are as hereinbefore defined.

It will be appreciated that also included within the scope of the invention are salts and carboxy-protected derivatives, including in vivo hydrolysable esters, of any carboxy groups that may be present as optional substitutents in compounds of formula (I). Also included within the scope of the invention are acid addition salts of any amino or substituted amino groups that may be present as optional substituents in compounds of formula (I).

Since the β-lactam antibiotic compounds of the present invention are intended for use in pharmaceutical compositions, it will be readily appreciated that preferred compounds within formula (I) are pharmaceutically acceptable, i.e. are compounds of formula (Ia) or pharmaceutically acceptable salts or pharmaceutically acceptable in vivo hydrolysable esters thereof: 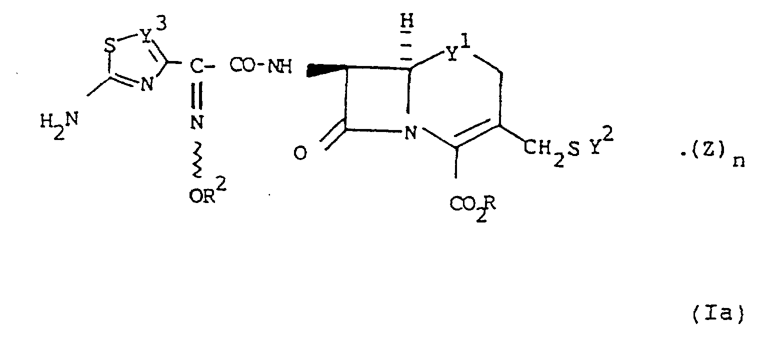 wherein R², Y¹, Y², Y³ and n are as hereinbefore defined, the group CO₂R is carboxy or a carboxylate anion and Z is a pharmaceutically acceptable inorganic or organic anion present in the appropriate stoichiometric proportion to balance the positive charge on the pyridinium ring of the group Y².

Suitable values of Z include chloride, bromide, iodide, phosphate (i.e. 1/3 PO₄³⁻), and sulphate (i.e. ½ SO₄²⁻), when the anion is an inorganic anion; and acetate, hydrogen maleate, methyl sulphonate,dihydrogen citrate, and hydrogen fumarate when the anion is an organic anion.

Non-pharmaceutically acceptable salts of the compound of formula (I) wherein R³ is hydrogen are primarily of use as intermediates in the preparation of a compound of formula (I) wherein R³ is hydrogen or a pharmaceutically acceptable salt thereof.

Salts within compounds of formula (I) may be prepared by salt exchange in conventional manner.

Similarly, carboxy-protected derivatives of formula (I), i.e. those compounds of formula (I) wherein R³ is a readily removable carboxy protecting group, may be used as intermediates in the preparation of a compound of the formula (I) wherein R³ is hydrogen, or a pharmaceutically acceptable salt thereof. Included within the scope of readily removable carboxy protecting groups for R³ are ester groups including pharmaceutically acceptable in vivo hydrolysable ester groups.

From the foregoing, it will be appreciated that within the compounds of the formula (Ia) there exists a sub-group of compounds of the formula (Ib): 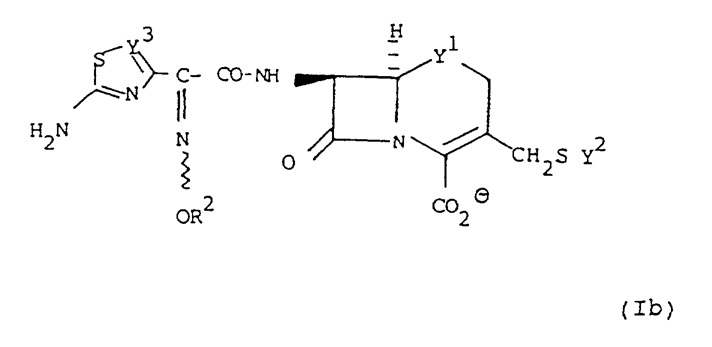 wherein R², Y¹, Y², and Y³ are defined with respect to formula (Ia);
which compounds of formula (Ib) may also be described as betaines, a betaine being defined as an uncharged species having isolated non-adjacent cationic and anionic sites, and not possessing a hydrogen atom bonded to the cationic site.

There also exists within the compounds of formula (Ia) a second sub-group, the compounds of the formula (Ic): 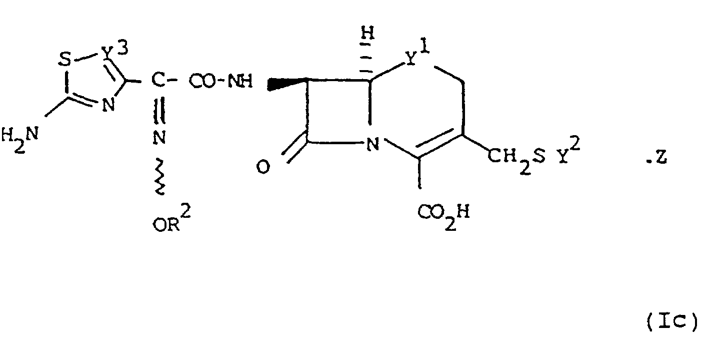 wherein R², Y¹, Y², Y³, and Z are defined with respect to formula (Ia).

Since the β-lactam antibiotic compounds of the present invention are intended for use in pharmaceutical compositions, it will be further understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical, it will readily be understood that the substantially pure form is preferred as for the β-lactam antibiotic compounds. Preferably, whenever possible, the compounds of the present invention are obtained in crystalline form.

When some of the compounds of this invention are allowed to crystallise, or are recrystallised, from organic solvents, solvent of crystallisation may be present in the crystalline product. This invention includes within its scope such solvates. Similarly, some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

Suitable readily removable carboxyl protecting groups for the group -CO₂R³ include groups forming ester derivatives of the carboxylic acid, including in vivo hydrolysable esters. The derivative is preferably one which may readily be cleaved.

Suitable ester-forming carboxyl-protecting groups are those which may be removed under conventional conditions. Such groups for R³ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofuran-2-yl, tetrahydropyran-2-yl, pentachlorophenyl, acetonyl, p-toluenesulphonylethyl, methoxymethyl, a silyl, stannyl or phosphorus-containing group, an oxime radical of formula -N=CHR¹² where R¹² is aryl or heterocyclic, or an in vivo hydrolysable ester radical such as defined below.

A carboxyl group may be regenerated from any of the above esters by usual methods appropriate to the particular R³ group, for example, acid- and base-catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

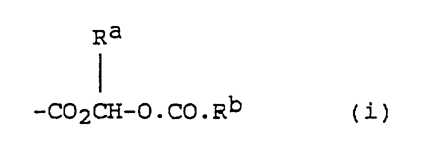

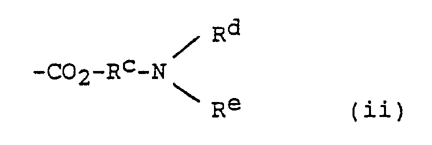

-CO₂CH₂-OR^{f} (iii)

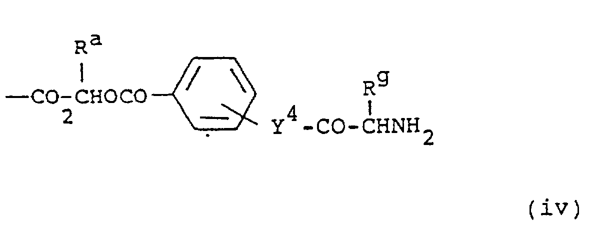

wherein R^{a} is hydrogen, (C₁₋₆)alkyl, (C₃₋₇)cycloalkyl, methyl, or phenyl; R^{b} is (C₁₋₆)alkyl, (C₁₋₆)alkoxy, phenyl, benzyl, (C₃₋₇)cycloalkyl, (C₁₋₆)alkyl(C₃₋₇)cycloalkyl, 1-amino(C₁₋₆)alkyl, or 1-(C₁₋₆alkyl)-amino(C₁₋₆)alkyl; or R^{a} and R^{b} together form a 1,2-phenylene group optionally substituted by one or two methoxy groups; R^{c} is (C₁₋₆)alkylene optionally substituted with a methyl or ethyl group and R^{d} and R^{e} independently are (C₁₋₆)alkyl; R^{f} represents (C₁₋₆)alkyl; R^{g} is hydrogen or phenyl optionally substituted by up to three groups selected from halogen, (C₁₋₆)-alkyl, or (C₁₋₆)alkoxy; and Y⁴ is oxygen or NH.

Examples of suitable pharmaceutically acceptable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)-carbonyloxymethyl; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula: 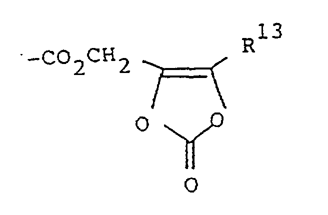 wherein R¹³ is hydrogen, (C₁₋₆)alkyl or phenyl.

Suitable pharmaceutically acceptable salts of the carboxy group of the compound of formula (I) include metal salts, for example aluminium, alkali metal salts such as sodium or potassium, alkaline earth metal salts such as calcium or magnesium; and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylenediamine, 1-ephenamine, N-methylmorpholine, N-ethylpiperidine, N-benzyl-β-phenethylamine, dehydroabietylamine, N,N'-bisdehydro-abietylamine, ethylenediamine or N-methylglucosamine; or basic amino acids such as lysine, arginine, or bases of the pyridine type such as pyridine, collidine or quinoline; or other amines which have been used to form salts with known penicillins and cephalosporins. Other useful salts include the lithium salt and silver salt.

Specific classes of compounds within compounds of formulae (I), (Ia), (Ib), Ic) and (II) as hereinbefore defined are those compounds in which Y¹ is sulphur or sulphoxide.

Specific compounds within this invention include the following and pharmaceutically acceptable salts and in-vivo hydrolysable esters thereof:
[6R,7R]-7-[2-(2-Amino-4-thiazolyl)-2-(Z)-[(R, S)-carboxy(3,4-dihydroxyphenyl)methyloxyimino]acetamido]-3-[1-(methylamino)pyridinium-4-thiomethyl]ceph-3-em-4-carboxylate;
[6R,7R]-7-[2-(2-Amino-4-thiazolyl)-2-(Z)-[(S)-carboxy(3,4-dihydroxyphenyl)methyloxyimino]acetamido]-3-[1-(methylamino)pyridinium-4-thiomethyl]ceph-3-em-4-carboxylate;
[6R,7R]-7-[2-(2-Amino-4-thiazolyl)-2-(Z)-[(R)-carboxy(3,4-dihydroxyphenyl)methyloxyimino]acetamido]-3-[1-(methylamino)pyridinium-4-thiomethyl]ceph-3-em-4-carboxylate;
[6R,7R]-3-[1-Aminopyridinium-4-thiomethyl]-7-[2(2-amino-4-thiazolyl)-2-(Z)-[(R,S)-carboxy(3,4-dihydroxyphenyl)methyloxyimino]acetamido]ceph-3-em-4-carboxylate;
[6R,7R]-3- [1-Aminopyridinium-4-thiomethyl]-7-[2-(2-amino-4-thiazolyl)-2-(Z)-[(S)-carboxy-(3,4-dihydroxyphenyl)methyloxyimino]acetamido]ceph-3-em-4-carboxylate;
[6R,7R]-3-[1-Aminopyridinium-4-thiomethyl]-7-[2-(2-amino-4-thiazolyl)-2-(Z)-[(R)-carboxy(3,4-dihydroxyphenyl)methyloxyimino]acetamido]ceph-3-em-4-carboxylate;
[6R,7R]-7-[2-(2-Amino-4-thiazolyl)-2-(Z)-[3,4-(methylenedioxy)benzyloxyimino]acetamido]-3-[1-(methylamino)pyridinium-4-thiomethyl)ceph-3-em-4-carboxylate;

The antibiotic compounds according to the invention may be formulated for administration in any convenient way for use in human or veterinary medicine, according to techniques and procedures per se known in the art with reference to other antibiotics, and the invention therefore includes within its scope a pharmaceutical composition comprising an antibiotic compound according to the present invention such as, for example a pharmaceutically acceptable compound of formula (I) or a salt or in vivo hydrolysable ester thereof above together with a pharmaceutically acceptable carrier or excipient. The compositions may be formulated for administration by any suitable route, such as oral or parenteral, or by topical application. Normally administration will be via a parenteral route. The compositions may be in the form of tablets, capsules, powders, granules, lozenges, creams or liquid preparations, such as oral or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats; emulsifying agents, for example lecithin, sorbitan monooleate, or acacia; non-aqueous vehicles (which may include edible oils), for example almond oil, oily esters, glycerine, propylene glycol, or ethyl alcohol; preservatives, for example methyl or propyl p-hydroxybenzoate or sorbic acid; and, if desired conventional flavouring or colouring agents. Suppositories will contain conventional suppository base, eg cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in-the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 mg to 12 g per day for an average adult patient (body weight 70 kg), for instance 1500 mg per day, depending on the route and frequency of administration. Such dosages correspond to approximately 1.5 to 170 mg/kg per day. Suitably the dosage is from 1 to 6g per day.

The daily dosage is suitably given by administering a compound of the invention several times in a 24-hour period. Typically, 250 mg is administered 4 times a day although, in practice, the dosage and frequency of administration which will be most suitable for an individual patient will vary with the age, weight and response of the patients, and there will be occasions when the physician will choose a higher or lower dosage and a different frequency of administration. Such dosage regimens are within the scope of this invention.

No toxicological effects are indicated when a pharmaceutically acceptable compound of the invention of formula (I) or a salt or in vivo hydrolysable ester thereof is administered in the above mentioned dosage range.

The antibiotic compounds according to the present invention may be the sole therapeutic agent in the compositions of the invention or a combination with other antibiotics and/or β-lactamase inhibitors may be employed.

Advantageously the compositions also comprise a compound of formula (III) or a pharmaceutically acceptable salt or ester thereof: 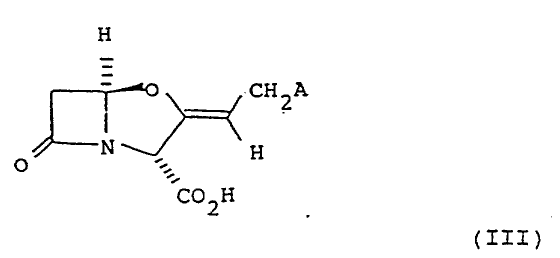 wherein A is hydroxyl; substituted hydroxyl; thiol; a group of formula SO₂R¹⁴ wherein R¹⁴ is (C₁₋₆)alkyl; substituted thiol; amino; mono- or di-(hydrocarbyl) substituted amino; mono- or di-acylamino; an optionally substituted triazolyl group; or an optionally substituted tetrazolyl group as described in EP-A-O 053 893 (to Beecham Group plc).

A further advantageous composition comprises a pharmaceutically acceptable antibiotic compound of the formula (I) or a salt or an in vivo hydrolysable ester thereof and a pharmaceutically acceptable carrier or excipient together with a β-lactamase inhibitor of formula (IV) or a pharmaceutically acceptable salt or in vivo hydrolysable ester thereof: 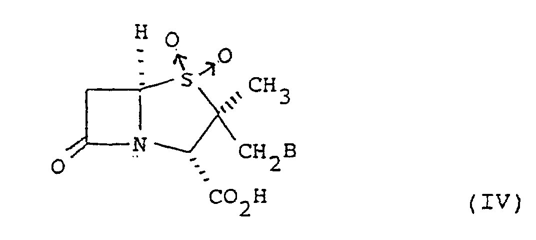 wherein B is hydrogen, halogen or a group of formula: 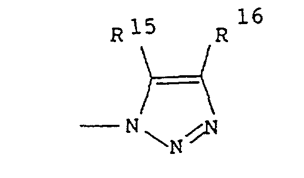 in which R¹⁵ and R¹⁶ are the same or different and each is hydrogen, (C₁₋₆)alkoxycarbonyl, or carboxy or a pharmaceutically acceptable salt thereof.

Further suitable β-lactamase inhibitors include 6-alkylidene penems as described in EP-A-0 041 768 and EP-A-0 154 132 (to Beecham Group plc).

Further suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention is useful in a method of treating bacterial infections in humans and animals which method comprises administering a therapeutically effective amount of an antibiotic compound of the present invention of the formula (Ia) or a pharmaceutically acceptable salt or in vivo hydrolysable ester thereof.

In a further aspect, the present invention also provides for the use of a compound of formula (Ia) or a pharmaceutically acceptable salt or an in vivo hydrolysable ester thereof for the manufacture of a medicament for treating bacterial infection.

The pharmaceutically acceptable antibiotic compounds of the present invention of formula (Ia) or salts or in vivo hydrolysable esters thereof are active against a broad range of Gram-positive and Gram-negative bacteria, and may be used to treat a wide range of bacterial infections including those in immunocompromised patients.

Amongst many other uses, the pharmaceutically acceptable compounds of the invention of formula (Ia) or salts or in vivo hydrolysable esters thereof are of value in the treatment of respiratory tract and urinary tract infections in humans and may also be used to treat mastitis in cattle.

A particular advantage of the antibacterially active compounds of this invention is their stability to β-lactamase enzymes and they are therefore effective against β-lactamase producing organisms.

The present invention further provides a process for the preparation of a compound of formula (I) which process comprises treating a compound of formula (V) : 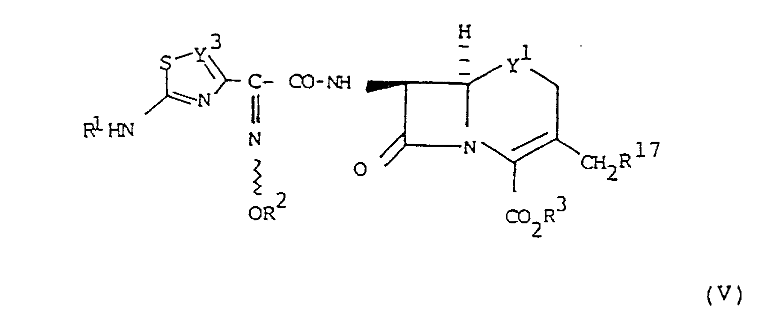 wherein R¹, R², R³, Y¹, and Y³ are as hereinbefore defined, and R¹⁷ is a leaving group; and wherein any reactive groups may be protected;
with a thiopyridone compound of formula (VI): 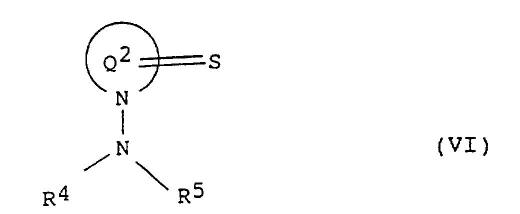 wherein the nucleus: 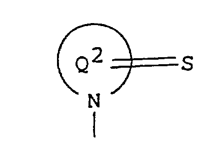 hereinafter referred to as the ring Q² is such that it is converted to the nucleus of the group Y² (as hereinbefore defined) in situ during the course of the reaction;
and R⁴ and R⁵ are as hereinbefore defined;
with the proviso that when R¹⁷ is an acyloxy group -CO₂R³ must be in the free acid form or a salt thereof;
and thereafter if necessary carrying out one or more of the following steps:
i) converting each or any one of the groups R², R³, R⁴ and R⁵ into a different group R², R³, R⁴ and R⁵;
ii) removing any protecting groups; or
iii) converting the product into a salt.

At the end of the process described hereinabove and in other processes for the preparation of the compound of formula (I) described hereinbelow it may be necessary to remove protecting groups. Deprotection may be carried out by any convenient method known in the art that does not cause unwanted side reactions to occur to any appreciable extent.

Suitable leaving groups R¹⁷ include halo such as chloro, bromo or iodo or an acyloxy group such as, for example, the acetoxy group. Preferred groups for R¹⁷ are chloro and iodo.

This reaction is desirably conducted in a solvent. For example, use can be made of water, or of organic solvents inert to the starting compounds, such as dimethylformamide, dimethylacetamide, dioxane, acetone, dichloromethane, 1,2-dichloroethane, acetonitrile, dimethylsulfoxide or tetrahydrofuran, or mixtures thereof. The reaction temperature and time depend, among other factors, upon the starting compounds and solvent to be employed but generally the reaction is carried out at a selected temperature within the range of 0 to 100°C for a selected time of a few minutes to several days.

Compounds of formula (V) wherein R¹⁷ is acyloxy may be prepared by analogy with procedures described in Bucourt R., et al, Tetrahedron, 1978, 34, 2233.

Compound of formula (V) wherein R¹⁷ is halo may be prepared from readily available starting materials by conventional methodology, for instance, by the coupling of compounds of formula (IX) and formula (XI), as hereinafter defined, wherein R¹⁷ is halo.

Preferred compounds of the formula (V) include salts and esters in which R³ is as hereinbefore defined and in particular in which R³ is diphenylmethyl, p-methoxybenzyl or trimethylsilyl.

Compounds of formula (VI) may be prepared by treating the corresponding thiopyranone with a hydrazine derivative of the formula (VII):

H₂NNR⁴R⁵ (VII)

wherein R⁴ and R⁵ are as hereinbefore defined, by analogy with the process described by Ibrahim El-Sayad El-Kholy et al., J. Het. Chem., 1974, 11, 487.

Alternatively, compounds of formula (VI) may be obtained by treating the corresponding pyridone with, for instance, Lawesson's reagent or phosphorus pentasulphide, according to conventional procedures. Suitable pyridones may be prepared according to the methodology of Freeman et al. J. Amer. Chem. Soc., 1947, 69, 858.

Included within the compounds of formula (VI) are compounds of the formula (VIa) : 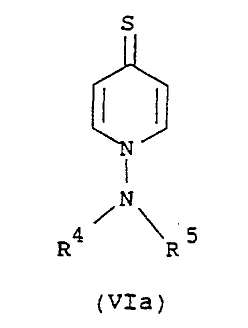 wherein R⁴ and R⁵ are as hereinbefore defined and in which the 4-thiopyridone ring may be optionally substituted at a ring carbon available for substitution by up to four substituents as hereinbefore defined.

It will be appreciated that within the process hereinbefore described there exists a specific process in which in the compound of formula (V), Y¹ is sulphur, -SO- or -SO₂-.

The compounds of formula (I) may also suitably be prepared by reacting a compound of formula (VIII) or a salt thereof: 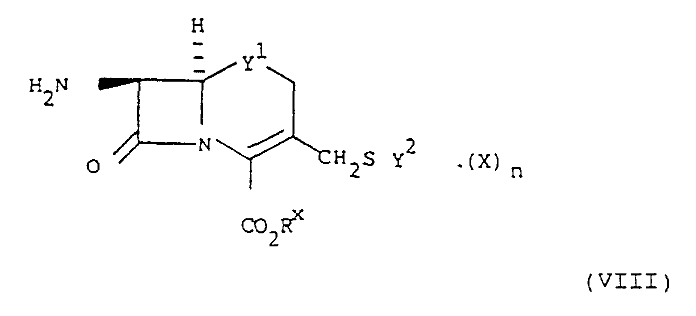 wherein X, Y¹, Y² and n are as hereinbefore defined, R^{x} is hydrogen or a readily removable carboxyl blocking group and the 7β-amino group is optionally substituted with a group which permits acylation to take place; and any reactive groups may be protected;
with an N-acylating derivative of an acid of formula (IX): 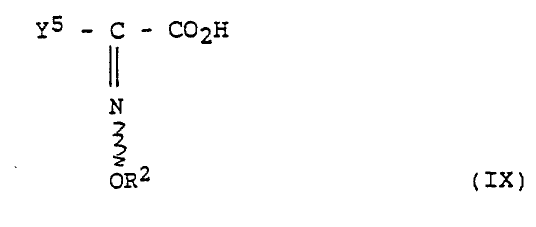 wherein R² is as hereinbefore defined and Y⁵ is a group of formula: 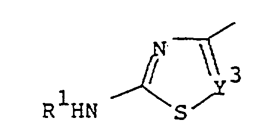 or a group which is convertible thereto, and R¹ is as hereinbefore defined; and wherein any reactive groups may be protected; and thereafter, if necessary, carrying out one or more of the following steps:
(i) removing any protecting group, including an amino-protecting group R¹;
(ii) converting the group R^{x} into a group R³;
(iii) converting the product to a salt;
(iv) converting a group which is convertible to Y⁵ into Y⁵, or
(v) converting each or any one of the groups R⁴ and R⁵ into a different group R⁴ and R⁵.

Suitable groups which permit acylation to take place and which are optionally present on the amino group of the starting material of the formula (VIII) include silyl, stannyl and phosphorus groups, for example trialkylsilyl groups such as trimethylsilyl, trialkyltin groups such as tri-n-butyltin, phosphorus groups of formula -PR¹⁸R¹⁹ wherein R¹⁸ is an alkyl, haloalkyl, aryl, aralkyl, alkoxy, haloalkoxy, aryloxy, aralkyloxy or dialkylamino group, R¹⁸ is the same as R¹⁹ or is halogen or R¹⁸ and R¹⁹ together form a ring; suitable such phosphorus groups being -P(OC₂H₅)₂, -P(C₂H₅)₂,

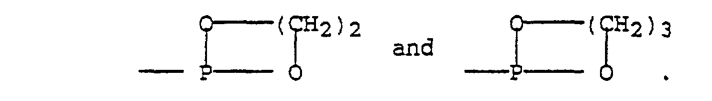

A group which may optionally be introduced in situ prior to acylation onto the amino group in the compound of formula (VIII) is trimethylsilyl.

An appropriate reactive N-acylating derivative of the acid (IX) is employed in the above process.

Suitable N-acylating derivatives include an acid halide, preferably the acid chloride or bromide.

Acylation with an acid halide may be effected in the presence of an acid binding agent, for example a tertiary amine (such as pyridine or dimethylaniline), molecular sieves, or an inorganic base (such as calcium carbonate or sodium bicarbonate) or a silylated derivate of acetamide [such as trimethylsilylacetamide or N,O-bis(trimethylsilylacetamide)] or an oxirane, which binds hydrogen halide liberated in the acylation reaction. The oxirane is preferably a (C₁₋₆)-1,2-alkylene oxide, such as ethylene oxide or propylene oxide. The acylation reaction using an acid halide may be carried out at a temperature in the range -50°C to +50°C, preferably -20°C to +20°C, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof.
Alternatively, the reaction may be carried out in an unstable emulsion of water-immiscible solvent, especially an aliphatic ester or ketone, such as methyl isobutyl ketone or butyl acetate. Preferred solvents include tetrahydrofuran, and anhydrous chlorinated hydrocarbons, especially dichloromethane.

The acid halide may be prepared by reacting the acid (IX) or a salt or suitable derivative thereof with a halogenating (eg chlorinating or brominating) agent such as phosphorus pentachloride, phosphorus oxychloride, thionyl chloride, oxalyl chloride, or phosgene.

Further suitable derivatives of the acid (IX) which may be employed in the above process include labile esters such as silyl esters. Suitable silyl esters include, for example, tri(C₁₋₆)alkyl silyl esters, especially the trimethylsilyl ester.

Other suitable N-acylating derivatives of the acid (IX) include symmetrical and mixed anhydrides. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid). Preferably the N-acylating derivative of the acid (IX) is a mixed anhydride with methanesulphonic acid.

When a symmetrical anhydride is employed, the reaction may be carried out in the presence of 2,6-lutidine as catalyst.

When a mixed anhydride is employed the N-acylating derivative is preferably prepared in the presence of an organic base such as triethylamine and/or N,N-diisopropylethylamine in a suitable solvent such as DMF or dichloromethane at between -50°C and 100°. Alternatively, the N-acylating derivative may be prepared from an alkali metal salt of the acid of formula (IX), such as the sodium salt, in a suitable solvent such as DMF at between -50°C and room temperature. The N-acylating derivative of the acid of formula (IX) so derived may then be reacted with a compound of formula (VIII). The acylation reaction may conveniently be carried out at between -50°C to +50°C in a suitable solvent such as water, acetonitrile, DMF or dichloromethane. The reaction may be carried out in the presence of a suitable base such as triethylamine, sodium hydrogen carbonate, pyridine or N,N-diisopropylethylamine.

Further N-acylating derivatives of acid (IX) are the acid azide, or activated esters such as esters with cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (IX) with an oxime.

Other N-acylating derivatives of the acid of formula (IX) are thioesters of formula (X): 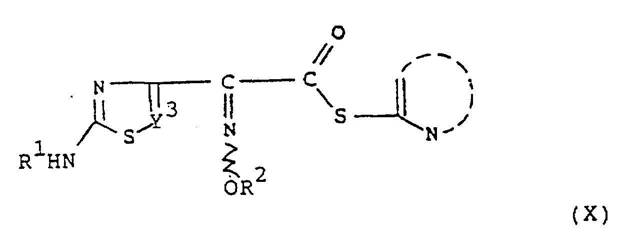 wherein R¹, R², and Y³ are as hereinbefore defined and the moiety: 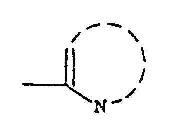 represents a 5- or 6-membered heterocyclic ring, which may contain, in addition to the nitrogen atom, one or two further heteroatoms, selected from oxygen, nitrogen and sulphur and which may be substituted or fused to a benzene ring which may itself be substituted.

Preferred thioester acylating agents derived from the acid of formula (IX) are the thioesters (Xa) or (Xb): 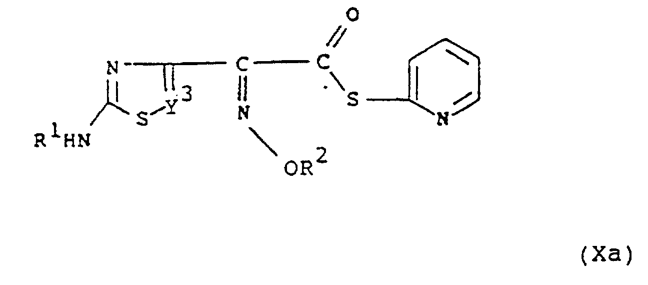 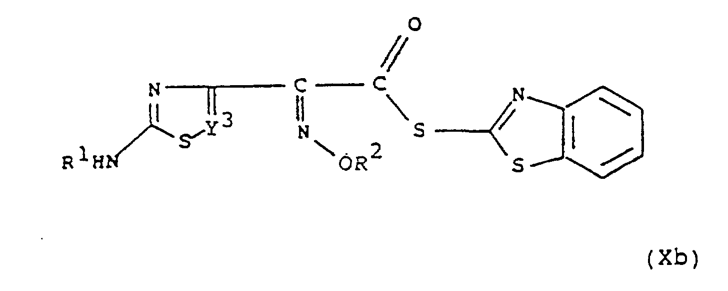 wherein R¹, R² and Y³ are as hereinbefore defined.

Compounds of the formula (Xa) and (Xb) may be prepared by treatment of the acid (IX) with 2,2'-dipyridyldisulphide or 2,2'-dibenzothiazolyldisulphide respectively, in the presence of triphenylphosphine, analogously to the routes described in EP-A-0 037 380 (to Biochemie GmbH). Conveniently, in compounds of the formula (Xa) and (Xb), R¹ may be hydrogen.

Other suitable N-acylating derivatives of the acid (IX) include the reactive intermediates formed by reaction in situ with a condensing agent such as a carbodiimide, for example, N,N'-diethyl-, di-n-propyl- or diisopropylcarbodiimide, N,N'-di-cyclohexylcarbodiimide, or N-ethyl-N'-[3-(dimethylamino)propyl]-carbodiimide; a suitable carbonyl compound, for example, N,N'-carbonyldiimidazole or N,N'-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example BBr₃.C₆H₆); or a phosphoric acid condensing agent such as diethylphosphorylcyanide. The condensation reaction is preferably carried out in an organic reaction medium, for example, dichloromethane, dimethylformamide, acetonitrile, alcohol, benzene, dioxan or tetrahydrofuran.

A further method of forming the N-acylating derivative of the acid (IX) is to treat the acid of formula (IX) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid (IX) so derived may then be caused to react with a compound of formula (VIII). The acylation reaction may conveniently be carried out at -40° to +30°C, if desired in the presence of an acid binding agent such as pyridine, trimethylsilylacetamide or N,O-bis(trimethylsilyl acetamide). A catalyst such as 4-dimethylaminopyridine may optionally also be added. A preferred solvent for the above acylation reaction is dichloromethane.

A preferred amino protecting group R¹ in the intermediate of formula (IX) is trityl, which R¹ group may be suitably removed from the product of formula (I) by treatment with formic acid or trifluoracetic acid.

Compounds of formula (IX) may be prepared by routes analogous to those disclosed in GB 2 025 398A and by Takasugi et al., J.Antibiotics [1983] 36, 846 et seq and modifications thereto described in EP-A-0 210 815 (Beecham Group plc).

The compounds of formula (VIII) herein which are, inter alia, intermediates for the compounds of formula (I) as hereinbefore defined may be prepared by reacting a compound of formula (XI) or acid addition salt thereof: 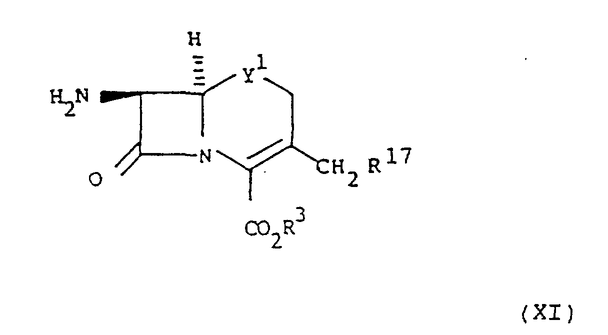 wherein R³, R¹⁷, and Y¹ are as hereinbefore defined, and the 7β-amino group is optionally protected with an amino protecting group; with a compound of the formula (VI) as hereinbefore defined;
with the proviso that when R¹⁷ is an acyloxy group, the group CO₂R³ must be in the free acid form or a salt thereof;
and thereafter if necessary carrying out one or more of the following steps:
i) converting each or any of the groups R⁴ and R⁵ into a different group R⁴ and R⁵;
ii) removing any protecting group; or
iii) converting the group R³ into a group R^{X}.

Compounds of formula (V), as hereinbefore defined, may be prepared by reacting a compound of formula (XI) as hereinbefore defined or a derivative thereof in which the 7β-amino group is substituted with a group which permits acylation to take place; and any reactive groups may be protected; with an N-acylating derivative of an acid of formula (IX), as hereinbefore defined and thereafter, if necessary,carrying out one or more of the following steps:
(i) removing any protecting group,
(ii) replacing a group R¹⁷ by another group R¹⁷; or
(iii converting a group which is convertible to Y⁵ into Y⁵.

Compounds of formula (XI) are well known and readily available.

Compounds of the formula (I) in which Y¹ is sulphur, -SO-, or -SO₂- may be inter-converted by methods known in the art.

Suitable examples of compounds for formula (VI) include:
1-(Methylamino)-4-thiopyridone;
1-[N-(t-Butyloxycarbonyl)-N-methylamino]-4-thiopyridone;
1-(Dimethylamino)-4-thiopyridone;
1-(t-Butyloxycarbonylamino)-4-thiopyridone;
1-[N-(t-Butyloxycarbonyl)-N-(t-butyloxycarbonylmethyl)-amino]-4-thiopyridone;
1-[N-(t-Butyloxycarbonyl)-N-ethylamino]-4-thiopyridone;
1-[N-(t-Butyloxycarbonyl)-N-(cyclopropylmethyl)amino] -4-thiopyridone;
1-[N-(t-Butyloxycarbonyl)-N-(prop-2-en-l-yl)amino]-4-thiopyridone;
1-Amino-4-thiopyridone;
1-(Propylamino)-4-thiopyridone;
1-[N-(t-Butyloxycarbonyl)-N-(cyclopentyl)amino]-4-thiopyridone;
1-[N-Butyl-N-(t-butyloxycarbonyl)amino]-4-thiopyridone;
1-[N-(t-Butyloxycarbonyl)-N-hexylamino]-4-thiopyridone ;
1-[N-(t-Butyloxycarbonyl)-N-(1-isopropyl)amino]-4-thiopyridone;
1-[N-(t-Butyloxycarbonyl)-N-(2-hydroxyethyl)amino]-4-thiopyridone;
1-(2-Pyridylamino)-4-thiopyridone;
1-(3,5-Dimethylisoxazol-4-yl)methylamino-4-thiopyridone;
1-(N-Acetyl-N-methylamino)-4-thiopyridone;
1-[N-Methyl-N-(4-nitrobenzoyl)amino]-4-thiopyridone;
1[N-(4-Methoxybenzoyl)-N-methylamino]-4-thiopyridone;
1-[N-(2-Furoyl)-N-methylamino]-4-thiopyridone
1-[N-[3,4-bis(4-Methoxybenzyloxy)benzoyl]-N-methyl amino]-4-thiopyridone;
1-(N-Benzoyl-N-methylamino)-4-thiopyridone;
1-[N-[3,4-bis(4-Methoxybenzyloxy)cinnamoyl]-N-methylamino]-4-thiopyridone;
1-Ureido-4-thiopyridone;
1-(1,3-Dimethylureido)-4-thiopyridone;
1-(1-Methylureido)-4-thiopyridone;
1-[N-(4-Diphenylmethoxycarbonylbenzoyl)-N-methylamino]-4-thiopyridone;
1-[N-[4-(t-Butoxycarbonylamino)benzoyl]-N-methylamino]-4-thiopyridone;
1-(t-Butyloxycarbonylamino)-5-methoxy-2-(methoxymethyl) -4-thiopyridone;
1-[N-(t-Butyloxycarbonyl)-N-(2-methoxyethyl)amino]-4-thiopyridone;
1-[(6-Chloropyridin-2-yl)amino]-4-thiopyridone;
1-(Pyrazineamino)-4-thiopyridone;
1-[N-(t-Butyloxycarbonyl)-N-(2-methyl-4-thiazolyl) methylamino]-4-thiopyridone;
1-[(2-Imidazolin-2-yl)amino]-4-thiopyridone;
1-(Phthalazin-1-ylamino)-4-thiopyridone;
1-[N-(t-Butyloxycarbonyl)-N-(cyanomethyl)amino]-4-thiopyridone;
1-[N-(t-Butyloxycarbonyl)-N-(prop-2-yn-l-yl)amino]-4-pyridone;
and corresponding analogues thereof lacking an amino protecting group such as, for example,
t-butoxycarbonyl, a hydroxy protecting group, such as for example, 4-methoxybenzyloxy, or a carboxy protecting group, such as, for instance, 4-diphenylmethoxy.

The compounds of formulae (Ia), (Ib), and (Ic) may be prepared by similar processes to those described hereinabove as suitable for the preparation of a compound of the formula (I), except that each process for the preparation of the compound of formulae (Ia), (Ib) or (Ic) further comprises, if necessary, the step of converting the product into a pharmaceutically acceptable salt or pharmaceutically acceptable in vivo hydrolysable ester.

Conversion of betaines of formula (Ib) into salts of formula (Ic) and vice versa may readily be carried out by conventional methods. For example salts of the formula (Ic) may be prepared from betaines of formula (Ib) by treatment with a dilute mineral acid such as hydrochloric acid or sulphuric acid.

Quaternary salts within formula (Ic) may also be prepared by salt exchange in a conventional manner, for example by means of an ion-exchange resin.

The antibiotic compounds of the present invention are active against a wide range of Gram-negative and Gram-positive organisms including E.coli such as, for example ESS, JT4, JT425 and NCTC 10418; Pseudomonas Spp. such as Ps.aeruqinosa for example 10662 and Dalgleish; Serratia marcescens US32; Klebsiella aerogenes A; Enterobacter cloacae N1; P.mirabilis such as, for example C977 and 889; P.morganii; P.rettgeri; B.subtilis; Staph. aureus such as, for example Oxford and Russell; N.catarrhalis 1502.

The following Examples illustrate the preparation of the compounds of the present invention.

### PREPARATION 1

### 1-(Methylamino)-4-thiopyridone

### a) t-Butyl 1-methylhydrazinecarboxylate.

N-Methylhydrazine (1.6ml; 0.03mol) in dichloromethane (20ml) was treated with di-t-butyl dicarbonate (6.6g; 0.03mol) in dichloromethane (25ml) dropwise, and stirred for 90 minutes. The mixture was decanted from the sticky residue and evaporated to minimum volume twice from dichloromethane and once from dichloromethane/toluene /toluene to give the title compound (4g, 97%); δH (CDCl₃) 1.47 (9H, s), and 3.03 (3H, s).

### b) 1-[N-(t-Butyloxycarbonyl)-N-methylamino]-4-thiopyridone

t-Butyl 1-methylhydrazinecarboxylate (0.5g; 3.4mmol) and 4-thiopyrone (0.34g; 3mmol) in ethanol (20ml) were refluxed for 24 hours. The solution was evaporated to dryness and the product purified by chromatography on silica gel eluting with mixtures of hexane and ethyl acetate to give the title compound (0.55g, 76%); δH (CDCl₃) 1.47 (9H, s), 3.37 (3H, s), 7.19 and 7.32 (4H, ABq, J 7Hz); λₘₐₓ (EtOH) 356nm (E 29050 dm³ mol⁻¹ cm⁻¹) (Found: M⁺, 240.0945, C₁₁H₁₆N₂O₂S requires M, 240.0932).

### c) 1-(Methylamino)-4-thiopyridone

1-[N-(t-Butyloxycarbonyl)-N-methylamino]-4-thiopyridone (1g, 4.16mmol) in dichloromethane (40ml) was treated with trifluoroacetic acid (5ml) and stirred for 2½ hours. On completion of the reaction the mixture was evaporated to dryness and the residue dissolved in ethyl acetate. The product was extracted into water maintained at pH 6.8 with sodium bicarbonate. The water was evaporated to low volume and then the product absorbed onto silica gel by evaporation.
The product was then purified by chromatography on silica gel eluting with mixtures of ethanol in dichloromethane to give the title compound (0.4g, 67%) υₘₐₓ (KBr) 1685, 1605, 1523 and 1108 cm⁻¹; δH (CDCl₃) 2.95 (3H, d, J 6Hz), 5.46 (1H, q, J 6Hz), and 7.34 (4H, s); M⁺ 140.

### Preparation 2

### 1-(Dimethylamino)-4-thiopyridone

4-Thiopyranone (0.112g, 1.0mmol) in N,N-dimethylformamide (2ml) was treated with N,N-dimethylhydrazine (0.76ml, 10mmol) and stirred for 3 hours at room temperature. The solvent was evaporated under reduced pressure and the residue chromatographed on silica gel eluting with dichloromethane and mixtures of dichloromethane and ethanol to give the title compound (0.069g, 45%); δ(CDCl₃) 7.55 (4H, s) 2.88 (6H, s); M⁺ 154.

### PREPARATION 3

### 1-(t-Butyloxycarbonylamino)-4-thiopyridone

t-Butylcarbazate (0.13g, 1.0mmol) and 4-thiopyranone (0.11g, 1.0mmol) were heated at reflux in ethanol for 2h. The mixture was allowed to cool, diluted with acetone, then evaporated under reduced pressure. Chromatography on silica gel 60 eluting with ethanol, dichloromethane (1:19) gave the title compound containing traces of unreacted t-butylcarbazate (0.49g); υₘₐₓ (KBr) 1745, 1611, and 1503cm⁻¹; δ_{H} (CDCl₃) 1.51 (9H, s), and 7.33 (4H, s); M⁺ 226.

### PREPARATION 4

### 1-[N-(t-Butyloxycarbonyl)-N-(t-butyloxycarbonylmethyl)-amino]-4-thiopyridone

### a) 1-(t-Butyloxycarbonylamino)-4-pyridone

4-Pyranone (1g, 10.4mmol) and t-butylcarbazate (1.32g, 10mmol) were heated at reflux in ethanol for 48h. The solvent was gradually allowed to distil from the mixture and the residue was chromatographed on silica gel 60, eluting with ethanol, dichloromethane (1:19) to give the title compound (1.16g, 50%); υₘₐₓ (KBr) 1723, 1630, and 1550cm⁻¹; δ_{H} 1.54 (9H, s), 6.47 (2H, d), and 7.60 (2H, d); M⁺ 210.

### (b) 1-[N-(t-Butyloxycarbonyl)-N-(t-butyloxycarbonylmethyl)amino]-4-pyridone

1-(t-Butyloxycarbonylamino)-4-pyridone (0.057g, 0.25mmol) in N,N-dimethylformamide (5ml) was treated successively with potassium carbonate (0.035g, 0.25mmol) and t-butyl bromoacetate (0.04ml, 0.25mmol). The reaction mixture was stirred for 0.5h, evaporated under reduced pressure and chromatographed on silica gel 60 eluting with ethanol, dichloromethane (1:19) to give the title compound containing traces of solvent (0.09g); υₘₐₓ (CH₂Cl₂) 1740, 1735 (sh), 1635, and 1590cm⁻¹; δ_{H} (CDCl₃) 1.47 (9H, s), 1.51 (9H, s), 4.33 (2H, s), 6.37 (2H, d), and 7.70 (2H, d); M⁺ 324.

### c) 1-[N-(t-Butyloxycarbonyl)-N-(t-butyloxycarbonylmethyl)amino]-4-thiopyridone

The product from 4(b) (0.09g, from 0.25mmol) in toluene (5ml) was treated with Lawesson's Reagent (0.057g, 0.14mmol). The mixture was heated at 80°C for 5mins, allowed to cool, then chromatographed on silica gel 60 eluting with ethanol, dichloromethane mixtures to give the title compound (0.081g, 95%); υₘₐₓ (CH₂Cl₂) 1740, 1615, and 1115cm⁻¹; ^{δ}H (CDCl₃) 1.49 (9H, s), 1.72 (9H, s), 4.41 (2H, s), and 7.42 (4H, s); M⁺ 340.

### PREPARATION 5

### 1-[N-(t-Butyloxycarbonyl)-N-ethylamino]-4-thiopyridone

### a) 1-[N-(t-Butyloxycarbonyl)-N-ethylamino]-4-pyridone

1-(t-Butyloxycarbonylamino)-4-pyridone (0.23g, 1.1mmol) in N,N-dimethylformamide (5ml) was treated with potassium carbonate (0.14g, 1.0mmol) followed by ethyl iodide (0.08ml, 1.0mmol). The mixture was stirred for 1h, evaporated under reduced pressure and chromatographed on silica gel 60, eluting with ethanol, dichloromethane (1:19) to give the title compound (0.124g, 49%); δ_{H} (CDCl₃) 1.21 (3H, t, J 7Hz), 1.45 (9H, s), 3.82 (2H, q, J 7Hz), 6.44 (2H, d, J 8Hz), and 7.41 (2H, d, J 8Hz); M⁺ 238.

### (b) 1-[N-(t-Butyloxycarbonyl)-N-ethylamino]-4-thiopyridone

The product from (a) was reacted in a similar manner to that described in Preparation 4(c) to give the title compound: υₘₐₓ (CH₂Cl₂) 1725, 1620, and 1598cm⁻¹; δ_{H} (CDCl₃) 1.23 (3H, t, J 7Hz), 1.48 (9H, s), 3.95 (2H, m), 7.0 - 7.7 (4H, m); M⁺ 254.

### PREPARATION 8

### 1-[N-(t-Butyloxycarbonyl)-N-(cyclopropylmethyl)amino]-4 -thiopyridone.

### a) 1-[N-(t-Butyloxycarbonyl)-N-(cyclopropylmethyl)-aminol-4-pyridone.

1-(t-Butyloxycarbonylamino)-4-pyridone (0.23g, 1.0mmol) in N,N-dimethylformamide (5ml) was treated successively with potassium carbonate (0.14g, 1.0mmol) and cyclopropylmethyl bromide (0.1g, 1.0mmol). The reaction mixture was stirred for 3h and evaporated under reduced pressure. Purification on silica gel 60 eluting with ethanol, dichloromethane (1:19) gave the title compound (0.22g, 76%); υₘₐₓ (KBr) 1708, 1649, 1629, and 1583 cm⁻¹; δ_{H} (CDCl₃) 0.18 (2H, q, J 5Hz),0.59(2H,m), 0.88-1.04 (1H,m) 1.45 (9H,s), 3.54 (2H,d, J 7Hz), 6.40 (2H,d, J 6Hz), and 7.30 (2H,d, J 6Hz); M⁺ 264.

### b) 1-[N-(t-Butyloxycarbonyl)-N-(cyclopropylmethyl)-amino]-4-thiopyridone.

The product of Preparation 8(a) (0.22g 0.8mmol) in toluene (5ml) was treated with Lawesson's reagent (0.17g, 0.4mmol) and heated at 80°C for 1h. The mixture was allowed to cool and chromatographed on silica gel 60 eluting with ethanol, dichloromethane mixtures to give the title compound (0.079g, 35%); δ_{H} (CDCl₃) 0.19 (2H,q, J 5.5Hz), 0.60 (2H,q, J 5.5Hz), 0.88 (1H,t, J 5.5Hz), 1.29 (9H,s), 3.62 (2H,d, J 7Hz), and 7.2-7.8 (4H,m); M⁺ 280.

### PREPARATION 9

### 1-[N-(t-Butyloxycarbonyl)-N-(prop-2-en-1-yl)amino]-4-thiopyridone.

### a) 1-[N-(t-Butyloxycarbonyl)-N-(prop-2-en-1-yl)amino]--4-pyridone.

(i) 1-(t-Butyloxycarbonylamino)-4-pyridone (0.21g, 1.0mmol) in 1,2-dimethoxyethane (10ml) was treated successively with 50% sodium hydride dispersion in oil (0.048g, 1.0mmol) and cyclopropyl bromide (1.0ml, 12.5mmol). After being stirred for 48h, the mixture was evaporated under reduced pressure. Purification on silica gel 60 eluting with ethanol, dichloromethane (1:9) gave the title compound (0.042g, 17%); δ_{H} (CDCl₃) 1.37 (9H,s), 4.24 (2H,d, J 9Hz), 5.0-5.9 (3H,m), 6.35 (2H,m), and 7.26 (2H,m); M⁺ 250.

(ii) 1-(t-Butyloxycarbonylamino)-4-pyridone (0.42g, 2.0mmol) in N,N-dimethylformamide was treated with potassium carbonate (0.3g, 2.2mmol) and cyclopropylbromide (1.0ml, 12.5 mmol). The mixture was stirred for 12 days, then evaporated under reduced pressure and chromatographed on silica gel 60 eluting with ethanol, dichloromethane (1:9) to give the title compound (0.046g, 9%).

### b) 1-[N-(t-Butyloxycarbonyl)-N-(prop-2-en-1-yl)amino]--4-thiopyridone.

The product from Preparation 9(a) (0.199g, 0.48mmol) in toluene (5ml) with Lawesson's reagent (0.194g, 0.48mmol) was heated at 80°C for 2h, then allowed to cool and chromatographed in silica gel 60 eluting with ethanol, dichloromethane (1:50) to give the title compound (0.089g, 70%); υₘₐₓ (KBr) 1711, and 1615 cm⁻¹; δ_{H} (CDCl₃) 1.46 (9H,s), 4.26 (2H,d, J 7Hz), 5.10-5.40 (2H,m), 5.62-5.77 (1H,m), 7.03 (2H,d, J 6Hz), and 7.39 (2H,d, J 6Hz); M⁺ 266.

### PREPARATION 10

### 1-Amino-4-thiopyridone

1-(t-Butyloxycarbonylamino)-4-thiopyridone (0.155g, 0.69mmol) was treated with trifluoroacetic acid (2.0ml, 26mmol) and stirred for 5 mins. The reaction mixture was diluted with toluene(1ml) and evaporated to dryness under reduced pressure. The residue was dissolved in water and adjusted to pH6.5 by addition of aqueous sodium hydrogen carbonate solution. The solution was washed with diethyl ether and evaporated under reduced pressure. The crude product was purified by chromatography on silica gel 60 eluting with ethanol, dichloromethane mixtures to give the title compound; υₘₐₓ (KBr) 1684 and 1624 cm⁻¹; δ_{H} (CDCl₃ + CD₃OD) 7.41 (2H,d, J 7Hz), and 7.50 (2H,d, J 7Hz); M⁺ 126.

### PREPARATION 11

### 1-(Propylamino)-4-thiopyridone

### a) 1-[N-(t-Butyloxycarbonyl)-N-propylamino]-4-pyridone.

1-(t-Butyloxycarbonylamino)-4-pyridone (0.1g, 0.047mmol) in N,N dimethylformamide (2ml) was treated successively with potassium carbonate (0.064g, 0.047mmol) and iodopropane (0.081g, 0.047mmol). The reaction mixture was stirred at room temperature for 3h and evaporated under reduced pressure. Purification on silica gel 60 eluting with ethanol, dichloromethane (1:9) gave the title compound (0.098g, 82%); δ_{H} (CDCl₃) 0.98 (3H,t J 7Hz), 1.45 (9H,s), 1.53-1.68 (2H,m) 3.62 (2H,t, J 7.5Hz), 6.35 (2H,d, J 8Hz), and 7.21 (2H,d, J 8Hz); M⁺ 252.

### b) 1-[N-(t-Butyloxycarbonyl)-N-propylamino]-4-thiopyridone

The product of Preparation 11(a) (0.098g, 0.39mmol) in toluene (10ml) was treated with Lawesson's reagent (0.15g, 0.37mmol) and heated at 80°C for 0.5h. The mixture was allowed to cool and chromatographed on silica gel 60 eluting with ethanol, dichloromethane mixtures to give the title compound (0.05g, 48%); δ_{H} (CDCl₃) 0.96 (3H,t, J 7Hz), 1.45 (9H,s), 1.5-1.65 (2H,m), 3.64 (2H,t, J 7Hz), 7.03 (2H,d, J 7Hz), and 7.40(2H,d, J 7Hz); M⁺ 268.

### c) 1-[N-Propylamino]-4-thiopyridone

The product of Preparation 11(b) (0.2g, 0.75mmol) was dissolved in trifluoroacetic acid (3ml), the reaction mixture was stirred for 10 minutes, toluene (10ml) was added and the mixture was evaporated under reduced pressure. A further two (10ml) volumes of toluene were added and evaporated under reduced pressure. Water (10ml) was added and the pH adjusted to 7.2 using aqueous sodium hydrogen carbonate. The product was extracted into dichloromethane and dried over magnesium sulphate, filtered and evaporated under reduced pressure. Purification on silica gel 60 eluting with ethanol, dichloromethane (1:9) gave the title compound (0.074g, 59%); δ_{H} (CDCl₃) 1.0 (3H,t, J 7Hz), 1.5-1.6 (2H,m), 3.09 (2H,t, J 7Hz), and 7.29-7.38 (4H,m); M⁺ 168.

### PREPARATION 12

### 1-[N-(t-Butyloxycarbonyl)-N-(cyclopentyl)amino]-4-thiopyridone

### a) 1-[N-(t-Butyloxycarbonyl)-N-(cyclopentyl)amino]-4-pyridone

1-(t-Butyloxycarbonylamino)-4-pyridone (0.2g, 0.94mmol) in N,N dimethylformamide (4ml) was treated successively with potassium carbonate (0.128g, 0.94mmol) and 1-iodocyclopentane (0.184g, 0.94mmol). The reaction mixture was stirred at room temperature for 3h and evaporated under reduced pressure. Purification on silica gel 60 eluting with ethanol, dichloromethane (1:9) gave the title compound (0.109g, 41%) δ_{H} (CDCl₃), 1.45 (9H,s), 1.5-2.07 (8H,m), 4.5-4.62 (1H,m), 6.4 (2H,d, J 8Hz) and 7.2 (2H,d, J 8Hz); M⁺ 278.

### b) 1-[N-(t-Butyloxycarbonyl)-N-(cyclopentyl)amino]-4-thiopyridone

The product of Preparation 12(a) (0.104g, 0.37mmol) in toluene (10ml) was treated with Lawesson's reagent (0.15g, 0.37mmol) and heated at 80°C for 0.5h under Argon. The mixture was allowed to cool and chromatographed on silica gel 60 eluting with ethanol, dichloromethane mixtures to give the title compound (0.042g, 37%); δ_{H} (CDCl₃) inter alia 1.45 (9H,s), 1.5-2.25 (8H,m), 4.5-4.65 (1H,m), 7.25 and 7.6 (4H, 2 s); M⁺ 294.

### PREPARATION 13

### 1-[N-(t-Butyloxycarbonyl)-N-(prop-2-en-1-yl) amino]-4-thiopyridone

### a) 1-[N-(t-Butyloxycarbonyl)-N-(prop-2-en-1-yl)amino]-4-pyridone

1-(t-Butyloxycarbonylamino)-4-pyridone (0.2g, 0.94mmol) in N,N dimethylformamide (3ml) was treated successively with potassium carbonate (0.31g, 0.95mmol) and allyl bromide (0.114g, 0.94mmol). The reaction mixture was stirred for 1h and evaporated under reduced pressure. Purification on silica gel 60 eluting with ethanol, dichloromethane (1:9) gave the title compound (0.185g, 78%); identical to the product in Preparation 9(a).

### b) 1-[N-(t-Butyloxycarbonyl)-N-(prop-2-en-1-yl)-amino]-4-thiopyridone

The product of Preparation 13(a) (0.185g, 0.74mmol) in toluene (15ml) was treated with Lawesson's reagent (0.225g, 0.56mmol) and heated at 80°C for 0.5h. The mixture was allowed to cool and chromatographed on silica gel 60 eluting with ethanol, dichloromethane mixtures to give the title compound (0.09g, 46%); identical to the product of Preparation 9(b).

### PREPARATION 14

### 1-[N-Butyl-N-(t-butyloxycarbonyl)amino]-4-thiopyridone

### a) 1-[N-Butyl-N-(t-butyloxycarbonyl)amino]-4-pyridone

1-(t-Butyloxycarbonylamino)-4-pyridone (0.1g, 0.47mmol) in N,N dimethylformamide (2ml) was treated successively with potassium carbonate (0.66g, 0.47mmol) and 1-bromobutane (0.065g, 0.47mmol). This mixture was stirred for 4h and then evaporated under reduced pressure. Purification on silica gel 60 eluting with ethanol, dichloromethane (1:9) gave the title compound (0.088g, 70%); δ_{H} (CDCl₃) 0.96 (3H,t, J 7Hz), 1.3-1.64 (4H,m), 1.45 (9H,s) 3.66 (2H,t, J 7Hz), 6.32 (2H,d,J 7Hz), 7.21 (2H,d, J 7Hz); M⁺ 266.

### b) 1-[N-Butyl-N-(t-butyloxycarbonyl)amino]-4-thiopyridone

The product of Preparation 14(a) (0.088g, 0.33mmol) in toluene (10ml) was treated with Lawesson's reagent (0.13g, 0.32mmol) and heated at 80°C for 0.5h. The mixture was allowed to cool and chromatographed on silica gel 60 eluting with ethanol, dichloromethane (1:9) to give the title compound (0.053g, 57%); δ_{H} (CDCl₃) 0.95 (3H,t, J 7Hz) 1.25-1.63 (4H,m), 1.45 (9H, s), 3.67 (2H, t, J 7.5Hz), 7.02 (2H, d,J 7.5Hz), and 7.40 (2H,d, J 7.5Hz); M⁺ 282.

### PREPARATION 15

### 1-[N-(t-Butyloxycarbonyl)-N-hexylamino]-4-thiopyridone

### a) 1-[N-(t-Butyloxycarbonyl)-N-hexylamino]-4-pyridone

1-(t-Butyloxycarbonyl amino)-4-pyridone (0.2g, 0.94mmol) in N, N dimethylformamide (4ml) was treated successively with potassium carbonate (0.132g, 0.95mmol) and 1-bromohexane (0.203g, 1.22mmol). The mixture was stirred for 18h and then evaporated under reduced pressure. Purification on silica gel 60 eluting with ethanol, dichloromethane (1:9) δ_{H} (CDCl₃) 0.89 (3H,t, J 6Hz), 1.21-1.76 (17H, m), 3.65 (2H,t, J 7.5Hz), 6.35 (2H,d, J 8Hz), and 7.21 (2H,d, J 8Hz); M⁺ 294.

### b) 1-[N-(t-Butyloxycarbonyl)-N-hexylamino]-4-thiopyridone

The product of Preparation 15(a) (0.28g, 0.95mmol) in toluene (10ml) was treated with Lawesson's reagent (0.38g, 0.94mmol) and heated at 80°C for 35 minutes. The mixture was allowed to cool and chromatographed on silica gel 60 eluting with ethanol, dichloromethane (1:9) to give the compound (0.179g, 66%); δ_{H} (CDCl₃) 0.89 (3H,t,J 6Hz), 1.23-1.70 (8H,m), 1.45 (9H,s), 3.67 (2H, t,J 7.5Hz), 7.02 (2H, d,J 7Hz), and 7.4 (2H, d,J 7Hz) M⁺ 310.

### PREPARATION 16

### 1-[N-(t-Butyloxycarbonyl)-N-(1-isopropyl)amino]-4-thiopyridone

### a) 1-[N-(t-Butyloxycarbonyl)-N-(1-isopropyl)amino]-4-pyridone

1-(t-Butyloxycarbonyl amino)-4-pyridone (0.2g, 0.94mmol) in N,N dimethylformamide (4ml) was treated successively with potassium carbonate (0.132g, 0.95mmol) and 2-bromopropane (0.152g, 1.22mmol). The mixture was stirred for 24h and then evaporated under reduced pressure. Purification on silica gel 60 eluting with ethanol, dichloromethane (1:9) gave the title compound (0.116g, 49%); δ_{H} (CDCl₃) 1.21 (6H,d, J 7Hz), 1.44 (9H,s), 4.61 (1H,t, J 7Hz), 6.33 (2H,d, J 8Hz) and 7.15 (2H,d, J 8Hz); M⁺ 252.

### b) 1-[N-(t-Butyloxycarbonyl)-N-(1-isopropyl)amino]-4-thiopyridone

The product of Preparation 16(a) (0.116g, 0.46mmol) in toluene (10ml) was treated with Lawesson's reagent (0.124g, 0.31mmol) and heated at 80°C for 35 minutes. The mixture was allowed to cool and chromatographed on silica gel 60 eluting with ethanol, dichloromethane (1:9) to give the title compound (0.05g, 41%); δ_{H} (CDCl₃) 1.22 (6H, d, J 7Hz), 1.45 (9H,s), 4.65 (1H,t, J 7Hz), 6.96 (2H, d, J 7Hz), and 7.38 (2H, d, J 7Hz) M⁺ 268.

### PREPARATION 17

### 1-[N-(t-Butyloxycarbonyl)-N-(2-hydroxyethyl)amino]-4--thiopyridone

### a) N-(t-Butyloxycarbonyl)-N-(2-hydroxyethyl)hydrazine

2-Hydroxyethylhydrazine (0.228g, 3.0mmol) in dichloromethane (10ml) was treated with di-t-butyldicarbonate (0.65g, 3.0mmol). The reaction mixture was stirred for 0.75h and then evaporated under reduced pressure. Purification on silica gel 60 eluting with ethanol, dichloromethane (1:9) gave the title compound (0.413g, 78%); δ_{H} (CDCl₃) 1.48 (9H,s), 3.58 (2H,t, J 4.5Hz) 3.83 (2H, t, J 4.5Hz), and 3.3-4.0 (1H, brs).

### b) 1-[N-(t-Butyloxycarbonyl)-N-(2-hydroxyethyl)amino]-4 -thiopyridone

N-(t-Butyloxycarbonyl)-N-(2-hydroxyethyl)hydrazine (0.413g, 2.37mmol) in ethanol (10ml) was treated with 4-thiopyranone (0.16g 1.43mmol). The mixture was stirred at 60°C for 18h, then allowed to cool and evaporated under reduced pressure. Purification on silica gel 60 eluting with ethanol, dichloromethane (1:9) gave the title compound (0.351g, 91%); δ_{H} (CDCl₃), 1.47 (9H,s), 3.30-3.95 (5H,m), and 7.32 (4H,d, J 5Hz); M⁺ 270.

### PREPARATION 18

### 2-[(Z)-[S]-(3,4-Diacetoxyphenyl)(diphenylmethyloxycarbo nyl)methyloxyimino]-2-(2-tritylamino-4-thiazolyl)acetic acid and 2-[(Z)-[R]-(3,4-Diacetoxyphenyl)(diphenylmethyloxycarbonyl)methyloxyimino]-2-(2-tritylamino-4-thiazolyl)acetic acid

### a) R-(+)-α-Methylbenzylamine salt of [S]-2-(3,4-diacetoxyphenyl)-2-(phthalimidooxy)acetic acid and S-(-)-α-methylbenzylamine salt of [R]-2-(3,4-diacetoxyphenyl)-2-(phthalimidooxy) acetic acid

Diphenylmethyl [RS]-2-(3,4-diacetoxyphenyl)-2-(phthalimidooxy) acetate (3.8g, 6.56mmol) was dissolved in dichloromethane (100ml), treated with trifluoroacetic acid (10ml) and stirred for 2h. The mixture was diluted with toluene (50ml) and evaporated to dryness under reduced pressure. The residue was triturated under hexane (2x200ml) and then dissolved in acetone (30ml). The resulting solution was treated with R-(+)-α-methylbenzylamine (0.84ml, 6.53mmol) and, after 30 minutes the solid was collected by filtration and washed with acetone, to give the R-(+)-α-methylbenzylamine salt of [S]-2-(3,4-diacetoxyphenyl)-2-(phthalimidooxy) acetic acid (1.05g, 60%); [α]_{D}²¹ + 189° (c 0.05 in EtOH); δ_{H} (CDCl₃) 1.48 (3H, d, J 6Hz), 2.19 (3H, s), 2.23 (3H, s), 4.25 (1H, q, J 6Hz), 5.76 (1H, s), 7.00-7.50 (8H, m), and 7.60-7.70 (4H, s).

The filtrate from above was evaporated under reduced pressure and partitioned between dilute hydrochloric acid and ethyl acetate. The ethyl acetate layer was dried (MgSO₄) and evaporated to dryness under reduced pressure. The residue was dissolved in acetone (15ml) and treated with S-(-)-α-methylbenzylamine (0.69ml, 5.3mmol). After 30 minutes the solid was collected by filtration and washed with acetone to give the S-(-)-α-methylbenzyl amine salt of [R]-2-(3,4-diacetoxyphenyl)-2-(phthalimidooxy) acetic acid (1.07g, 67%); [α]_{D}²¹ -184° (c 0.04 in EtOH); δ_{H} (CDCl₃) 1.50 (3H, d, J 6 Hz), 2.21 (3H, s), 2.24 (3H, s), 4.28 (1H, q, J 6 Hz), 5.78 (1H, s), 7.05-7.50 (8H, m), and 7.70 (4H, s).

### b) Diphenylmethyl [S]-2-(3,4-diacetoxyphenyl)-2-(phthalimidooxy)acetate

R-(+)-α-Methylbenzylamine salt of [S]-2-(3,4-diacetoxyphenyl)-2-(phthalimidooxy)acetic acid (1.31g, 2.45mmol) was partitioned between dilute hydrochloric acid and ethyl acetate. The ethyl acetate later was dried and treated with an excess of diphenyldiazomethane. The solution was evaporated under reduced pressure and the residue purified by chromatography on silic gel 60, eluting with mixtures of hexane and ethyl acetate, to give the title compound (1.4g, 98%); δ_{H} (CDCl₃), 2.21 (6H, s), 6.00 (1H, s), 6.87 (1H, s), 7.00-7.60 (13H, m), and 7.65-7.75 (4H, m).

### c) 2-[(Z)-[S]-(3,4-Diacetoxyphenyl) (diphenylmethyloxycarbonyl)methyloxyimino]-2-(2-tritylamino-4-thiazolyl)acetic acid

Diphenylmethyl [S]-2-(3,4-diacetoxyphenyl)-2-(phthalimidooxy)acetate (1.36g, 2.35mmol) was dissolved in dichloromethane (25ml) and cooled to -60°C. This solution was treated with methylhydrazine (0.125ml, 2.35mmol) and the temperature allowed to rise slowly to +5°C. The mixture was then stirred at ambient temperature for 1h. The solid was removed by filtration and the filtrate evaporated to dryness under reduced pressure. The residue was dissolved in methanol (40ml) and treated with 2-(2-tritylamino-4-thiazolyl)glyoxylic acid (0.95g, 2.3mmol). After 2h, the reaction mixture was evaporated to dryness under reduced pressure. Chromatography on silica gel 60, eluting with mixtures of dichloromethane and ethanol gave the title compound (1.15g, 58%); δ_{H} (CDCl₃) 2.27 (3H, s), 2.29 (3H, s), 6.00 (1H, s), 6.76 (1H, s), 6.85 (1H, s), and 6.90-7.40 (29H, m).

### d) 2-[(Z)-[R]-(Diacetoxyphenyl)(diphenylmethyloxycarbonyl)methyloxyimino]-2-(2-tritylamino-4-thiazolyl) acetic acid

This was prepared from the [S]-(-)-α-methylbenzylamine salt of [R]-2-(3,4-diacetoxyphenyl)-2-(phthalimidooxy)-acetic acid (1.20g, 2.28mmol) as described in Preparation 18 (b) and (c) (0.91g, 47%), δ_{H} (CDCl₃), 2.27 (3H, s), 2.29 (3H, s), 6.01 (1H, s), 6.79 (1H, s), 6.85 (1H, s), and 6.90-7.40 (29H, m).

### PREPARATION 20

### 1-(2-Pyridylamino)-4-thiopyridone

4-Thiopyranone (0.112g, 1mmol) and 2-hydrazinopyridine (0.109g, 1mmol) in ethanol (5ml) was heated at reflux for 7h, cooled, evaporated to dryness and the residue chromatographed to afford the title compound (0.035g, 17%); υₘₐₓ (KBr) 3430, 1616 and 1572cm⁻¹; δ_{H} (CDCl₃ + (CD₃)₂SO) 6.59 (1H, d, J 8.3Hz), 6.91 (1H, dd, J 6.6 and 7.6Hz), 7.33 and 7.42 (4H, 2ABq, J 7.2Hz), 7.58 (1H, m), 8.18 (1H, d, J 4.6 Hz), and 10.12 (1H, br s, exch.); M⁺ 203.

### PREPARATION 21

### 1-(3,5-Dimethylisoxazol-4-yl)methylamino-4-thiopyridone

### a) 1-[N-(t-Butyloxycarbonyl)-N-[(3,5-dimethylisoxazol-4-yl)methyl]amino]-4-pyridone

1-(t-Butyloxycarbonylamino)-4-pyridone (0.105g, 0.5mmol) in N,N-dimethylformamide (2.5ml) containing 4-chloromethyl-3,5-dimethylisoxazole (0.072g, 0.5mmol) was treated with powdered potassium carbonate (0.07g, 0.5mmol). The reaction was stirred for 2h, diluted with dichloromethane (20ml) and washed with water (x2), brine, dried (Na₂SO₄) and evaporated. The residue was chromatographed to afford the title compound (0.142g, 90%); υₘₐₓ (KBr) 1718, 1646, 1628 and 1586cm⁻¹; ^{δ}H (CDCl₃) 1.47 (9H, s), 2.18 (3H, s), 2.20 (3H, s), 4.62 (2H, s), 6.29 (2H, d, J 7.8Hz), and 6.96 (2H, d, J 7.8Hz); M⁺ 319.

### b) 1-[N-(t-Butyloxycarbonyl)-N-[(3,5-dimethylisoxazol-4-yl)methyl]amino]-4-thiopyridone

To a solution of the product from preparation 21(a) (0.133g, 0.42mmol) in dry toluene (5ml) at 80°C was added Lawessons reagent (0.084g, 0.21mmol). The mixture was stirred for 20 minutes, cooled and the solution decanted and chromatographed on silica gel to afford the title product (0.90g, 64%); υₘₐₓ (KBr) 1728, and 1616cm⁻¹; δ_{H} (CDCl₃) 1.47 (9H, s), 2.18 (3H, s), 2.19 (3H, s), 4.63 (2H, s), 6.76 (2H, d, J 7.4Hz), and 7.31 (2H, d, J 7.4 Hz); M⁺ 335.

### c) 1-(3,5-Dimethylisoxazol-4-yl)methylamino-4-thiopyridone

To the product from preparation 21(b) (0.083g, 0.35mmol) in dichloromethane (5.2ml) was added trifluoroacetic acid (0.57ml, 10.5mmol) and the mixture stirred for 3h. The solution was evaporated to low volume and added dropwise to vigorously stirred ether (50ml). The precipitate was filtered off, washed with ether and dried in vacuo to afford the title product (0.033g, 57%); υₘₐₓ (KBr) 1611 and 1530cm⁻¹; δ_{H} (CDCl₃ + (CD₃)₂SO) 2.18 (3H, s), 2.24 (3H, s), 3.95 (2H, s), 7.31 (2H, d, J 7 Hz), and 7.38 (2H, d, J 7 Hz); M⁺ 235.

### PREPARATION 22

### 1-(N-Acetyl-N-methylamino)-4-thiopyridone

1-(Methylamino)-4-thiopyridone(0.21g, 1.5mmol) in dichloromethane (20ml) was treated with triethylamine (0.21ml, 1.5mmol) and then acetic anhydride (0.15ml, 1.6mmol) and stirred for 5 minutes. The product was obtained by purification on silica gel 60 eluting with 2% ethanol in dichloromethane (0.17g, 62%); υₘₐₓ (CH₂Cl₂) 1700 and 1615cm⁻¹; δH (CDCl₃) 2.02 (3H, s), 3.40 (3H, s), 7.08 (2H,d, J 7.5Hz), and 7.41 (2H, d, J 7.5Hz); M⁺ 182.

### PREPARATION 23

### 1-[N-Methyl-N-(4-nitrobenzoyl)amino]-4-thiopyridone

1-Methylamino-4-thiopyridone (0.05g, 0.36mmol) in dichloromethane (2ml) was treated with triethylamine (0.05ml, 0.36mmol) followed by p-nitrobenzoyl chloride (0.066g, 0.36mmol) dissolved in dichloromethane (5ml) and stirred for 0.5h. Purification on silica gel 60 eluting with methanol and dichloromethane mixtures gave the title compound (0.092mg, 89%); δH [(CD₃)₂SO] 3.36 (3H, s), 7.8 (2H, brs), 7.96 (2H, s), 7.99 (2H, s), and 8.35 (2H, brs); M⁺ 289.

### PREPARATION 24

### 1-[N-(4-Methoxybenzoyl)-N-methylamino]-4-thiopyridone

Reaction of 1-methylamino-4-thiopyridone (0.078g, 0.56mmol), triethylamine (0.056g, 0.56mmol) and p-anisoyl chloride (0.095g, 0.56mmol) in tetrahydrofuran (25ml) for 0.5h followed by purification on silica gel 60 eluting with mixtures of methanol and dichloromethane gave the title compound (0.148g, 96%); υₘₐₓ (CHCl₃) 1670 and 1615cm⁻¹; δH (CDCl₃) 3.49 (3H, s), 3.80 (3H, s), 6.86 (2H, d, J 8 Hz), 7.11 and 7.3 (4H, 2d, J 7Hz), and 7.44 (2H, d, J 8Hz); M⁺ 274.

### PREPARATION 25

### 1-[N-(2-Furoyl)-N-methylamino]-4-thiopyridone

Reaction of 1-methylamino-4-thiopyridone (0.090g, 0.64mmol), triethylamine (0.09ml, 0.64mmol) and 2-furoyl chloride (0.084g, 0.64mmol) in dichloromethane (15ml) for 0.5h followed by purification on silica gel 60 eluting with mixtures of methanol, dichloromethane gave the title compound (0.138g, 92%); υₘₐₓ (CHCl₃) 1660 and 1615cm⁻¹; δH (CDCl₃/CD₃OD) 3.55 (3H, s), 6.46 (1H, dd, J 2 and 4Hz), 6.90 (1H, d, J 4Hz), 7.29 (2H, s), 7.38 (2H, s), and 7.45 (1H, brs); M⁺ 234.

### PREPARATION 26

### 1-[N-[3,4-bis(4-Methoxybenzyloxy)benzoyl]-N-methylamino]-4-thiopyridone

### a) 4-Methoxybenzyl 3,4-bis(4-methoxybenzyloxy)-benzoate.

3,4-Dihydroxybenzoic acid (3.08g, 0.02mol) was dissolved in N,N-dimethylformamide (50ml) and treated with 4-methoxybenzyl chloride (10ml, 0.07mol) and potassium carbonate (10g, 0.07mol). The mixture was warmed to 60°C for 6h and then stirred overnight. The mixture was partitioned between water and ethyl acetate. The organic phase was washed exhaustively with water and then the product was purified on silica gel 60 eluting with mixtures of ethyl acetate and hexane to give the title compound (6.48g, 63%).

### b) 3,4-bis(4-Methoxybenzyloxy)benzoic acid.

4-Methoxybenzyl 3,4-bis(4-methoxybenzyloxy)benzoate (6.48g, 0.013mol) was suspended in ethanol and treated with 2.5N aqueous sodium hydroxide solution (7.6ml, 0.015mol). The mixture was warmed to 60°C for 4h. The mixture was evaporated to low volume and then partitioned between water and ethyl acetate. The aqueous layer was washed again with ethyl acetate and then acidified and extracted into ethyl acetate. As the ethyl acetate solution was concentrated the product precipitated from solution and was filtered off to give the title compound (4.36g, 87%).

### c) N-[3,4-bis(4-Methoxybenzyloxy)benzoyl)-N-methylhydrazine.

3,4-bis-(4-Methoxybenzyloxy)benzoic acid (0.792g, 2.0mmol) was dissolved in dichloromethane (20ml) and treated with N,N-diisopropylethylamine (0.35ml, 2.0mmol). The solution was cooled to -40°C and treated with methanesulphonyl chloride (0.15ml, 2.0mmol). The mixture was allowed to warm to room temperature for 10 minutes and then re-cooled to -40°C and added to a solution of methylhydrazine (0.215ml, 4mmol) in dichloromethane (10ml) at -40°C. The mixture was allowed to warm to room temperature for 1.5h and then evaporated to dryness under reduced pressure. The residue was dissolved in ethyl acetate which was washed with water and brine. Evaporation of the solvent gave the title compound (0.823g, 94%); δ_{H} (CDCl₃) 3.08 (3H, s), 3.74 (6H, S), 4.25 (2H, brs), 5.03 (4H, S), and 6.70-7.40 (11H, m).

### d) 1-[N-[3,4-bis(4-Methoxybenzyloxy)benzoyl]-N-methylamino]-4-thiopyridone

N-[3,4-bis-(4-Methoxybenzyloxy)benzoyl]-N-methylhydrazine (0.80g, 1.8mmol) was heated at reflux in ethanol (60ml) with 4-thiopyranone (0.224g, 2.0mmol) for 48 hours. After evaporation under reduced pressure, purification on silica gel 60 gave the title compound (0.404g; 43% ); υₘₐₓ. (KBr) 1656, 1612, and 1513 cm⁻¹; δH (CDCl₃) 3.40 (3H, S), 3.88 (6H, s), 5.02 (2H, s), 5.07 (2H, s), 6.90-7.30 (7H, m), 6.85 (2H, d), J 9Hz), and 7.30 (2H, d, J 9Hz); M⁺ 516.

### PREPARATION 27

### 1-(N-Benzoyl-N-methylamino)-4-thiopyridone

### a) N-Benzoyl-N-methylhydrazine

Methylhydrazine (0.212ml, 4.0mmol) was dissolved in dichloromethane (10ml) and was treated dropwise with benzoylchloride (0.232ml, 2.0mmol) in dichloromethane (5ml). After stirring for 0.5h the solution was evaporated to dryness. The residue was partitioned between water and ethyl acetate. The organic phase was dried and evaporated under reduced pressure to give the title compound (0.20g, 65%); δ_{H} (CDCl₃) 3.13 (3H, s), 4.5 (2H, brs), and 7.30 (5H, s).

### b) 1-(N-Benzoyl-N-methylamino)-4-thiopyridone.

N-Benzoyl-N-methylhydrazine (0.20g, 1.3mmol) in ethanol (10ml) was heated at reflux for 28h with 4-thiopyranone (0.15g, 1.3mmol). The solvent was evaporated and the product purified by chromatography on silica gel eluting with mixtures of ethyl acetate and hexane to give the title compound (0.114g, 36%); δH (CDCl₃) 3.50 (3H, s), 7.09 (2H, d, J 8Hz), 7.12 (2H, d, J 8Hz), and 7.43 (5H, s).

### PREPARATION 28

### 1-[N-[3,4-bis(4-Methoxybenzyloxy)cinnamoyl]-N-methylamino]-4-thiopyridone

### a) N-[3,4-bis(4-Methoxybenzyloxy)cinnamoyl]-N-methylhydrazine

The title compound was obtained by the method of Preparation 26 using 3,4-dihydroxycinnamic acid in place of 3,4-dihydroxybenzoic acid; δ_{H} (CDCl₃) 3.25 (3H, s), 3.86 (6H, s), 3.95 (2H, brs), 5.05 (4H, s), and 6.65-6.80 (13H, m).

### b) 1-[N-[3,4-bis(4-Methoxybenzyloxy)cinnamoyl]-N-methylamino]-4-thiopyridone

N-[3,4-bis(4-Methoxybenzyloxy)cinnamoyl]-N-methylhydrazine (0.225g, 0.54mmol) in ethanol (15ml) containing 4-thiopyranone (0.056g, 0.48mmol) was heated at reflux for 96h. Purification on silica gel 60 eluting with mixtures of ethyl acetate and hexane gave the title compound (0.86g, 35%); δ_{H} (CDCl₃) 3.36 (3H, s), 3.74 (6H, s), 4.98 (2H, s), 5.02 (2H, s), 5.94 (1H, d, J 15Hz), 6.75-7.45 (15H, m), and 7.63 (1H, d, J 15Hz).

### PREPARATION 29

### 1-Ureido-4-thiopyridone

4-Thiopyranone (0.265g, 2.37mmol), semicarbazide hydrochloride (0.265g, 2.37mmol), and triethylamine (0.4ml, 5.4mmol) in ethanol (20ml), were heated at reflux for 24h then cooled. The white precipitate was filtered off, washed with ethanol and then dried in vacuo to give the title compound (0.224g, 60%); δ_{H} [(CD₃)₂SO], 6.62 (2H, brs), 7.13 (2H, d, J 9Hz), and 7.51 (2H, d, J 9Hz).

### PREPARATION 30

### 1-(1,3-Dimethylureido)-4-thiopyridone

4-Thiopyranone (0.267g, 2.38mmol) and 1,3-dimethylsemicarbazide (0.245g, 2.38mmol) were heated at reflux in ethanol (10ml) for 45h. The mixture was evaporated and the residue was purified on silica gel 60 eluting with dichloromethane, methanol mixtures to give the title compound as a white solid (0.333g, 71%); m/z (F.A.B., thioglycerol) M⁺ 197.

### PREPARATION 31

### 1-(1-Methylureido)-4-thiopyridone

4-Thiopyranone (0.143g, 1.28mmol) and 2-methylsemicarbazide (0.114g, 1.28mmol) were heated at reflux in ethanol (10ml) for 48h. The reaction mixture was evaporated and the residue was purified on silica gel 60 eluting with acetone to give the title compound as a white solid (0.031g, 13%); ^{δ}H [(CD₃)₂SO] 3.27 (3H, s), 6.74 (2H, s, exch. with D₂O), 7.13 (2H, d, J 6Hz), and 7.69 (2H, d, J 6Hz).

### PREPARATION 34

### 1-[N-(4-Diphenylmethoxycarbonylbenzoyl)-N-methylamino]-4-thiopyridone

4-(Diphenylmethoxycarbonyl)benzoic acid (0.161g, 0.5mmol) was dissolved in dry dichloromethane (20ml) and treated with oxalyl chloride (0.1ml, 1.15mmol) and N,N-dimethylformamide (0.01ml). After 1h, the reaction mixture was evaporated to dryness under reduced pressure. The residue was redissolved in dry dichloromethane (10ml) and added to a solution of 1-(methylamino)-4-thiopyridone (0.07g, 0.5mmol) and triethylamine (0.0505g, 0.5mmol) in acetonitrile (10ml). After 15 minutes at room temperature, the volatiles were removed under reduced pressure and the residue purified on silica gel 60, eluting with dichloromethane then ethyl acetate, to give the title compound (0.185g, 81%); υₘₐₓ (KBr) 1715, 1677 and 1615cm⁻¹; δ_{H} [(CD₃)₂SO] 5.44 (3H, s), 7.05 (1H, s), 7.24-7.59 (16H, m), and 7.96 (2H, d, J 7Hz); (Found: M⁺ 454.1358. C₂₇H₂₂N₂O₃S requires M, 454.1351).

### PREPARATION 35

### 1-[N-[4-(t-Butoxycarbonylamino)benzoyl]-N-methylamino]-4-thiopyridone

1-[4-(t-Butyloxycarbonylamino)benzoyl]-1-methylhydrazine (0.205g, 0.77mmol) and 4-thiopyranone (0.087g, 0.78mmol) were heated at reflux in ethanol (20ml) for 16h, then the mixture left to stand at room temperature for 90h. The volatiles were removed under reduced pressure and the residue purified on silica gel 60, eluting with dichloromethane then mixtures of ethyl acetate and hexane, to give the title compound (0.09g, 32%); υₘₐₓ (KBr), 1725, 1670 and 1612cm⁻¹; δ_{H} (CDCl₃ + CD₃OD) 1.51 (9H, s), 3.52 (3H, s), 7.35-7.50 (8H, m), and 8.48 (1H, brs, exch.) (Found: M⁺, 359.1311. C₁₈H₂₁N₃O₃S requires M, 359.1304).

### PREPARATION 36

### 4-Methoxybenzyl [6R,7R]-7-amino-3-[1-(methylamino)-pyridinium-4-thiomethyl]ceph-3-em-4-carboxylate iodide

4-Methoxybenzyl [6R,7R]-7-amino-3-(chloromethyl)ceph-3-em-4-carboxylate hydrochloride (2.4g, 6mmol) was partitioned between dilute, aqueous sodium hydrogen carbonate and ethyl acetate. The organic phase was washed with brine and evaporated to dryness under reduced pressure. The residue was dissolved in acetonitrile (20ml) and treated with sodium iodide (0.75g, 5mmol). After 5 minutes 1-(methylamino)-4-thiopyridone (0.7g, 5mmol) was added and the mixture stirred for 1.5h. The mixture was filtered into vigorously stirred diethylether (500ml) and the product collected by filtration (2.2g, 72%); υₘₐₓ (KBr) 1772, 1718, 1618, and 1513cm⁻¹; δ_{H} (CDCl₃) 3.07 (3H, d, J 6Hz), 3.49 and 3.73 (2H, ABq, J 18Hz), 3.81 (3H, s), 4.29 and 4.45 (2H, ABq, J 13Hz), 4.79 (1H, d, J 5Hz), 4.97 (1H, d, J 5Hz), 5.20 and 5.28 (2H, ABg, J 12Hz), 6.87 (2H, d, J 8.5Hz), 7.36 (2H, d, J 8.5Hz), 7.73 (2H, d, J 7Hz), 8.51 (1H, q, J 6Hz, exch.), and 8.89 (2H, d, J 7Hz); m/z (F.A.B., thioglycerol) M⁺ 473.

### PREPARATION 37

### 2-(Z)-[3,4-(Methylenedioxy)benzyloxyimino]-2-(2-tritylamino-4-thiazolyl)acetic acid

2-(2-Tritylamino-4-thiazolyl)glyoxylic acid (1.61g, 3.89mmol) was suspended in methanol (50ml) and treated with 3,4-(methylenedioxy)benzyloxyamine (0.65g, 3.89mmol). After 2h, the volatiles were removed under reduced pressure and the residue partitioned between ethyl acetate (50ml) and saturated, aqueous sodium hydrogen carbonate solution (50ml). The phases were separated, the aqueous phase was washed with ethyl acetate (50ml) and acidified with 5M hydrochloric acid in the presence of ethyl acetate (50ml). The phases were separated and the organic phase washed with water (20ml), saturated brine (20ml), dried over anhydrous magnesium sulphate and evaporated to dryness under reduced pressure to give the title compound (2.01g, 92%); υₘₐₓ (KBr) 1731cm⁻¹; δ_{H} [(CD₃)₂CO] 5.04 (2H, brs), 6.94 (2H, s), 6.79-6.95 (4H, m), and 7.18-7.50 (15H, m); m/z (F.A.B., 3-nitrobenzyl alcohol/sodium acetate) MNa⁺ 586.

### PREPARATION 38

### 1-(t-Butyloxycarbonylamino)-5-methoxy-2-(methoxymethyl)-4-thiopyridone

### a) 5-Methoxy-2-(methoxymethyl)-4-thiopyranone

5-Methoxy-2-(methoxymethyl)-4-pyranone (3.24g, 19mmol) (K. Heyns and G. Vogelsang, Chem. Ber. 1954, 87, 1377) in toluene (60ml) was heated at 80°C with Lawessons reagent (4.23g, 10mmol) for 2h. After cooling the reaction mixture was chromatographed on silica gel 60 eluting with toluene/ethyl acetate mixtures to give the title compound (3.54g, 100%); υₘₐₓ (CH₂Cl₂) 3050, 2950, 2830, 1625, and 1560cm⁻¹; δ_{H} (CDCl₃) 3.4 (3H, s), 3.8 (3H, s), 4.2 (2H, s), 7.3 (1H, s), 7.4 (1H, s); M⁺186.

### b) 1-(t-Butyloxycarbonylamino)-5-methoxy-2-(methoxymethyl)-4-thiopyridone

A solution of the product from Preparation 38(a) (500mg, 0.27mmol) and t-butylcarbazate (350mg, 0.27mmol) in ethanol (10ml) was heated at reflux for 18h. The solvent was evaporated under reduced pressure and the residue chromatographed on silica gel 60 eluting with 5% ethanol in dichloromethane to give the title compound (400mg, 50%) as a brown foam; υₘₐₓ (CH₂Cl₂) 3350, 3050, 2970, 1745, and 1605cm⁻¹; δ_{H} (CDCl₃) 1.45 (9H, s), 3.35 (3H, s), 3.75 (3H, s), 4.25 (2H, s), 6.8 (1H, s), 7.4 (1H, s); M⁺ 300.

### PREPARATION 39

### 1-(t-Butyloxycarbonylamino)-3-methoxy-2-methyl-4-thiopyridone

### a) 3-Methoxy-2-methyl-4-pyranone

Dimethylsulphate (3.8ml, 5.05g, 0.04mmol) was added to a solution of maltol (5.0g, 0.04mmol) in 10% aqueous potassium hydroxide (22.5ml). After stirring for 5h the reaction mixture was extracted into CH₂Cl₂ (x3). The organic extract was dried (MgSO₄) and concentrated under reduced pressure. The residue was chromatographed on silica gel 60 eluting with 60% ethyl acetate in hexane to give the title compound (2.06g, 37%) as a pale yellow oil; υₘₐₓ (film) 3080, 1620, and 1580cm⁻¹; δ_{H} (CDCl₃) 2.3 (3H, s), 3.8 (3H, s), 6.3 (1H, d, J 6Hz), 7.6 (1H, d, J 6Hz).

### b) 3-Methoxy-2-methyl-4-thiopyranone

The product from Preparation 39(a) (2.05g, 14.6mmol) in toluene (30ml) was heated at 80°C with Lawessons reagent (3.25g, 8.0mmol) for 1.5h. After cooling the reaction mixture was chromatographed on silica gel 60 eluting with toluene/ether mixtures to give the title compound (2.07g, 91%) as a brown foam; υₘₐₓ (CH₂Cl₂) 3030, 1750, 1620, and 1550cm⁻¹; δ_{H} (CDCl₃) 2.3 (3H, s), 3.8 (3H, s), 7.1 (1H, d, J 5Hz), 7.35 (1H, d, J 5Hz); M⁺ 156.

### c) 1-(t-Butyloxycarbonylamino)-3-methoxy-2-methyl-4-thiopyridone

A solution of the product from Preparation 39(b) (2.03g, 13mmol) and t-butylcarbazate (1.72g, 13mmol) in ethanol (30ml) was heated at reflux for 64h. Further t-butylcarbazate was then added to the reaction mixture and reflux continued for a further 36h before allowing the reaction to stand at room temperature for 40h. The solvent was evaporated under reduced pressure and the residue chromatographed on silica gel 60 eluting with dichloromethane/ethanol mixtures to give the title compound (2.65g) as a yellow foam; υₘₐₓ (CH₂Cl₂) 1740, and 1605cm⁻¹.

### PREPARATION 40

### 1-[N-(t-Butyloxycarbonyl)-N-(2-methoxyethyl)amino]-4-thiopyridone

1-(t-Butyloxycarbonylamino)-4-thiopyridone (0.24g, 1.06mmol) in N,N-dimethylformamide (5ml) was treated with potassium carbonate (0.15g, 1.0mmol) and bromoethyl methyl ether (0.2ml, 2.lmmol). The mixture was stirred for 2h, then evaporated under reduced pressure. Purification on silica gel 60 eluting with ethanol, dichloromethane mixtures gave the title compound (0.028g, 9%); δ_{H} (CDCl₃) 1.45 (9H, s), 3.35 (3H, s), 3.52 (2H, t, J 5Hz), 3.87 (2H, t, J 5Hz), 7.17 (2H, d, J 7Hz), and 7.40 (2H, d, J 7Hz).

### PREPARATION 42

### 1-[(6-Chloropyridin-2-yl)amino]-4-thiopyridone

6-Chloro-2-hydrazinopyridine (0.143g, 1.0mmol) and 4-thiopyranone (0.112g, 1.0mmol) were heated together at reflux in ethanol (15ml). After 18h the solution was allowed to cool and evaporated to dryness under reduced pressure. Purification of the residue on silica gel 60 eluting with ethanol, dichloromethane mixtures gave the title compound (0.031g, 13%); δ_{H} (CDCl₃ + CD₃OD) 6.48 (1H, d, J 8Hz), 6.97 (1H, d, J 8Hz), 7.44 (2H, d, J 7Hz), 7.53 (2H, d, J 7Hz), and 7.60 (1H, t, J 8Hz); M⁺ 237.

### PREPARATION 43

### 1-(Pyrazineamino)-4-thiopyridone

### a) Hydrazinopyrazine

A mixture of chloropyrazine (1.0g, 11.56mmol) and hydrazinehydrate (3.0g, 93.7mmol) was refluxed for 1h to obtain a bright yellow solution. The solution was then cooled and maintained at 4°C for 18h. The precipitate was filtered, washed with hexane (20ml) and dried in vacuo to give the title compound (0.24g, 16%); δ_{H} (CDCl₃) 3.85 (2H,s), 6.01 (1H, s), 7.91-7.93 (1H, m), 8.02-8.04 (1H, m), and 8.21 (1H, s); M⁺ 110.

### b) 1-(Pyrazineamino)-4-thiopyridone

Hydrazino pyrazine (0.24g, 2.26mmol) in ethanol (10ml) was treated with 4-thiopyranone (0.2g, 1.78mmol). The mixture was refluxed for 6h, cooled and evaporated under reduced pressure. Purification on silica gel 60 eluting with dichloromethane ethanol (9:1) gave the title compound (0.024g, 5%); M⁺ 204.

### PREPARATION 44

### 1-[N-(t-Butyloxycarbonyl)-N-(2-methyl-4-thiazolyl)-methylamino]-4-thiopyridone

### a) 1-[N-(t-Butyloxycarbonyl)-N-(2-methyl-4-thiazolyl)-methylamino]-4-pyridone

1-(t-Butyloxycarbonylamino)-4-pyridone (0.1g, 0.47mmol) in N,N-dimethylformamide (2ml) was treated successively with 4-(chloromethyl)-2-methylthiazole hydrochloride (0.07g, 0.47mmol) and potassium carbonate (0.131g, 0.95mmol). The mixture was stirred at room temperature for 18h, followed by evaporation to dryness under reduced pressure. Purification on silica gel 60 eluting with dichloromethane and ethanol (9:1) gave the title compound (0.064g, 42%); δ_{H} (CDCl₃) 1.45 (9H, s), 2.71 (3H, s), 4.86 (2H, s), 6.26 (2H, d, J 8Hz), 7.00 (1H, s), and 7.20 (2H, d, J8Hz) ; MH⁺ 322.

### b)1-[N-(t-butyloxycarbonyl)-N-(2-methyl-4-thiazolyl)methylamino]-4-thiopyridone

The product of Preparation 44(a) (0.064g, 0.2mmol) in toluene (5ml) was treated with Lawesson's reagent and the mixture was stirred at 80°C for 40 minutes. The mixture was then cooled and purified on silica gel 60 eluting with dichloromethane and ethanol (9:1) to give the title compound (0.041g, 61%); M⁺ 337.

### PREPARATION 45

### 1-[(2-Imidazolin-2-yl)amino]-4-thiopyridone

2-Hydrazino-2-imidazoline hydrobromide (0.181g, 1.0mmol) in ethanol (10ml) was treated with triethylamine (0.1g, 1.0mmol) and 4-thiopyranone (0.112g, 1.0mmol). The reaction mixture was heated at reflux for 7h and stirred at room temperature for 18h. The mixture was then evaporated to dryness under reduced pressure. Purification on silica gel 60 eluting with dichloromethane and ethanol mixtures gave the title compound (0.03g, 15%); δ_{H} [(CD₃)₂CO], 3.59 (4H, s), 6.02 (1H, br s), 6.33 (1H, br s), 7.17 (2H, d, J 7Hz), and 7.26 (2H, d, J 7Hz); M⁺ 194.

### PREPARATION 46

### 1-(Phthalazin-1-ylamino)-4-thiopyridone

Hydralazine hydrochloride (0.196g, 1.0mmol) in ethanol (10ml) was treated with triethylamine (0.1ml, 1.0mmol) and 4-thiopyranone (0.112g, 1.0mmol). The reaction mixture was refluxed for 5h, followed by stirring at room temperature for a further 72h. The mixture was evaporated to dryness under reduced pressure. Purification on silica gel 60 eluting with dichloromethane and ethanol mixtures gave the title compound (0.055g, 22%); δ_{H} 7.32 (2H, d, J 7Hz), 7.64 (2H, d, J 7Hz), 7.77-7.90 (3H, m), 8.25 (1H, s), and 8.42 (1H, d, J 8Hz); M⁺ 254.

### PREPARATION 47

### 1-N-(t-Butyloxycarbonyl-N-cyanomethyl)amino]-4-thiopyridone

### a) 1-[N-(t-Butyloxycarbonyl)-N-(cyanomethyl)-amino]-4-pyridone

1-(t-Butyloxycarbonylamino)-4-pyridone (0.1g, 0.47mmol) in N,N-dimethylformamide (2ml) was treated successively with potassium carbonate (0.064g, 0.47mmol) and bromoacetonitrile (0.056g, 0.47mmol). The reaction mixture was stirred at room temperature for 2h, filtered and evaporated under reduced pressure. Purification on silica gel 60 eluting with ethanol, dichloromethane (1:9) gave the title compound (0.106g, 90%); δ_{H} (CDCl₃) 1.49 (9H, s), 4.59 (2H, s), 6.37 (2H, d, J 8Hz), and 7.32 (2H, d, J 8Hz); M⁺ 249.

### b) 1-[N-(t-Butyloxycarbonyl)-N-(cyanomethyl)-aminol-4-thiopyridone

The product of Preparation 47(a) (0.1g, 0.4mmol) in toluene (10ml) was treated with Lawesson's reagent (0.15g, 0.37mmol) and treated at 80°C for 40 minutes. The mixture was allowed to cool and was purified on silica gel 60 eluting with ethanol dichloromethane mixtures to give the title compound (0.053g, 50%); δ_{H} (CDCl₃) 1.50 (9H, s), 4.6 (2H, s), 7.1 (2H, d, J 7.5Hz), and 7.30 (2H, d, J 7.5Hz); M⁺ 265.

### PREPARATION 48

### a) 1-[N-(t-Butyloxycarbonyl)-N-(prop-2-yn-1-yl)-amino]-4-pyridone

1-(t-Butyloxycarbonylamino)-4-pyridone (0.1g, 0.47mmol) in N,N-dimethylformamide (2ml) was treated successively with potassium carbonate (0.064g, 0.47mmol) and propargyl bromide (0.056g, 0.47mmol). The reaction mixture was stirred at room temperature for 2h and evaporated under reduced pressure. Purification on silica gel 60 eluting with dichloromethane gave the title compound (0.093g, 79%); δ_{H} (CDCl₃) 1.47 (9H, s), 2.43 (1H, t, J 2Hz), 4.45 (2H, d, J 2Hz), 6.35 (2H, d, J 8Hz), and 7.35 (2H, d, J 8Hz); M⁺ 248.

### b) 1-[N-(t-Butyloxycarbonyl)-N-(prop-2-yn-1-yl)-amino]-4-thiopyridone

The product of Preparation 48(a) (0.082g, 0.33mol) in toluene (10ml) was treated with Lawesson's reagent (0.133g, 0.33mmol) and heated at 80°C for 0.5h. The mixture was allowed to cool and chromatographed on silica gel 60 eluting with dichloromethane to give the title compound (0.048g, 55%); δ_{H} (CDCl₃), 1.48 (9H, s), 2.46 (1H, t, J 2.4Hz), 4.47 (2H, d, J 2.4Hz), 7.15 (2H, d, J 7.5Hz), and 7.39 (2H, d, J 7.5Hz); MH⁺ 265.

### PREPARATION 49

### 1-(t-Butyloxycarbonylamino)-2,6-dimethyl-4-thiopyridone

### a) 2,6-Dimethyl-4-thiopyranone

2,6-Dimethyl-4-pyranone (0.25g, 2.0mmol) in toluene (10ml) was treated with Lawesson's reagent (0.22g, 1.1mmol) and heated at 100°C for 3h. A further quantity of Lawesson's reagent (0.22g, 1.1mmol) was added and the mixture heated at 100°C for 0.75h. The mixture was allowed to cool, then purified on silica gel 60 eluting with ethanol and dichloromethane (1:9) to give the title compound (0.28g, 98%).

### b) 1-(t -Butyloxycarbonylamino)-2,6-dimethyl-4-thiopyridone

2,6-Dimethyl-4-thiopyranone (0.14g, 1.0mmol) and t-butylcarbazate (0.26g, 2.0mmol) were heated together at reflux in ethanol for 48h. A little acetone was added to the mixture which was then evaporated to dryness under reduced pressure. Purification of the residue on silica gel 60 eluting with ethanol and dichloromethane (1:50) gave the title compound (0.089g, 35%); υₘₐₓ (CH₂Cl₂) 1735 and 1615cm⁻¹; δ_{H} (CDCl₃) 1.50 (9H, s), 2.24 (6H, s), and 7.01 (2H, s); M⁺ 254.

### EXAMPLE 23

Sodium [6R,7R]-7-[2-(2-amino-4-thiazolyl)-2-(Z)-[[R,S]-carboxy(3,4-dihydroxyphenyl)methyloxyimino]-acetamido]-3-[1-(methylamino)pyridinium-4-thiomethyl]ceph-3-em-4-carboxylate

### a) 4-Methoxybenzyl [6R,7R] 3-(chloromethyl)-7-[2-(Z)-[R,S]-(3,4-diacetoxyphenyl)(diphenylmethyloxycarbonyl)-methyloxyimino]-2-(2-tritylamino-4-thiazolyl)-acetamido]ceph-3-em-4-carboxylate

2-[(Z)-[R,S](3,4-Diacetoxyphenyl)(diphenylmethyloxycarbonyl)methyloxyimino]-2-(2-tritylamino-4-thiazolyl)-acetic acid (0.8g, 0.95mmol) was dissolved in dichloromethane (16ml) and treated with N,N-diisopropylethylamine (0.25ml, 1.43mmol). Methanesulphonyl chloride (0.11ml, 1.40mmol) was added dropwise to the stirred solution. After 1h, the mixture was treated dropwise with a solution of 4-methoxybenzyl [6R,7R]-7-amino-3-(chloromethyl)ceph-3-em-4-carboxylate hydrochloride (0.85g, 2.1mmol) and N,N-diisopropylethylamine (0.72ml, 4.2mmol) in dichloromethane (20ml). The mixture was stirred for 1.5h and evaporated to dryness. The residue was partitioned between ethyl acetate and water and the organic phase washed with dilute citric acid and brine, then dried and evaporated under reduced pressure. The residue was purified on silica gel 60, eluting with hexane, ethyl acetate mixtures, to give the title compound (0.85g, 72%); δ_{H} (CDCl₃ + D₂O) 2.23, 2.26, 2.29 and 2.30 (each 3H, 4s), 3.15 and 3.33 (2H, ABq, J 18 Hz), 3.33 and 3.47 (2H, ABq, J 18 Hz), 3.81 (6H, s), 4.00 and 4.55 (2H, ABq, J 12 Hz), 4.00 and 4.62 (2H, ABq, J 12 Hz), 4.90 (1H, d, J 5 Hz), 4.97 (1H, d, J 5 Hz), 5.20 and 5.26 (each 2H, 2 ABq, J 12 Hz), 5.8-5.9 (2H, m), 6.01 (1H, s), 6.10 (1H, s), 6.76 (2H, s), 6.8-7.4 (approx 66H, m), 8.18 (1H, brs), and 8.24 (1H, brs); m/z (F.A.B., 3-nitrobenzyl alcohol, sodium acetate) MNa⁺ 1218.

### b) 4-Methoxybenzyl [6R,7R]-7-[2-(Z)[[R,S]-(3,4-diacetoxyphenyl)(diphenylmethyloxycarbonyl)methyloxyimino]-2-(2-tritylamino-4-thiazolyl)acetamido]-3-[1-(methylamino)pyridinium-4-thiomethyl]ceph-3-em-4-carboxylate iodide

4-Methoxybenzyl [6R,7R]-3-(chloromethyl)-7-[2-(Z)-[[R,S]-(3,4-diacetoxyphenyl)(diphenylmethyloxycarbonyl)methyloxyimino]-2-(2-tritylamino-4-thiazolyl)-acetamido]ceph-3-em-4-carboxylate (0.2g, 0.17mmol) was dissolved in acetonitrile (10ml) and treated with sodium iodide (0.025g, 0.17mmol). After 5 minutes 1-(methylamino)-4-thiopyridone (0.025g, 0.18mmol) was added and the mixture stirred for 3 h. The mixture was added dropwise to stirred diethyl ether (70ml). The solid was collected by filtration, washed with dry ether and water and dried at reduced pressure over KOH overnight (0.15g, 65%); υₘₐₓ (KBr) 1775, 1724, 1684, 1617, and 1513cm⁻¹; δ_{H} (CDCl₃ + D₂O) 2.21, 2.26, 2.29 and 2.31 (each 3H, 4s), 3.00 and 3.02 (each 3H, 2s), 3.16 and 3.25 (2H, ABq, J 16Hz), 3.25 (2H, s), 3.81 (6H, s), 4.22 and 4.38 (2H, ABq, J 13 Hz), 4.25 and 4.38 (2H, ABq, J 13 Hz), 4.93 (1H, d, J 5 Hz), 5.00 (1H, d, J 5 Hz), 5.16 and 5.27 (2H, ABq, J 12 Hz), 5.18 and 5.28 (2H, ABq, J 12 Hz), 5.76 (1H, d, J 5 Hz), 5.81 (1H, d, J 5 Hz), 6.01 (1H, s), 6.10 (1H, s), 6.76 (2H, s), 6.8-7.4 (66H, m), 7.52 (2H, d, J 8 Hz), 7.57 (2H, d, J 8 Hz), and 8.72 (4H, d, J 8 Hz); m/z (F.A.B., thioglycerol) MH⁺ 1300.

### c) Sodium [6R,7R]-7-r2-(2-amino-4-thiazolyl)-2-(Z)-[[R,S]-carboxy(3,4-diacetoxyphenyl)methyloxyimino]-acetamido]-3-[1-(methylamino)pyridinium-4-thiomethyl]-ceph-3-em-4-carboxylate

4-Methoxybenzyl [6R,7R]-7-[2-(Z)[[R,S]-(3,4-diacetoxyphenyl)(diphenylmethyloxycarbonyl)methyloxyimino]-2-(2-tritylamino-4-thiazolyl)acetamido]-3-[1-(methylamino)pyridinium-4-thiomethyl]ceph-3-em-4-carboxylate iodide (0.15g, 0.105mmol) was dissolved in dichloromethane (10ml) and treated with trifluoroacetic acid (0.5ml, 6.5mmol). The mixture was stirred for 1h. and evaporated to dryness under reduced pressure. The residue was triturated with diethyl ether (3 x 20ml) and then dissolved in water with dilute aqueous sodium hydrogen carbonate to pH 7. The product was purified on Diaion HP20SS resin, eluting with water and mixtures of water and tetrahydrofuran. Fractions containing the product were combined and freeze dried to give the title compound (0.03g, 36%); υₘₐₓ (KBr) 1764, 1670, 1617, 1529, and 1501cm⁻¹; δ_{H} (D₂O) 2.29 (6H, s), 2.31 (6H, s), 2.99 (6H, s), 3.24 and 3.51 (2H, ABq, J 19 Hz), 3.24 and 3.53 (2H, ABq, J 19 Hz), 4.14 and 4.35 (2H, ABq, J 14 Hz), 4.14 and 4.37 (2H, ABq, J 14 Hz), 5.02 (1H, d, J 5 Hz), 5.03 (1H, d, J 5 Hz), 5.54 (2H, s), 5.64 (1H, d, J 5 Hz), 5.68 (1H, d, J 5 Hz), 6.98 (2H, s), 7.2-7.5 (6H, m), 7.79 (4H, d, J 7 Hz), and 8.48 (4H, broad); m/z (F.A.B., thioglycerol) M (H-Na)⁺ 772.

### d) Sodium [6R,7R]-7-[2-(2-amino-4-thiazolyl)-2-(Z)-[[R,S]carboxy(3,4-dihydroxyphenyl)methyloxyimino]-acetamido]-3-[1-(methylamino)pyridinium-4-thiomethyl]-ceph-3-em-4-carboxylate

Sodium [6R,7R]-7-[2-(2-amino-4-thiazolyl)-2-(Z)-[[R,S]-carboxy(3,4-diacetoxyphenyl)methyloxyimino]-acetamido]-3-[1-(methylamino)pyridinium-4-thiomethyl]-ceph-3-em-4-carboxylate (0.023g, 0.03mmol) was dissolved in water (5ml) and methanol (5ml) and treated with dilute sodium hydroxide to pH 9.4. After 5 minutes the mixture was neutralised with 0.1M hydrochloric acid and the solvent removed under reduced pressure. The product was purified on Diaion HP20SS resin, eluting with mixtures of water and tetrahydrofuran. Fractions containing product were combined and freeze dried to give the title compound (0.016g, 75%); υₘₐₓ (KBr) 1762, 1660, 1617, and 1527cm⁻¹; δ_{H} (D₂O) 3.00 (6H, s), 3.12 and 3.46 (2H, ABq, J 18 Hz), 3.15 and 3.46 (2H, ABq, J 18 Hz), 4.13 and 4.30 (2H, ABq, J 14 Hz), 4.13 and 4.36 (2H, ABq, J 14 Hz), 4.97 (1H, d, J 5 Hz), 4.99 (1H, d, J 5 Hz), 5.37 (2H, s), 5.58 (1H, d, J 5 Hz), 5.61 (1H, d, J 5 Hz), 6.75-6.92 (4H, m), 6.96 (4H, s), 7.79 (4H, d, J 7 Hz), and 8.47 (4H, d, J 7Hz); m/z (F.A.B., thioglycerol) M(H-Na)⁺ 688.

### EXAMPLE 24

### Sodium [6R,7R]-7-[2-(2-amino-4-thiazolyl)-2-(Z)-[(S)-carboxy (3,4-dihydroxyphenyl)methyloxyimino]acetamido]-3-[1-(methylamino)pyridinium-4-thiomethyl]-ceph-3-em-4-carboxylate

The title compound was prepared from 2-[(Z)-(S)-(3,4-diacetoxyphenyl)(diphenylmethyloxycarbonyl)methyloxyimino]-2-(2-tritylamino-4-thiazolyl)acetic acid using the method described in Example 23; υₘₐₓ (KBr) 1762, 1660 sh, 1617, 1528 and 1387 cm⁻¹; δ_{H}(250 MHz D₂O) 3.01 (3H,s), 3.16 and 3.49 (2H, ABq, J = 18 Hz), 4.16 and 4.30 (2H, ABq, J = 14 Hz), 5.01 (1H, d, J = 5 Hz), 5.38 (1H, s), 5.62 (1H, d, J = 5 Hz), 6.80 - 6.97 (4H, m), 7.80 (2H, d, J = 7 Hz), 8.48 (2H, d, J = 7 Hz); m/z (F.A.B., thioglycerol) MH⁺ 710.

### EXAMPLE 25

### Sodium [6R,7R]-7-[2-(2-amino-4-thiazolyl)-2-(Z)-[(R)-carboxy(3,4-dihydroxyphenyl)methyloxyimino]-acetamido] -3-[1-(methylamino)pyridinium-4-thiomethyl]ceph-3-em-4-carboxylate

The title compound was prepared from 2-[(Z)-(R)-(3,4-diacetoxyphenyl)(diphenylmethyloxycarbo nyl)methyloxyimino]-2-(2-tritylamino-4-thiazolyl)acetic acid using the method described in Example 23, (0.037g, 18%); υₘₐₓ (KBr) 1762, 1650 (sh), 1617, and 1527cm⁻¹; δ_{H} (D₂O) 2.99 (3H, s), 3.13 and 3.45 (2H, ABq, J 17.5Hz), 4.11 and 4.35 (2H, ABq, J 14Hz), 4.96 (1H, d, J 5Hz), 5.36 (1H, s), 5.59 (1H, d, J 5Hz), 6.79 (1H, d, J 8Hz), 6.86 (1H, dd, J 8 and 1.5Hz), 6.95 (1H, s), 6.97 (1H, d, J 1.5Hz), 7.77 (2H, d, J 7Hz), and 8.46 (2H, d, J 7Hz); m/z (F.A.B., thioglycerol) MH⁺ 710.

### EXAMPLE 26

### Sodium [6R,7R]-3-[1-aminopyridinium-4-thiomethyl]-7-[2-(2-amino-4-thiazolyl)-2-(Z)-[(R,S)-carboxy (3,4-dihydroxyphenyl)methyloxyimino]acetamido]ceph-3-em-4-carboxylate

The title compound is prepared as described in Example 23 except that 1-amino-4-thiopyridone replaced 1-(methylamino)-4-thiopyridone.

### EXAMPLE 27

### Sodium [6R,7R]-3-[1-(acetylamino)pyridinium-4-thiomethyl]-7-[2-(2-amino-4-thiazolyl)-2-(Z)-[(S)-carboxy-(3,4-dihydroxyphenyl)methyloxyimino]acetamido]ceph-3-em-4-carboxylate and Sodium [6R,7R]-3-[1-aminopyridinium-4-thiomethyl]-7-[2-(2-amino-4-thiazolyl)-2-(Z)-[(S)-carboxy (3,4(3,4-dihydroxyphenyl)methyloxyimino]-acetamido]ceph-3-em-4-carboxylate

Sodium [6R,7R]-3-(1-aminopyridinium-4-thiomethyl)-7-[2-(2-amino-4-thiazolyl)-2-(Z)-[(S)-carboxy(3,4-dihydroxyphenyl)methyloxyimino]acetamido]ceph-3-em-4-carboxylate was prepared from 2-[(Z)-(S)-(3,4-diacetoxyphenyl)(diphenylmethyloxycarbonyl)methyloxyimino]-2-(2-tritylamino-4-thiazolyl)acetic acid and 1-amino-4-thiopyridone using the method described in Example 23, (0.013g, 7%); υₘₐₓ (KBr) 1762, 1603, and 1528cm⁻¹; δ_{H} (D₂O) 3.11 and 3.47 (2H, ABq, J 18Hz), 4.10 and 4.30 (2H, ABq, J 14Hz), 4.98 (1H, d, J 4.5Hz), 5.36 (1H, s), 5.62 (1H, d, J 4.5Hz), 6.82 (1H, d, J 8Hz), 6.89 (1H, dd, J 8 and 1Hz), 6.97 (1H, s), 6.98 (1H, d, J 1Hz), 7.74 (2H, d, J 6Hz), and 8.37 (2H, d, J 6Hz); m/z (F.A.B., thioglycerol) MH⁺ 696.

Also isolated by chromatography was sodium [6R,7R]-3-[1-(acetylamino)pyridinium-4-thiomethyl]-7-[2 -(2-amino-4-thiazolyl)-2-(Z)-[(S)-carboxy(3,4-dihydroxy phenyl)methyloxyimino]acetamido]ceph-3-em-4-carboxylate (0.0467g; 26%); υₘₐₓ (KBr) 1762 and 1612cm⁻¹; δ_{H} (D₂O) 1.98 (3H,s), 3.10 and 3.52 (2H, ABq, J 17Hz), 4.06 and 4.12 (2H, ABq, J 14Hz), 5.00 (1H, d, J 5Hz), 5.39 (1H, s), 5.65 (1H, d, J 5Hz), 6.82 - 6.95 (3H, m), 7.02 (1H, s), 7.70 (2H, d, J 7Hz) and 8.13 (2H, d, J 7Hz); m/z (F.A.B., thioglycerol) MNa⁺ 760 and MH⁺ 738.

### EXAMPLE 28

### Sodium [6R,7R]-3-[1-aminopyridinium-4-thiomethyl]-7-[2-(2-amino-4-thiazolyl)-2-(Z)-[(R)-carboxy (3,4-dihydroxyphenyl)methyloxyimino]acetamido]ceph-3-em-4-carboxylate

The title compound is prepared from 2-(Z)-[(R)-(3,4-diacetoxyphenyl)(diphenylmethyl-oxycarbonyl)methyloxyimino]-2-(2-tritylamino-4-thiazolyl)acetic acid and 1-amino-4-thiopyridone using the method described in Example 23.

### EXAMPLE 49

### Sodium [6R,7R]-7-[2-(2-amino-4-thiazolyl)-2-(Z)-[(S)-carboxy (3,4-dihydroxyphenyl)methyloxyimino]-acetamido]-3-[1-(methylamino)pyridinium-4-thiomethyl]-ceph-3-em-4-carboxylate

### a) 4-Methoxybenzyl [6R,7R]-7-[(Z)-[(S)-(3,4-diacetoxyphenyl)(diphenylmethyloxycarbonyl)methyloxyimino]-2-(2-tritylamino-4-thiazolyl)acetamido]-3-[1-(methylamino)pyridinium-4-thiomethyl]ceph-3-em-4-carboxylate iodide

2-[(Z)-[S]-(3,4-Diacetoxyphenyl)(diphenylmethyloxycarbo nyl)methyloxyimino]-2-(2-tritylamino-4-thiazolyl)acetic acid (0.845g, 1mmol) was dissolved in dry N,N-dimethylformamide (5ml) under argon and cooled to 0-5°C. N,N-Diisopropylethylamine (0.129g, 1mmol) was added and the mixture cooled to -40°C. Methanesulphonyl chloride (0.114g, 1mmol) was added and the mixture allowed to warm to -20°C and stirred at -20°C for 0.5h. The reaction mixture was re-cooled to -40°C and treated with a solution of 4-methoxybenzyl [6R,7R]-7-amino-3-[1-(methylamino)pyridinium-4-thiomethyl]ceph-3-em-4-carboxylate iodide (0.6g, 1mmol) and pyridine (0.079g, 1mmol) in dry dichloromethane (10ml). Cooling was removed and the mixture allowed to regain room temperature. After 1h, the reaction mixture was diluted with dichloromethane (50ml) and washed with water (5 x 20ml), saturated brine, dried over magnesium sulphate and evaporated to dryness under reduced pressure. The residue was dissolved in acetone (10ml) and treated with sodium iodide (0.15g, 1mmol). After 0.5h, the reaction mixture was filtered through Celite and evaporated to dryness under reduced pressure. The residue was purified on silica gel 60, eluting with methanol, dichloromethane mixtures, to give the title compound (0.271g, 19%); υₘₐₓ (KBr) 1774, 1730, 1670 and 1616cm⁻¹; δ_{H} [(CD₃)₂SO] 2.23-2.28 (6H, br), 3.00 (3H, d, J 6Hz), 3.71 (3H, s), 4.27 (2H, br s), 5.12 (1H, d, J 5Hz), 5.20 (2H, br s), 5.55 (1H, dd, J 8 and 5Hz), 5.87 (1H, s), 6.78-6.89 (4H, m), 7.14-7.48 (30H, m), 7.88 (2H, d, J 7Hz), 8.11-8.20 (1H, q, J 6Hz, exch.), 8.76 (2H, d, J 7Hz), 8.96 (1H, br s, exch.), 9.62 (1H, d, J 8Hz, exch.); m/z (F.A.B., thioglycerol) MH⁺ 1300.

### b) Sodium [6R,7R]-7-[2-(2-amino-4-thiazolyl)-2-(Z)-[(S)-carboxy (3,4-dihydroxyphenyl)methyloxyimino]-acetamido]-3-[1-(methylamino)pyridinium-4-thiomethyl]-ceph-3-em-4-carboxylate

4-Methoxybenzyl [6R,7R]-7-[2-(Z)-[(S)-(3,4-diacetoxyphenyl) (diphenylmethyloxycarbonyl)methyloxyimino]-2-(2-tritylamino-4-thiazolyl)acetamido]-3-[1-(methylamino)pyridinium-4-thiomethyl]ceph-3-em-4-carboxylate iodide (0.25g, 0.18mmol) was dissolved in dichloromethane (15ml) and treated with trifluoroacetic acid (0.906g, 7.9mmol). After 1h, the volatiles were removed under reduced pressure, the residue treated with toluene (2ml) and re-evaporated to dryness under reduced pressure. The residue was triturated under diethyl ether and the resulting solid collected by filtration and washed with diethyl ether. This solid was dissolved in methanol (5ml) and the pH adjusted to 10.5 by the addition of saturated, aqueous sodium hydrogen carbonate. After 10 minutes, the reaction mixture was concentrated to low volume under reduced pressure and freeze-dried. The product was purified on Diaion HP20SS resin, eluting with mixtures of tetrahydrofuran in water, to give, after freeze-drying, the title compound (0.03g, 24%). This material was identical in all respects with the material obtained in Example 24.

### EXAMPLE 51

### [6R,7R]-7-[2-(2-Amino-4-thiazolyl)-2-(Z)-[3,4-(methylenedioxy)benzyloxyimino]acetamido]-3-[1-(methylamino)-pyridinium-4-thiomethyl)ceph-3-em-4-carboxylate

### a) 4-Methoxybenzyl [6R,7R]-3-[1-(methylamino)-pyridinium-4-thiomethyl]-7-[2-(Z)-[3,4-(methylenedioxy)benzyloxyimino)-2-(2-tritylamino-4-thiazolyl)-acetamido]ceph-3-em-4-carboxylate iodide

The product of Preparation 36 (0.375g, 0.6mmol) was acylated with the product of Preparation 37 (0.352g, 0.6mmol) by the method described in Example 49(a). The product was purified by chromatography on silica gel 60, eluting with mixtures of ethanol in dichloromethane (0.186g, 26%); υₘₐₓ (KBr) 1775, 1719 and 1675cm⁻¹; δ_{H} (CDCl₃) 3.07 (3H, d, J 6Hz), 3.41 and 3.65 (2H, ABq, J 18Hz), 3.81 (3H, s), 4.27 and 4.47 (2H, ABq, J 13Hz), 5.00 (1H, d, J 5Hz), 5.05-5.28 (4H, m), 5.86 (1H, dd, J 5 and 9Hz), 5.94 (2H, s), 6.65-7.44 (25H, m), 7.70 (2H, d, J 7Hz), 8.77 (3H, d, J 7Hz); m/z (F.A.B., thioglycerol) M⁺ 1018.

### b) [6R,7R]-7-[2-(2-Amino-4-thiazolyl)-2-(Z)-[3,4-(methylenedioxy)benzyloxyimino]acetamido]-3-[1-(methylamino)pyridinium-4-thiomethyl]ceph-3-em-4-carboxylate

The product of Example 51(a) (0.176g, 0.15mmol) was deprotected as described in Example 2(ii) (b) to give the title compound (0.03g, 30%); υₘₐₓ (KBr) 1765 and 1670cm⁻¹; δ_{H} [(CD₃)₂SO] 2.99 (3H, br s), 3.20-3.50 (2H, m), 4.39 and 4.59 (2H, ABq, J 14Hz), 5.01 (3H, m), 5.57 (1H, dd, J 5 and 8Hz), 6.00 (2H, s), 6.71 (1H, s), 6.80-6.97 (3H, m), 7.23 (2H, br, exch.), 8.52 and 8.81 (each 2H, d, J 7Hz), 9.60 (1H, d, J 8Hz, exch.), 8.45-8.70 (1H, br, exch.); m/z (F.A.B., thioglycerol/acetic acid) MH⁺ 656.

## Claims (Claims for the following Contracting State(s): AT, BE, CH, DE, DK, FR, GB, GR, IT, LI, LU, NL, SE)

1. A compound of formula (I): 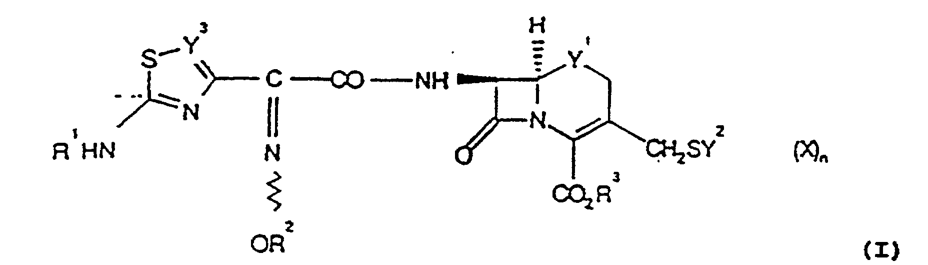 wherein
Y¹ ¹ is sulphur, -SO-, or SO₂-;
R¹ is hydrogen or an amino protecting group;
R² is (C₁-C₁₂)alkyl, (C₂-C₁₂)alkenyl, (C₂-C₁₂)alkynyl, (C₃₋₇)cycloalkyl, (C₅₋₈)cycloalkenyl, each of which is substituted with a phenyl group which may be unsubstituted or substituted up to 3 times with (C₁₋₆)alkoxy or hydroxy; and wherein each of the R² substituents hereinbefore defined is optionally further substituted with carboxyl; or R² is (methylenedioxy)-benzyl;
CO₂R³ is carboxy or a carboxylate anion, or the group R³ is a readily removable carboxy protecting group;
Y² is a group of the formula: 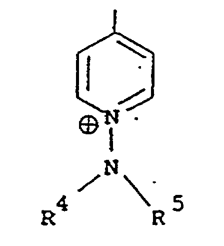 wherein the pyridinium group is unsubstituted or substituted at a ring carbon atom available for substitution by up to four substituents selected from (C₁₋₆)alkyl, and (C₁₋₆)alkoxy;
R⁴ and R⁵ which may be the same or different are selected from hydrogen or a group R⁶;
R⁶ is (C₁₋₆)alkyl, (C₃₋₇)cycloalkyl, (C₂₋₆)alkenyl, (C₅₋₈)cycloalkenyl, (C₂₋₆)alkynyl, each of which may be unsubstituted or substituted by halogen, cyano, azido, nitro, carboxy, (C₁₋₆)alkoxycarbonyl, carbamoyl, mono- or di-(C₁₋₆)alkylcarbamoyl, sulphono, sulphamoyl, mono- and di-(C₁₋₆)alkylsuphamoyl, amino, mono- and di-(C₁₋₆)alkylamino, acylamino, (C₁₋₆)alkoxycarbonyl amino, hydroxy, (C₁₋₆)alkoxy, acyloxy, oxo, phenylcarbonyl, naphthylcarbonyl, heterocyclylcarbonyl, (C₁₋₆)alkylthio, (C₁₋₆)alkanesulphinyl, (C₁₋₆)alkanesulphonyl, phenyl, naphthyl or heterocyclyl; wherein the phenyl or naphthyl may be unsubstituted or substituted up to 5 times by halogen, (C₁₋₆)alkyl, phenyl, (C₁₋₆)alkoxy, hydroxy(C₁₋₆)alkyl, mercapto(C₁₋ ₆)alkyl, halo(C₁₋₆)alkyl, mercapto, hydroxy, amino, mono- or di-(C₁₋₆)alkylamino, nitro, carboxy, (C₁₋ ₆)alkoxycarbonyl, (C₁₋₆)alkoxycarbonyl-(C₁₋₆)alkyl, (C₁₋ ₆)alkylcarbonyloxy, formyl, or (C₁₋₆)alkylcarbonyl; wherein the heterocyclyl moiety may be unsubstituted or substituted up to 3 times by (C₁₋₆)alkyl, (C₁₋₆)alkoxy, (C₁₋ ₆)alkoxy(C₁₋₆)alkyl, halo(C₁₋₆)alkyl, hydroxy, amino, mono- or di-(C₁₋₆)alkylamino, carboxy, carbamoyl, (C₁₋₆)alkoxycarbonyl, (C₁₋₆)alkoxycarbonyl(C₁₋₆)alkyl, oxo; and the heterocyclyl moiety is aromatic or non-aromatic, single or bicyclic rings containing up to four heteroatoms in each ring selected from oxygen, nitrogen and sulphur; or R⁶ is Pyridyl; isoxazolylmethyl; thiazolymethyl; chloropyridinyl; pyrazinyl; imidazolinyl; benzopyridinyl; benzoyl; 3,4-dihydroxybenzoyl; 4-nitrobenzoyl; 4-methoxybenzoyl ; 4-carboxybenzoyl; 4-arninobenzoyl; 2-furanoyl; 3,4-dihydroxycinnamoyl; carbamoyl; and N-methylcarbamoyl.
Y³ is nitrogen or -CH;
X is an inorganic or organic anion, and
n is 0 or 1,
with the proviso that:
(i) when CO₂R³ is carboxylate, n is 0, and
(ii) when CO₂R³ is carboxy or the group R³ is a readily removable carboxy protecting group, then n is 1,
and the anion X is present in the appropriate stoichiometric proportion to balance the positive charge on the pyridinium group.

2. A compound according to claim 1 in which R² is a substituted (C₁₋₆)alkyl.

3. A compound according to claim 2 in which R² is a methyl substituted with a phenyl group which is unsubstituted or substituted up to 3 times with (C₁₋₆)alkoxy or hydroxy.

4. A compound according to claim 3 in which the methyl is further substituted with a carboxy group.

5. A compound according to claim 2 in which R² is carboxy(3,4-dihydroxyphenyl)methyl or (methylenedioxy)-benzyl.

6. A compound according to claim 3 in which R⁴ and R⁵ are each selected from hydrogen, methyl, ethyl, carboxymethyl, methoxyethyl, cyanomethyl, propargyl, 4-carboxy-butan-1-yl, 2-amino-2-(methoxycarbonyl)ethyl, cyclopropylmethyl, propyl, cyclopentyl, prop-2-en-1-yl, butyl, hexyl, isopropyl, 2-hydroxyethyl, isoxazolylmethyl, thiazolylmethyl.

7. A compound of formula (Ia) or a pharmaceutically acceptable salt or a pharmaceutically acceptable *in vivo* hydrolysable ester thereof: 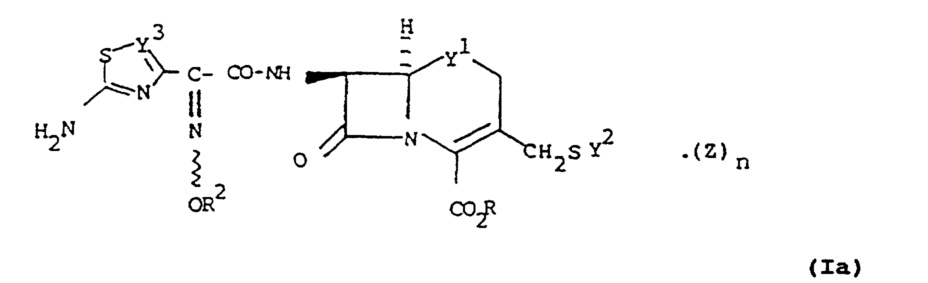 wherein R², Y¹, Y², Y³ and n are as defined in claim 1; the group CO₂R is carboxy or a carboxylate anion and Z is a pharmaceutically acceptable inorganic or organic anion present in the appropriate stoichiometric proportion to balance the positive charge on the pyridinium ring of the group Y^{2.}

8. A compound according to claim 1 of formula (II): 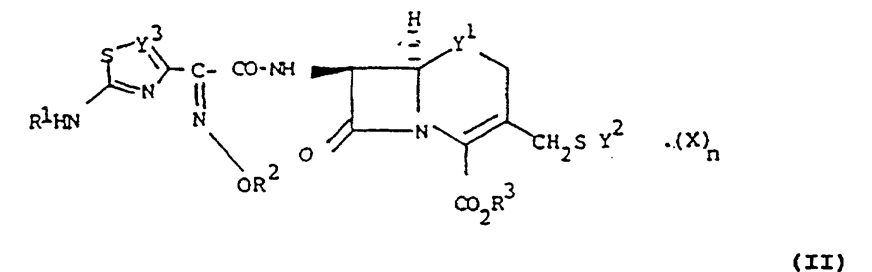 wherein R¹, R², R³, X, Y¹, Y², Y³ and n are as defined with respect to formula (I) according to claim 1.

9. A pharmaceutical composition comprising a compound of formula (Ia) as defined in claim 7 or a pharmaceutically acceptable salt or *in vivo* hydrolysable ester thereof, in combination with a pharmaceutically acceptable excipient or diluent.

10. A pharmaceutical composition as claimed in claim 9 further comprising a β-lactamase inhibitor.

11. A compound according to claim 8 in which R² is a (C₁₋₆)alkyl, or (C₃₋₇)cycloalkyl, each of which is substituted with a phenyl group which may be unsubstituted or substituted up to 3 times with (C₁₋₆) alkoxy or hydroxy.

12. A compound according to claim 11 wherein R² is methyl optionally further substituted by a carboxy group.

13. A compound according to claim 11 wherein R² is carboxy(3,4-dihydroxyphenyl)methyl.

14. A compound according to claim 8 wherein R⁴ and R⁵ are each selected from hydrogen, methyl, ethyl, carboxymethyl, methoxyethyl, cyanomethyl, propargyl, 4-carboxy-butan-1-yl, 2-amino-2-(methoxycarbonyl)ethyl, cyclopropylmethyl, propyl, cyclopentyl, prop-2-en-1-yl, butyl, hexyl, isopropyl, 2-hydroxyethyl, pyridyl, isoxazolylmethyl, thiazolylmethyl, chloropyridinyl, pyrazinyl, imidazolinyl, benzopyrazidinyl, acetyl, benzoyl, 3,4-dihydroxybenzoyl, 4-nitrobenzoyl, 4-methoxybenzoyl, 4-carboxybenzoyl, 4-aminobenzoyl, 2-furanoyl, 3,4-dihydroxycinnamoyl, carbamoyl, N-methylcarbamoyl and t-butyloxycarbonyl.

15. The compound according to claim 7 which is [6R,7R]-7-[2-(2-Amino-4-thiazolyl)-2-(Z)-[(R)-carboxy(3,4-dihydroxyphenyl)-methyloxyimino]-acetamido]-3-[1-(methylamino)pyridinium-4-thiomethyl]ceph-3-em-4-carboxylate or pharmaceutically acceptable salts thereof.

16. The compound according to claim 7 which is [6R,7R]-7-[2-(2-Amino-4-thiazolyl)-2-(Z)-[(R) -carboxy(3,4-dihydroxyphenyl)-methyloxyimino]-acetamido]-3-[1-(methylamino)pyridinium-4-thiomethyl]ceph-3-em-4-carboxylic acid or pharmaceutically acceptable salts thereof.

## Claims (Claims for the following Contracting State(s): ES)

1. A process for the preparation of a compound of formula (I) or a salt thereof: 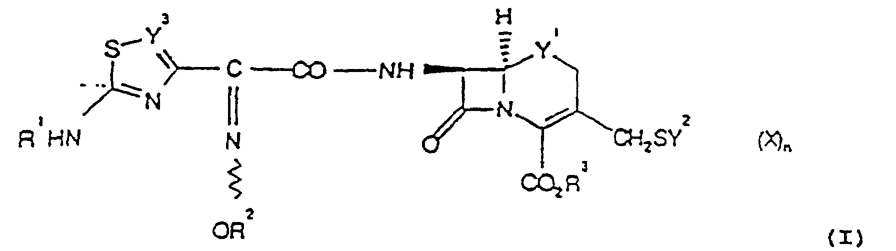 wherein
Y¹ is sulphur, -SO-, or SO₂-;
R1 is hydrogen or an amino protecting group;
R² is (C₁-C₁₂)alkyl, (C₂-C₁₂)alkenyl, (C₂-C₁₂)alkynyl, (C₃₋₇)cycloalkyl, (C₅₋₈)cycloalkenyl, each of which is substituted with a phenyl group which may be unsubstituted or substituted up to 3 times with (C₁₋₆)alkoxy or hydroxy; and wherein each of the R² substituents hereinbefore defined is optionally further substituted with carboxyl; or R² is (methylenedioxy)-benzyl;
CO₂R³ is carboxy or a carboxylate anion, or the group R³ is a readily removable carboxy protecting group;
Y² is a group of the formula: 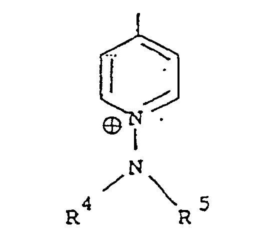 wherein the pyridinium group is unsubstituted or substituted at a ring carbon atom available for substitution by up to four substituents selected from (C₁₋₆)alkyl, and (C₁₋₆)alkoxy;
R⁴ and R⁵ which may be the same or different are selected from hydrogen or a group R⁶,
R⁶ is (C₁₋₆) alkyl, (C₃₋₇) cycloalkyl, (C₂₋₆)alkenyl, (C₅₋₈)cycloalkenyl, (C₂₋₆)alkynyl, each of which may be unsubstituted or substituted by halogen, cyano, azido, nitro, carboxy, (C₁₋₆)alkoxycarbonyl, carbamoyl, mono- or di-(C₁₋₆)alkylcarbamoyl, sulphono, sulphamoyl, mono- and di-(C₁₋₆)alkylsuphamoyl, amino, mono- and di-(C₁₋₆)alkylamino, acylamino, (C₁₋₆)alkoxycarbonyl amino, hydroxy, (C₁₋₆)alkoxy, acyloxy, oxo, phenylcarbonyl, naphthylcarbonyl, heterocyclylcarbonyl, (C₁₋₆)alkylthio, (C₁₋₆) alkanesulphinyl, (C₁₋₆) alkanesulphonyl, phenyl, naphthyl or heterocyclyl wherein
the phenyl or naphthyl may be unsubstituted or substituted up to 5 times by halogen, (C₁₋₆)alkyl, phenyl, (C₁₋₆)alkoxy, hydroxy(C₁₋₆)alkyl, mercapto(C₁₋ ₆)alkyl, halo(C₁₋₆)alkyl, mercapto, hydroxy, amino, mono- or di-(C₁₋₆)alkylamino, nitro, carboxy, (C₁₋ ₆) alkoxycarbonyl, (C₁₋₆)alkoxycarbonyl-(C₁₋₆)alkyl, (C₁₋ ₆)alkylcarbonyloxy, formyl, or (C₁₋₆)alkylcarbonyl; wherein the heterocyclyl moiety may be unsubstituted or substituted up to 3 times by (C₁₋₆)alkyl, (C₁₋₆)alkoxy, (C₁₋ ₆)alkoxy(C₁₋₆)alkyl, halo(C₁₋₆)alkyl, hydroxy, amino, mono- or di-(C₁₋₆)alkylamino, carboxy, carbamoyl, (C₁₋₆)alkoxycarbonyl, (C₁₋₆)alkoxycarbonyl(C₁₋₆)alkyl, oxo, and the heterocyclyl moiety is aromatic or non-aromatic, single or bicyclic rings containing up to four heteroatoms in each ring selected from oxygen, nitrogen and sulphur; or R⁶ is Pyridyl; isoxazolylmethyl; thiazolymethyl; chloropyridinyl; pyrazinyl; imidazolinyl; benzopyridinyl; benzoyl; 3,4-dihydroxybenzoyl; 4-nitrobenzoyl; 4-methoxybenzoyl ; 4-carboxybenzoyl; 4-aminobenzoyl; 2-furanoyl; 3,4-dihydroxycinnamoyl; carbamoyl; and N-methylcarbamoyl.
Y³ is nitrogen or -CH;
X is an inorganic or organic anion, and
n is 0 or 1,
with the proviso that:
(i) when CO₂R³ is carboxylate, n is 0, and
(ii) when CO₂R³ is carboxy or the group R³ is a readily removable carboxy protecting group, then n is 1,
and the anion X is present in the appropriate stoichiometric proportion to balance the positive charge on the pyridinium group; which process comprises:
(a) treating a compound of formula (V): 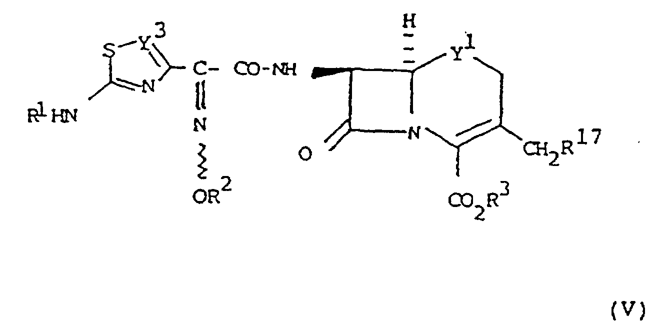 wherein R¹, R², R³, Y¹, and Y³ are as hereinbefore defined, and R¹⁷ is a leaving group; and wherein any reactive groups may be protected;
with a thiopyridone compound of formula (VI): 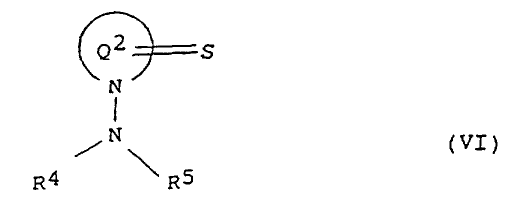 wherein the nucleus: 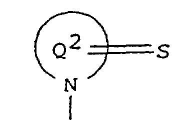 is such that it is converted to the nucleus of the group Y² (as defined in claim 1) in situ during the course of the reaction;
and R⁴ and R⁵ are as hereinbefore defined;
with the proviso that when R¹⁷ is an acyloxy group -CO₂R³ is in the free acid form or a salt thereof;
and thereafter if necessary carrying out one or more of the following steps:
i) converting each or any one of the groups R², R³, R⁴ and R⁵ into a different group R², R³, R⁴ and R⁵;
ii) removing any protecting groups; or
iii) converting the product into a salt; or
(b) reacting a compound of formula (VIII) or a salt thereof: 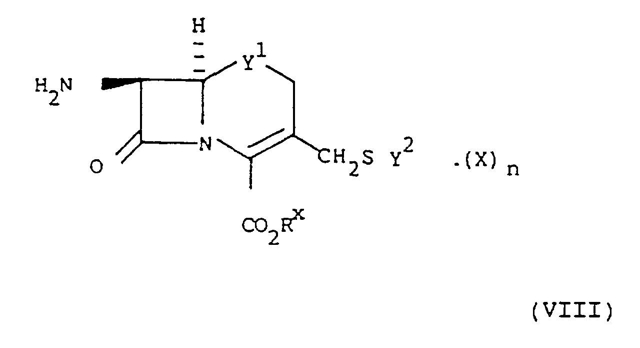 wherein X, Y¹, Y² and n are as hereinbefore defined, R^{x} is hydrogen or a readily removable carboxyl blocking group and the 7β-amino group is optionally substituted with a group which permits acylation to take place; and any reactive groups may be protected;
with an N-acylating derivative of an acid of formula (IX): 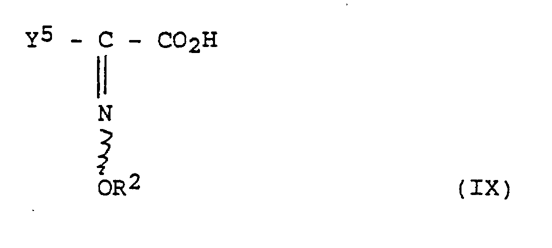 wherein R² is as hereinbefore defined and Y⁵ is a group of formula: 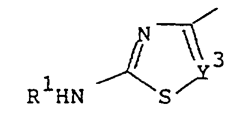 or a group which is convertible thereto, and R¹ is as hereinbefore defined; and wherein any reactive groups may be protected; and thereafter, if necessary, carrying out one or more of the following steps:
(i) removing any protecting group, including an amino-protecting group R¹;
(ii) converting the group R^{x} into a group R³;
(iii) converting the product to a salt;
(iv) converting a group which is convertible to Y⁵ into Y⁵, or
(v) converting each or any one of the groups R⁴ and R⁵ into a different group R⁴ and R⁵.

2. A process as claimed in claim 1 for the preparation of a compound of formula (Ia) or a pharmaceutically acceptable salt or a pharmaceutically acceptable in vivo hydrolysable ester thereof: 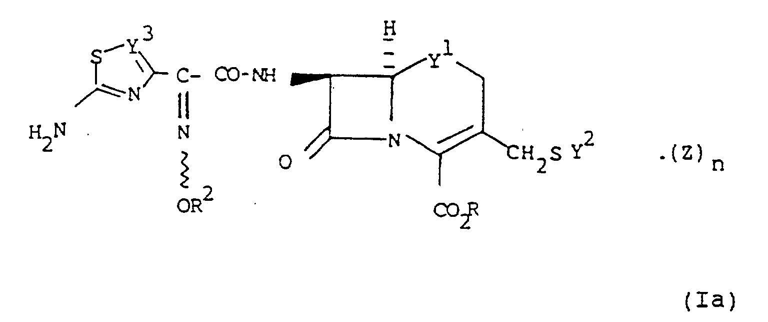 wherein R², Y¹, Y², y³ and n are as defined in claim 1, the group CO₂R is carboxy or a carboxylate anion and Z is a pharmaceutically acceptable inorganic or organic anion present in the appropriate stoichiometric proportion to balance the positive charge on the pyridinium ring of the group Y².

3. A process as claimed in claim 1 for the preparation of a compound of the formula (Ib): 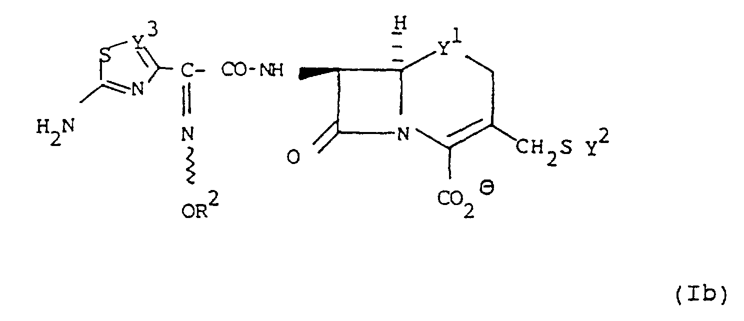 wherein R², Y¹, Y², and Y³ are as defined with respec to formula (Ia).

4. A process as claimed in claim 1 for the preparation of a compound of the formula (Ic): 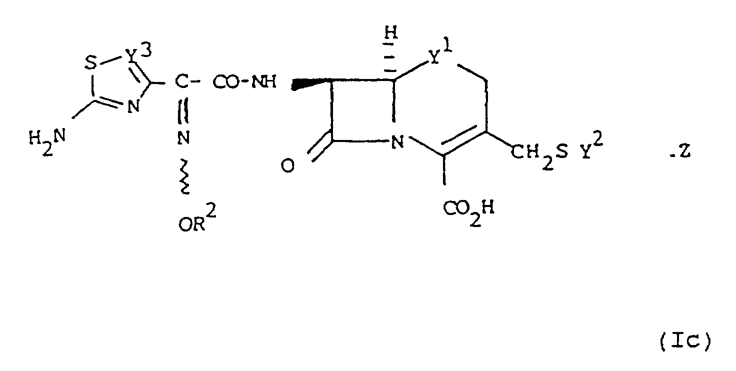 wherein R², Y¹, Y², Y³, and Z are as defined with respect to formula (Ia).

5. A process as claimed in claim 1 for the preparation of a compound of the formula (II): 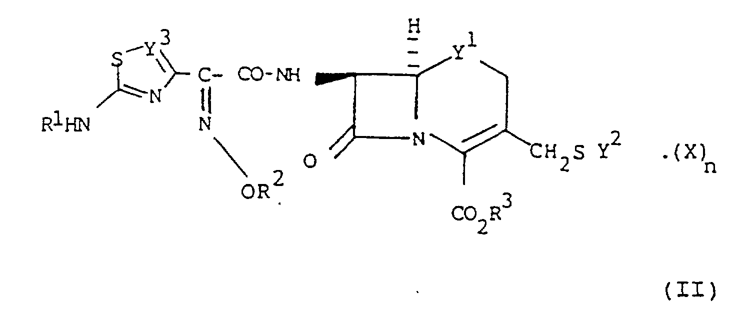 wherein R¹, R², R³, X, Y¹, Y², Y³ and n are as defined with respect to formula (I).

6. A process as claimed in any one of claims 1 to 5 in which R² is (C₁₋₆)alkyl or (C₃₋₇) cycloalkyl, each of which is substituted with a phenyl group which may be unsubstituted or substituted up to 3 times with (C₁₋₆) alkoxy or hydroxy.

7. A process as claimed in claim 6 in which R² is methyl, optionally further substituted by carboxy.

8. A process as claimed in claim 7 in which R² is 3,4-dihydroxyphenylmethyl, optionally further substituted by carboxy.

9. A process as claimed in claim 6 in which R² is selected from carboxy (3,4-dihydroxy-phenyl) methyl and (methylenedioxy) benzyl.

10. A process as claimed in claim 1 in which R⁴ and R⁵ is each selected from hydrogen, methyl, ethyl, carboxymethyl, methoxyethyl, cyanomethyl, propargyl, 4-carboxy-butan-1-yl, 2-amino-2-(methoxycarbonyl)ethyl, cyclopropylmethyl, propyl, cyclopentyl, prop-2-en-1-yl, butyl, hexyl, isopropyl, 2-hydroxyethyl, pyridyl, isoxazolylmethyl, thiazolylmethyl, chloropyridinyl, pyrazinyl, imidazolinyl, benzopyrazidinyl, acetyl, benzoyl, 3,4-dihydroxybenzoyl, 4-nitrobenzoyl, 4-methoxybenzoyl, 4-carboxybenzoyl, 4-aminobenzoyl, 2-furanoyl, 3,4-dihydroxycinnamoyl, carbamoyl, N-methylcarbamoyl and t-butyoxycarbonyl.

11. A process as claimed in any one of the preceding claims for the preparation of selected from the following and pharmaceutically acceptable salts and in-vivo hydrolysable esters thereof:
[6R,7R]-7-[2-(2-Amino-4-thiazolyl)-2-(Z)-[(R,S)-carboxy(3,4-dihydroxyphenyl)methyloxyimino]acetamido]-3-[1-(methylamino)pyridinium-4-thiomethyl]ceph-3-em-4-carboxylate;
[6R,7R]-7-[2-(2-Amino-4-thiazolyl)-2-(Z)-[(S)-carboxy(3,4-dihydroxyphenyl)methyloxyimino]acetamido]-3-[1-(methylamino)pyridinium-4-thiomethyl]ceph-3-em-4-carboxylate;
[6R,7R]-7-[2-(2-Amino-4-thiazolyl)-2-(Z)-[(R)-carboxy(3,4-dihydroxyphenyl)methyloxyimino]acetamido]-3-[1-(methylamino)pyridinium-4-thiomethyl]ceph-3-em-4-carboxylate;
[6R,7R]-3-[1-Aminopyridinium-4-thiomethyl]-7-[2(2-amino-4-thiazolyl)-2-(Z)-[(R,S)-carboxy(3,4-dihydroxyphenyl)methyloxyimino]acetamido]ceph-3-em-4-carboxylate;
[6R,7R]-3-[1-Aminopyridinium-4-thiomethyl]-7-[2-(2-amino-4-thiazolyl)-2-(Z)-[(S)-carboxy-(3,4-dihydroxyphenyl)methyloxyimino]acetamido]ceph-3-em-4-carboxylate;
[6R,7R]-3-[1-Aminopyridinium-4-thiomethyl]-7-[2-(2-amino-4-thiazolyl)-2-(Z)-[(R)-carboxy(3,4-dihydroxyphenyl)methyloxyimino]acetamido]ceph-3-em-4-carboxylate;
[6R,7R]-7-[2-(2-Amino-4-thiazolyl)-2-(Z)-[3,4-(methylenedioxy)benzyloxyimino]acetamido]-3-[1-(methylamino)pyridinium-4-thiomethyl)ceph-3-em-4-carboxylate;

12. A process as claimed in any of the preceding claims in which R¹⁷ is chloro or iodo.

13. A process for the preparation of a pharmaceutical composition which process comprises admixing a pharmaceutically acceptable compound as defined in any one of claims 1 to 11 with a pharmaceutically acceptable excipient or carrier.

14. A process as claimed in claim 13 further comprising the admixing of a β-lactamase inhibitor.

15. A compound of formula (Ia) as defined in claim 2 or a pharmaceutically acceptable salt or in vivo hydrolysable ester thereof for use in the manufacture of a medicament for the treatment of bacterial infection.

## Patentansprüche (Patentansprüche für folgende(n) Vertragsstaat(en): AT, BE, CH, DE, DK, FR, GB, GR, IT, LI, LU, NL, SE)

1. Verbindung der Formel (I): 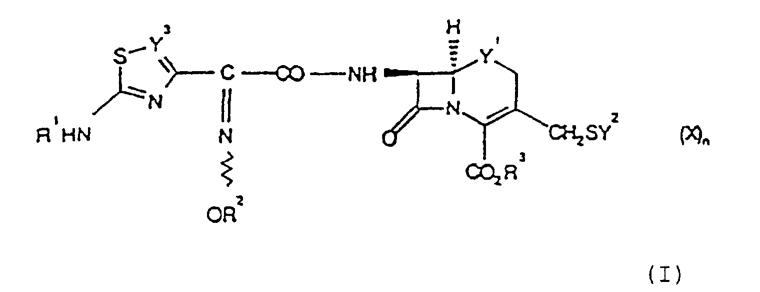 wobei
Y¹ für Schwefel, -SO- oder SO₂-,
R¹ für Wasserstoff oder eine Amino-Schutzgruppe,
R² für (C₁-C₁₂)-Alkyl, (C₂-C₁₂)-Alkenyl, (C₂-C₁₂)-Alkynyl, (C₃₋₇)-Cycloalkyl, (C₅₋₈)-Cycloalkenyl steht, wobei jedes derselben mit einer Phenylgruppe substituiert ist, die unsubstituiert oder bis zu dreimal mit
(C₁₋₆)-Alkoxy oder Hydroxy substituiert sein kann, und in der ein jeder der oben definierten R²-Substituenten gegebenenfalls des Weiteren mit Carboxyl substituiert ist,
oder R² für (Methylendioxy)-benzyl,
CO₂R³ für Carboxy oder ein Carboxylat-Anion steht oder die R³-Gruppe eine ohne Weiteres zu entfernende Carboxy-Schutzgruppe darstellt,
Y² für eine Gruppe der Formel: 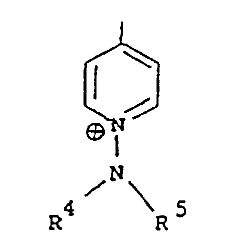 steht,
wobei die Pyridinium-Gruppe unsubstituiert oder an einem Ringkohlenstoffatom substituiert ist, das für die Substitution durch bis zu vier unter (C₁₋ ₆)-Alkyl und (C₁₋₆)-Alkoxy ausgewählten Substituenten verfügbar ist,
R⁴ und R⁵, die gleich oder verschieden sein können, unter Wasserstoff oder einer R⁶-Gruppe ausgewählt werden,
R⁶ für (C₁₋₆)-Alkyl, (C₃₋₇)-Cycloalkyl, (C₂₋₆)-Alkenyl, (C₅₋₈)-Cycloalkenyl, (C₂₋₆)-Alkynyl steht, wobei jedes derselben unsubstituiert oder durch Halogen, Cyano, Azido, Nitro, Carboxy, (C₁₋₆)-Alkoxycarbonyl, Carbamoyl, Mono- oder Di-(C₁₋₆)-alkylcarbamoyl, Sulfono, Sulfamoyl, Mono- und Di-(C₁₋₆)-alkylsulfamoyl, Amino, Mono- und Di-(C₁₋₆)-Alkylamino, Acylamino, (C₁₋₆)-Alkoxycarbonylamino, Hydroxy, (C₁₋₆)-Alkoxy, Acyloxy, Oxo, Phenylcarbonyl, Naphthylcarbonyl, Heterocyclylcarbonyl, (C₁₋₆)-Alkylthio, (C₁₋₆)-Alkansulfinyl, (C₁₋₆)-Alkansulfonyl, Phenyl, Naphthyl oder Heterocyclyl substituiert sein kann, wobei das Phenyl oder Naphthyl unsubstituiert oder bis zu fünfmal durch Halogen, (C₁₋₆)-Alkyl, Phenyl, (C₁₋₆)-Alkoxy, Hydroxy-(C₁₋₆)-alkyl, Mercapto-(C₁₋₆)-alkyl, Halo(C₁₋₆)-alkyl, Mercapto, Hydroxy, Amino, Mono- oder Di-(C₁₋₆)-alkylamino, Nitro, Carboxy, (C₁₋₆)-Alkoxycarbonyl, (C₁₋₆)-Alkoxycarbonyl-(C₁₋₆)-alkyl, (C₁₋₆)-Alkylcarbonyloxy, Formyl oder (C₁₋₆)-Alkylcarbonyl substituiert sein kann,
wobei der Heterocyclyl-Anteil unsubstituiert oder bis zu dreimal durch(C₁₋₆)-Alkyl, (C₁₋₆)-Alkoxy, (C₁₋₆)-Alkoxy(C₁₋₆)-alkyl, Halo(C₁₋₆)-alkyl, Hydroxy, Amino, Mono- oder Di-(C₁₋₆)-alkylamino, Carboxy, Carbamoyl, (C₁₋₆)-Alkoxycarbonyl, (C₁₋₆)-Alkoxycarbonyl(C₁₋₆)-alkyl, Oxo substituiert sein kann, und der Heterocyclyl-Anteil aromatische oder nichtaromatische, einfache oder bicyclische Ringe darstellt, die bis zu vier Heteroatome in jedem Ring enthalten, die unter Sauerstoff, Stickstoff und Schwefel ausgewählt werden, oder R⁶ für Pyridyl, Isoxazolylmethyl, Thiazolymethyl, Chloderopyridinyl, Pyrazinyl, Imidazolinyl, Benzopyridinyl, Benzoyl, 3,4-Dihydroxybenzoyl, 4-Nitrobenzoyl, 4-Methoxybenzoyl, 4-Carboxybenzoyl, 4-Aminobenzoyl, 2-Furanoyl, 3,4-Dihydroxycinnamoyl, Carbamoyl und N-Methylcarbamoyl steht,
Y³ für Stickstoff oder -CH steht,
X ein anorganisches oder organisches Anion darstellt und
n 0 oder 1 beträgt,
unter der Voraussetzung, dass:
(i) wenn CO₂R³ für Carboxylat steht, n 0 beträgt und
(ii) wenn CO₂R³ für Carboxy steht oder die R³-Gruppe eine leicht zu entfernende Carboxy-Schutzgruppe darstellt, n 1 beträgt und das Anion X im entsprechenden stöchiometrischen Verhältnis vorliegt, um die positive Ladung an der Pyridinium-Gruppe auszugleichen.

2. Verbindung nach Anspruch 1, bei der R² ein substituiertes (C₁₋₆)-Alkyl darstellt.

3. Verbindung nach Anspruch 2, bei der R² ein Methyl darstellt, das durch eine Phenyl-Gruppe substituiert ist, die unsubstituiert oder bis zu dreimal mit (C₁₋₆)-Alkoxy oder Hydroxy substituiert ist.

4. Verbindung nach Anspruch 3, bei der das Methyl des Weiteren mit einer Carboxy-Gruppe substituiert ist.

5. Verbindung nach Anspruch 2, bei der R² für Carboxy(3,4-dihydroxyphenyl)methyl oder (Methylendioxy)benzyl steht.

6. Verbindung nach Anspruch 3, bei der R⁴ und R⁵ jeweils einzeln unter Wasserstoff, Methyl, Ethyl, Carboxymethyl, Methoxyethyl, Cyanomethyl, Propargyl, 4-Carboxy-butan-1-yl, 2-Amino-2-(methoxycarbonyl)ethyl, Cyclopropylmethyl, Propyl, Cyclopentyl, Prop-2-en-1-yl, Butyl, Hexyl, Isopropyl, 2-Hydroxyethyl, Isoxazolylmethyl, Thiazolylmethyl ausgewählt werden.

7. Verbindung der Formel (Ia) oder pharmazeutisch akzeptables Salz oder pharmazeutisch akzeptabler, *in vivo* hydrolysierbarer Ester desselben: 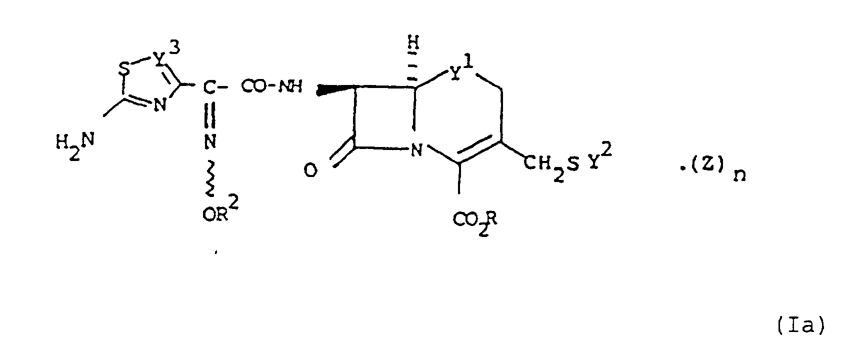 wobei R², Y¹, Y², Y³ und n die in Anspruch 1 angegebene Definition aufweisen, die Co₂R-Gruppe Carboxy oder ein Carboxylat-Anion darstellt und Z ein pharmazeutisch akzeptables anorganisches oder organisches Anion darstellt, das im entsprechenden stöchiometrischen Verhältnis vorliegt, um die positive Ladung am Pyridinium-Ring der Y²-Gruppe auszugleichen.

8. Verbindung nach Anspruch 1 der Formel (II): 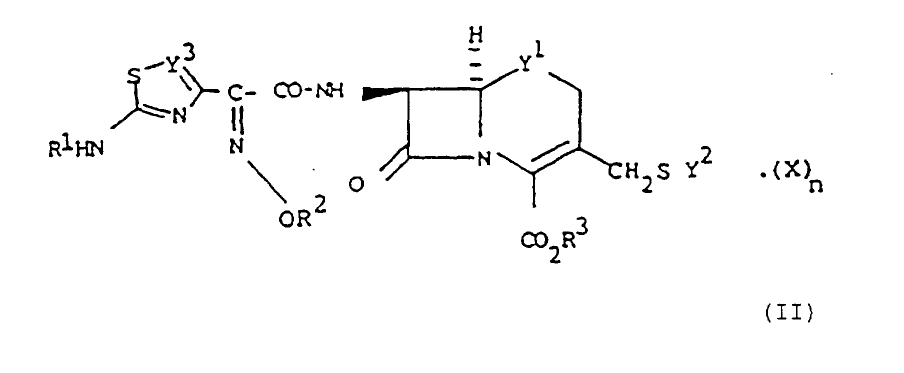 in der R¹, R², R³, X, Y¹, Y², Y³ und n die mit Bezug auf Formel (I) gemäß Anspruch 1 angegebene Definition aufweisen.

9. Pharmazeutische Zusammensetzung, die eine Verbindung der Formel (Ia), wie in Anspruch 7 definiert, oder ein pharmazeutisch akzeptables Salz oder einen *in vivo* hydrolysierbaren Ester desselben in Verbindung mit einem pharmazeutisch akzeptablen Bindemittel oder Verdünnungsmittel umfasst.

10. Pharmazeutische Zusammensetzung nach Anspruch 9, die des Weiteren einen β-Lactamase-Inhibitor umfasst.

11. Verbindung nach Anspruch 8, bei der R² ein (C₁₋₆)-Alkyl oder (C₃₋₇)-Cycloalkyl darstellt, wobei jedes mit einer Phenyl-Gruppe substituiert ist, die unsubstituiert oder bis zu dreimal mit (C₁₋₆)-Alkoxy oder Hydroxy substituiert sein kann.

12. Verbindung nach Anspruch 11, wobei R² für Methyl steht und gegebenenfalls des Weiteren durch eine Carboxy-Gruppe substituiert sein kann.

13. Verbindung nach Anspruch 11, wobei R² für Carboxy(3,4-dihydroxyphenyl)methyl steht.

14. Verbindung nach Anspruch 8, wobei R⁴ und R⁵ jeweils einzeln unter Wasserstoff, Methyl, Ethyl, Carboxymethyl, Methoxyethyl, Cyanomethyl, Propargyl, 4-Carboxy-butan-1-yl, 2-Amino-2-(methoxycarbonyl)ethyl, Cyclopropylmethyl, Propyl, Cyclopentyl, Prop-2-en-1-yl, Butyl, Hexyl, Isopropyl, 2-Hydroxyethyl, Pyridyl, Isoxazolylmethyl, Thiazolylmethyl, Chloderopyridinyl, Pyrazinyl, Imidazolinyl, Benzopyrazidinyl, Acetyl, Benzoyl, 3,4-Dihydroxybenzoyl, 4-Nitrobenzoyl, 4-Methoxybenzoyl, 4-Carboxybenzoyl, 4-Aminobenzoyl, 2-Furanoyl, 3,4-Dihydroxycinnamoyl, Carbamoyl, N-Methyl-carbamoyl und tert.-Butyloxycarbonyl ausgewählt werden.

15. Verbindung nach Anspruch 7, die [6R,7R]-7-[2-(2-Amino-4-thiazolyl)-2-(Z)-[(R)-carboxy(3,4-dihydroxyphenyl)-methyloxyimino]-acetamido]-3-[1-(methylamino)pyridinium-4-thiomethyl]ceph-3-em-4-carboxylat oder pharmazeutisch akzeptable Salze derselben darstellt.

16. Verbindung nach Anspruch 7, die [6R,7R]-7-[2-(2-Amino-4-thiazolyl)-2-(Z)-[(R)-carboxy (3,4-dihydroxyphenyl)-methyloxyimino]-acetamido]-3-[1-(methylamino)pyridinium-4-thiomethyl]ceph-3-em-4-carbonsäure oder pharmazeutisch akzeptable Salze desselben darstellt.

## Patentansprüche (Patentansprüche für folgende(n) Vertragsstaat(en): ES)

1. Verfahren für die Zubereitung einer Verbindung der Formel (I) oder eines Salzes derselben: 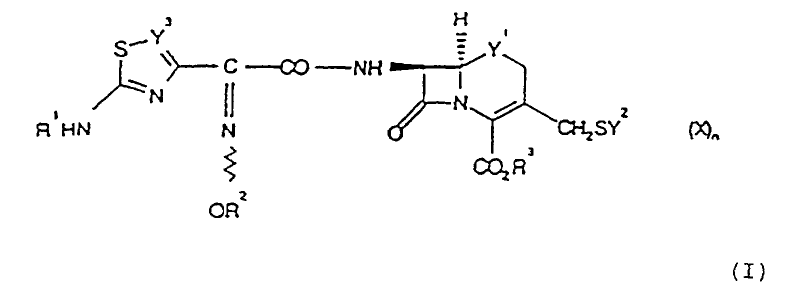 wobei
Y¹ für Schwefel, -SO- oder SO₂-,
R¹ für Wasserstoff oder eine Amino-Schutzgruppe,
R2 für(C₁-C₁₂)-Alkyl, (C₂-C₁₂)-Alkenyl, (C₂-C₁₂)-Alkynyl, (C₃₋₇)-Cycloalkyl, (C₅₋₈)-Oycloalkenyl steht, wobei jedes derselben mit einer Phenylgruppe substituiert ist, die unsubstituiert oder bis zu dreimal mit
(C₁₋₆)-Alkoxy oder Hydroxy substituiert sein kann, und in der ein jeder der oben definierten R²-Substituenten gegebenenfalls des Weiteren mit Carboxyl substituiert ist,
oder R² für (Methylendioxy)-benzyl,
CO₂R³ für Carboxy oder ein Carboxylat-Anion steht oder die R³-Gruppe eine ohne Weiteres zu entfernende Carboxy-Schutzgruppe darstellt,
Y² für eine Gruppe der Formel: 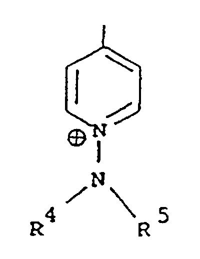 steht,
wobei die Pyridinium-Gruppe unsubstituiert oder an einem Ringkohlenstoffatom substituiert ist, das für die Substitution durch bis zu vier unter (C₁₋ ₆)-Alkyl und (C₁₋₆)-Alkoxy ausgewählten Substituenten verfügbar ist,
R⁴ und R⁵, die gleich oder verschieden sein können, unter Wasserstoff oder einer R⁶-Gruppe ausgewählt werden,
R⁶ für (C₁₋₆)-Alkyl, (C₃₋₇)-Cycloalkyl, (C₂₋₆)-Alkenyl, (C₅₋₈)-Cycloalkenyl, (C₂₋₆)-Alkynyl steht, wobei jedes derselben unsubstituiert oder durch Halogen, Cyano, Azido, Nitro, Carboxy, (C₁₋₆)-Alkoxycarbonyl, Carbamoyl, Mono- oder Di-(C₁₋₆)-alkylcarbamoyl, Sulfono, Sulfamoyl, Mono- und Di-(C₁₋₆)-alkylsulfamoyl, Amino, Mono- und Di-(C₁₋₆)-Alkylamino, Acylamino, (C₁₋₆)-Alkoxycarbonylamino, Hydroxy, (C₁₋₆)-Alkoxy, Acyloxy, Oxo, Phenylcarbonyl, Naphthylcarbonyl, Heterocyclylcarbonyl, (C₁₋₆)-Alkylthio, (C₁₋₆)-Alkansulfinyl, (C₁₋₆)-Alkansulfonyl, Phenyl, Naphthyl oder Heterocyclyl substituiert sein kann, wobei das Phenyl oder Naphthyl unsubstituiert oder bis zu fünfmal durch Halogen, (C₁₋₆)-Alkyl, Phenyl, (C₁₋₆)-Alkoxy, Hydroxy-(C₁₋₆)-alkyl, Mercapto-(C₁₋₆)-alkyl, Halo(C₁₋₆)-alkyl, Mercapto, Hydroxy, Amino, Mono- oder Di-(C₁₋₆)-alkylamino, Nitro, Carboxy, (C₁₋₆)-Alkoxycarbonyl, (C₁₋₆)-Alkoxycarbonyl-(C₁₋₆)-alkyl, (C₁₋₆)-Alkylcarbonyloxy, Formyl oder (C₁₋₆)-Alkylcarbonyl substituiert sein kann,
wobei der Heterocyclyl-Anteil unsubstituiert oder bis zu dreimal durch(C₁₋₆)-Alkyl, (C₁₋₆)-Alkoxy, (C₁₋₆)-Alkoxy(C₁₋₆)-alkyl, Halo (C₁₋₆)-alkyl, Hydroxy, Amino, Mono- oder Di-(C₁₋₆)-alkylamino, Carboxy, Carbamoyl, (C₁₋₆)-Alkoxycarbonyl, (C₁₋₆)-Alkoxycarbonyl(C₁₋₆)-alkyl, Oxo substituiert sein kann, und der Heterocyclyl-Anteil aromatische oder nichtaromatische, einfache oder bicyclische Ringe darstellt, die bis zu vier Heteroatome in jedem Ring enthalten, die unter Sauerstoff, Stickstoff und Schwefel ausgewählt werden, oder R⁶ für Pyridyl, Isoxazolylmethyl, Thiazolymethyl, Chloderopyridinyl, Pyrazinyl, Imidazolinyl, Benzopyridinyl, Benzoyl, 3,4-Dihydroxybenzoyl, 4-Nitrobenzoyl, 4-Methoxybenzoyl, 4-Carboxybenzoyl, 4-Aminobenzoyl, 2-Furanoyl, 3,4-Dihydroxycinnamoyl, Carbamoyl und N-Methylcarbamoyl steht,
Y³ für Stickstoff oder -CH steht,
X ein anorganisches oder organisches Anion darstellt und
n 0 oder 1 beträgt,
unter der Voraussetzung, dass:
(i) wenn CO₂R³ für Carboxylat steht, n 0 beträgt und
(ii) wenn CO₂R³ für Carboxy steht oder die R³-Gruppe eine leicht zu entfernende Carboxy-Schutzgruppe darstellt, n 1 beträgt und das Anion X im entsprechenden stöchiometrischen Verhältnis vorliegt, um die positive Ladung an der Pyridinium-Gruppe auszugleichen, welches Verfahren Folgendes umfasst:
a) Behandeln einer Verbindung der Formel (V): 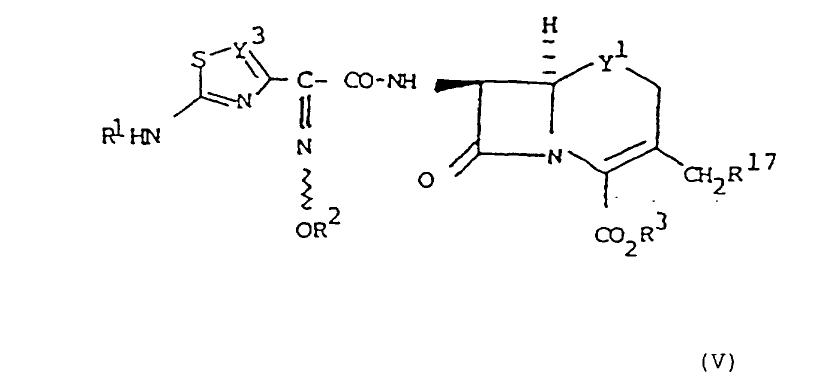 wobei R¹, R², R³, Y¹, und Y³ die oben angegebene Definition aufweisen und R¹⁷ eine Austrittsgruppe darstellt und wobei irgendwelche reaktionsfähigen Gruppen geschützt sein können,
mit einer Thiopyridonverbindung der Formel (VI): 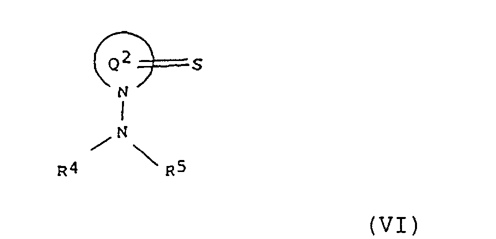 wobei der Ring: 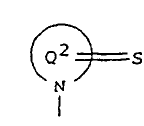 derart ausgebildet ist, dass er im Laufe der Reaktion *in situ* in den Ring der Y²-Gruppe (wie in Anspruch 1 definiert) umgewandelt wird,
und R⁴ und R⁵ die oben angegebene Definition aufweisen,
unter der Voraussetzung, dass, wenn R¹⁷ eine AcyloxyGruppe darstellt, -CO₂R³ in Form der freien Säure oder eines Salzes derselben vorliegt,
und nötigenfalls darauffolgendes Ausführen eines oder mehrerer der folgenden Schritte:
i) Umwandeln einer jeden oder irgendeiner der Gruppen R²-, R³-, R⁴- und R⁵-Gruppen in eine andere R²-, R³-, R⁴- und R⁵-Gruppe,
ii) Entfernen irgendeiner der Schutzgruppen, oder
iii) Umwandeln des Produkts in ein Salz, oder
(b) Reagieren einer Verbindung der Formel (VIII) oder eines Salzes derselben: 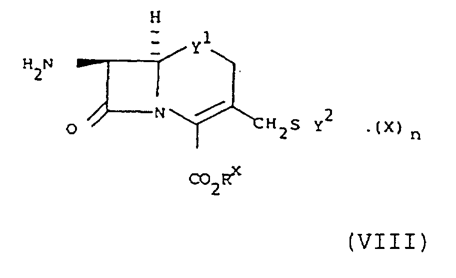
wobei X, Y¹, Y² und n die oben angegebene Definition aufweisen, R^{x} für Wasserstoff oder eine leicht zu entfernende Carboxyl blockierende Gruppe und die 7β-Aminogruppe gegebenenfalls mit einer Gruppe substituiert ist, die das Stattfinden der Acylierung ermöglicht, und irgendeine der reaktionsfähigen Gruppen geschützt sein kann,
mit einem N-acylierenden Derivat einer Säure der Formel (IX) : 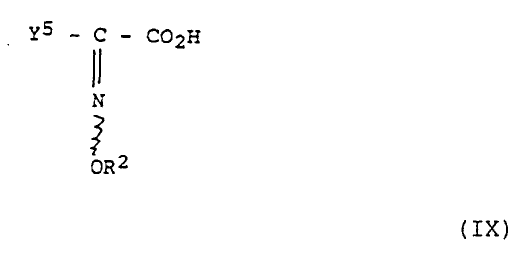 wobei R² die oben angegebene Definition aufweist und Y⁵ eine Gruppe der Formel: 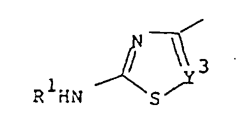 oder eine Gruppe darstellt, die in diese umgewandelt werden kann, und R¹ die oben angegebene Definition aufweist, und wobei irgendeine der reaktionsfähigen Gruppen geschützt sein kann, und nötigenfalls darauffolgendes Durchführen eines oder mehrerer der folgenden Schritte
(i) Entfernen irgendeiner der Schutzgruppen, einschließlich einer R¹-Amino-Schutzgruppe,
(ii) Umwandeln der R^{x}-Gruppe in eine R³-Gruppe,
(iii) Umwandeln des Produkts in ein Salz;
(iv) Umwandeln einer in Y⁵ umwandelbaren Gruppe in Y⁵, oder
(v) Umwandeln jeder oder irgendeiner der R⁴und R⁵-Gruppen in eine andere R⁴- und R⁵-Gruppe.

2. Verfahren nach Anspruch 1 für die Zubereitung einer Verbindung der Formel (Ia) oder eines pharmazeutisch akzeptablen Salzes oder eines pharmazeutisch akzeptablen, *in vivo* hydrolysierbaren Esters desselben: 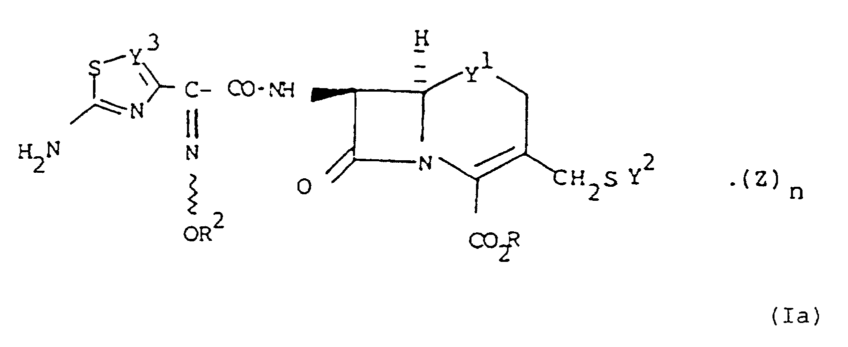 wobei R², Y¹, Y², Y³ und n die in Anspruch 1 angegebene Definition aufweisen, die CO₂R-Gruppe ein Carboxy- oder ein Carboxylat-Anion darstellt und Z ein pharmazeutisch akzeptables anorganisches oder organisches Anion darstellt, das im entsprechenden stöchiometrischen Verhältnis vorliegt, um die positive Ladung am Pyridinium-Ring der Y²-Gruppe auszugleichen.

3. Verfahren nach Anspruch 1 für die Zubereitung einer Verbindung der Formel (Ib): 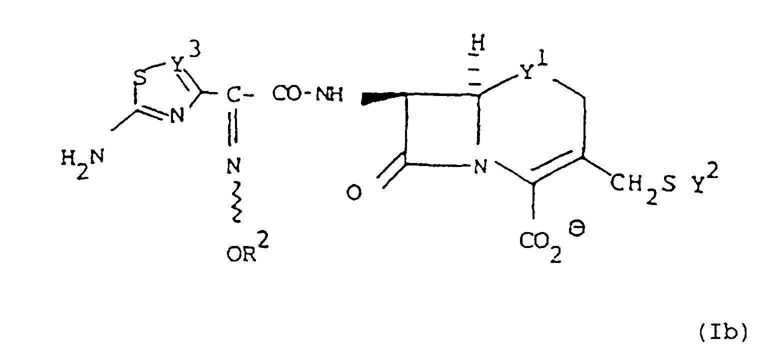 wobei R², Y¹, Y², und Y³ die mit Bezug auf Formel (Ia) angegebenen Definition aufweisen.

4. Verfahren nach Anspruch 1 für die Zubereitung einer Verbindung der Formel (Ic): 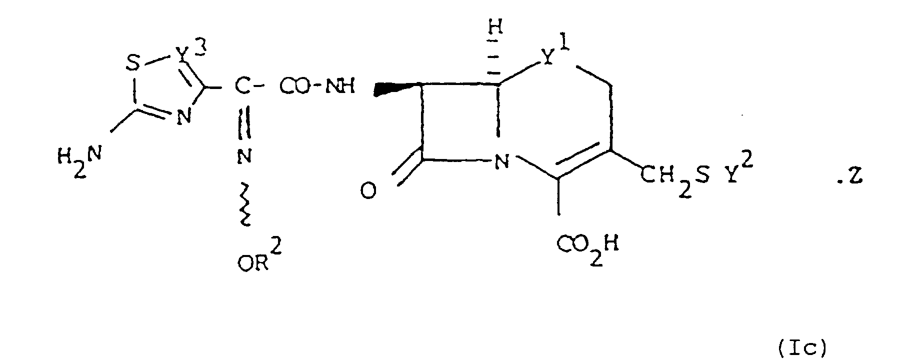 wobei R², Y¹, Y², Y³ und Z die mit Bezug auf Formel (Ia) angegebene Definition aufweisen.

5. Verfahren nach Anspruch 1 für die Zubereitung einer Verbindung der Formel (II): 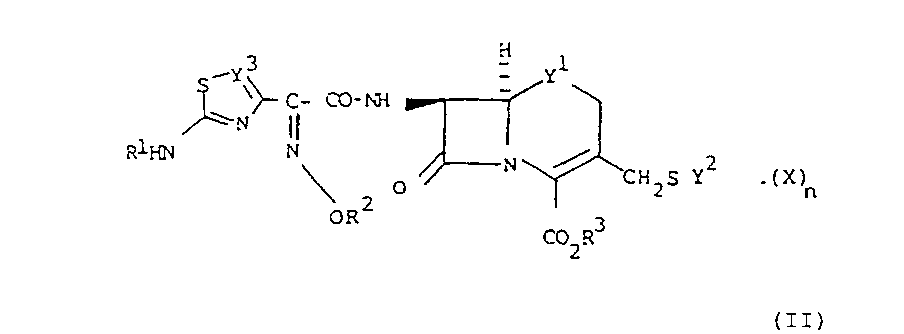 wobei R¹, R², R³, X, Y¹, Y², Y³ und n die mit Bezug auf Formel (I) angegebene Definition aufweisen.

6. Verfahren nach einem der Ansprüche 1 to 5, bei dem R² für (C₁₋₆)-Alkyl oder (C₃₋₇)-Cycloalkyl steht, wobei jedes mit einer Phenyl-Gruppe substituiert ist, die unsubstituiert oder bis zu dreimal mit (C₁₋₆)-Alkoxy oder Hydroxy substituiert sein kann.

7. Verfahren nach Anspruch 6, bei dem R² für Methyl steht, das gegebenenfalls des Weiteren durch Carboxy substituiert ist.

8. Verfahren nach Anspruch 7, bei dem R² für 3,4-Dihydroxyphenylmethyl steht, das gegebenenfalls des Weiteren durch Carboxy substituiert ist.

9. Verfahren nach Anspruch 6, bei dem R² unter Carboxy (3,4-dihydroxy-phenyl)methyl und (Methylendioxy)benzyl ausgewählt wird.

10. Verfahren nach Anspruch 1, bei dem R⁴ und R⁵ jeweils einzeln unter Wasserstoff, Methyl, Ethyl, Carboxymethyl, Methoxyethyl, Cyanomethyl, Propargyl, 4-Carboxy-butan-l-yl, 2-Amino-2-(methoxycarbonyl)ethyl, Cyclopropylmethyl, Propyl, Cyclopentyl, Prop-2-en-1-yl, Butyl, Hexyl, Isopropyl, 2-Hydroxyethyl, Pyridyl, Isoxazolylmethyl, Thiazolylmethyl, Chloderopyridinyl, Pyrazinyl, Imidazolinyl, Benzopyrazidinyl, Acetyl, Benzoyl, 3,4-Dihydroxybenzoyl, 4-Nitrobenzoyl, 4-Methoxybenzoyl, 4-Carboxybenzoyl, 4-Aminobenzoyl, 2-Furanoyl, 3,4-Dihydroxycinnamoyl, Carbamoyl, N-Methylcarbamoyl und tert.-Butyoxycarbonyl ausgewählt wird.

11. Verfahren nach einem der vorhergehenden Ansprüche für die Zubereitung von unter den folgenden pharmazeutisch akzeptablen Salzen und *in vivo* hydrolysierbaren Estern derselben ausgewählten:
[6R,7R]-7-[2-(2-Amino-4-thiazolyl)-2-(Z)-[(R,S)-carboxy(3,4-dihydroxyphenyl)methyloxyimino]acetamido]-3-[1-(methylamino)pyridinium-4-thiomethyl]ceph-3-em-4-carboxylat;
[6R,7R]-7-[2-(2-Amino-4-thiazolyl)-2-(Z)-[(S)-carboxy(3,4-dihydroxyphenyl)methyloxyimino]acetamido]-3-[1-(methylamino)pyridinium-4-thiomethyl]ceph-3-em-4-carboxylat;
[6R,7R]-7-[2-(2-Amino-4-thiazolyl)-2-(Z)-[(R)-carboxy(3,4-dihydroxyphenyl)methyloxyimino]acetamido]-3-[1-(methylamino)pyridinium-4-thiomethyl]ceph-3-em-4-carboxylat;
[6R,7R]-3-[1-Aminopyridinium-4-thiomethyl]-7-[2-(2-amino-4-thiazolyl)-2-(Z)-[(R,S)-carboxy(3,4-dihydroxyphenyl)methyloxyimino]acetamido]ceph-3-em-4-carboxylat;
[6R,7R]-3-[1-Aminopyridinium-4-thiomethyl]-7-[2-(2-amino-4-thiazolyl)-2-(Z)-[(S)-carboxy(3,4-dihydroxyphenyl)methyloxyimino]acetamido]ceph-3-em-4-carboxylat;
[6R,7R]-3-[1-Aminopyridinium-4-thiomethyl]-7-[2(2-amino-4-thiazolyl)-2-(Z)-[(R)-carboxy(3,4-dihydroxyphenyl)methyloxyimino]acetamido]ceph-3-em-4-carboxylat;
[6R,7R]-7-[2-(2-Amino-4-thiazolyl)-2-(Z)-[3,4-(methylendioxy)benzyloxyimino]acetamido]-3-[1-(methylamino)pyridinium-4-thiomethyl]ceph-3-em-4-carboxylat.

12. Verfahren nach einem der vorhergehenden Ansprüche, bei dem R¹⁷ für Chloro oder Iodo steht.

13. Verfahren für die Zubereitung einer pharmazeutischen Zusammensetzung, welches Verfahren das Vermischen einer pharmazeutisch akzeptablen Verbindung, wie in einem der Ansprüche 1 bis 11 definiert, mit einem pharmazeutisch akzeptablen Bindemittel oder Träger umfasst.

14. Verfahren nach Anspruch 13, das des Weiteren das Zumischen eines β-Lactamase-Inhibitors umfasst.

15. Verbindung der Formel (Ia), wie in Anspruch 2 definiert, oder pharmazeutisch akzeptables Salz oder *in vivo* hydrolysierbarer Ester desselben für die Verwendung bei der Herstellung eines Medikaments für die Behandlung einer bakteriellen Infektion.

## Revendications (Revendications pour l'(les) Etat(s) contractant(s) suivant(s): AT, BE, CH, DE, DK, FR, GB, GR, IT, LI, LU, NL, SE)

1. Composé de formule (I) : 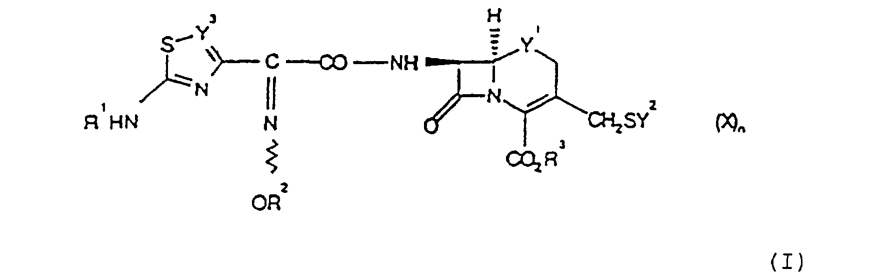 dans laquelle
Y¹ est le soufre, -SO-, ou SO₂- ;
R¹ est l'hydrogène ou un groupement de protection amino ;
R² est un radical (C₁-C₁₂)alkyle, (C₂-C₁₂)alkényle, (C₂-C₁₂)alkynyle, (C₃₋₇)cycloalkyle, (C₅₋₈)cycloalkényle, chacun d'eux étant substitué par un groupement phényle qui peut être non substitué ou substitué jusqu'à 3 fois avec le radical (C₁₋₆)alkoxy ou hydroxy ; et dans lequel chacun des substituants R² définis antérieurement est, en option, en plus substitué par le radical carboxyle ;
ou R² est le radical (méthylènedioxy)-benzyle ;
CO₂R³ est le radical carboxy ou l'anion carboxylate, ou le groupement R³ est un groupement de protection carboxy facile à éliminer ;
Y² est un groupement de formule : 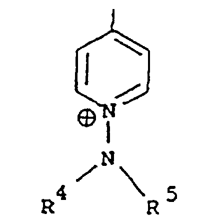 dans lequel le groupement pyridinium est non substitué ou substitué à un atome de carbone de cycle, disponible en vue d'une substitution de jusqu'à quatre substituants sélectionnés parmi les radicaux (C₁₋₆)alkyle, et (C₁₋₆)alkoxy ;
R⁴ et R⁵, qui peuvent être identiques ou différents, sont sélectionnés parmi l'hydrogène ou un groupement R⁶ ;
R⁶ est le radical (C₁₋₆)alkyle, (C₃₋₇)cycloalkyle, (C₂₋ ₆) alkényle, (C₅₋₈)cycloalkényle, (C₂₋₆)alkynyle, chacun d'eux pouvant être non substitué ou substitué par un radical halogène, cyano, azido, nitro, carboxy, (C₁₋₆)alkoxycarbonyle, carbamoyle, mono- ou di-(C₁₋₆)alkylcarbamoyle, sulfono, sulfamoyle, mono- et di-(C₁₋₆)alkylsulfamoyle, amino, mono- et di-(C₁₋₆)alkylamino, acylamino, (C₁₋ ₆) alkoxycarbonylamino, hydroxy, (C₁₋₆)alkoxy, acyloxy, oxo, phénylcarbonyle, naphtylcarbonyle, hétérocyclylcarbonyle, (C₁₋₆)alkylthio, (C₁₋ ₆) alkanesulfinyle, (C₁₋₆)alkanesulfonyl, phényle, naphtyle ou hétérocyclyle ;
dans lequel le radical phényle ou naphtyle peut être non substitué ou substitué jusqu'à 5 fois par le radical halogène, (C₁₋₆)alkyl, phényle, (C₁₋₆)alkoxy, hydroxy(C₁₋₆)alkyle, mercapto(C₁₋₆)alkyle, halo(C₁₋ ₆)alkyle, mercapto, hydroxy, amino, mono- ou di-(C₁₋ ₆)alkylamino, nitro, carboxy, (C₁₋₆)alkoxycarbonyle, (C₁₋₆)alkoxycarbonyle-(C₁₋₆) alkyle, (C₁₋ ₆)alkylcarbonyloxy, formyle, ou (C₁₋ ₆)alkylcarbonyle ; dans lequel la moitié hétérocyclique peut être non substituée ou substituée jusqu'à 3 fois par les radicaux (C₁₋ ₆)alkyle, (C₁₋₆)alkoxy, (C₁₋₆)alkoxy(C₁₋₆)alkyle, halo(C₁₋₆)alkyle, hydroxy, amino, mono -ou di-(C₁₋ ₆)alkylamino, carboxy, carbamoyle, (C₁₋ ₆) alkoxycarbonyle, (C₁₋₆) alkoxycarbonyl(C₁₋₆)alkyle, oxo ; et la moitié hétérocyclique est aromatique ou non aromatique, des cycles simples ou bicycliques contenant jusqu'à quatre hétéroatomes dans chaque cycle sélectionné parmi l'oxygène, l'azote, et le soufre ; ou R⁶ est le radical pyridyle ; isoxazolylméthyle ; thiazolylméthyle ; chloropyridinyle ; pyrazinyle ; imidazolinyle ; benzopyridinyle ; benzoyle ; 3,4-dihydroxybenzoyle ; 4-nitrobenzoyle ; 4-méthoxybenzoyle ; 4-carboxybenzoyle ; 4-aminobenzoyle ; 2-furanoyle ; 3,4-dihydroxycinnamoyle ; carbamoyle ; et N-méthylcarbamoyle.
Y³ est l'azote ou le radical -CH ;
X est un anion inorganique ou organique, et
n est 0 ou 1,
sous réserve que :
(i) quand CO₂R³ est le radical carboxylate, n est 0, et
(ii) quand CO₂R³ est le radical carboxy ou le groupement R³ est un groupement de protection facile à éliminer, alors n est 1, et l'anion X est présent dans la proportion stoechiométrique appropriée pour équilibrer la charge positive sur le groupement pyridinium.

2. Composé selon la revendication 1 dans lequel R² est un radical (C₁₋₆)alkyle substitué.

3. Composé selon la revendication 2 dans lequel R² est un radical méthyle substitué par un groupement phényle qui est non substitué ou substitué jusqu'à 3 fois par le radical (C₁₋₆)alkoxy ou hydroxy.

4. Composé selon la revendication 3 dans lequel le radical méthyle est substitué en plus par un groupement carboxy.

5. Composé selon la revendication 2 dans lequel R² est un radical carboxy(3,4-dihydroxyphényl)méthyle ou (méthylènedioxy)benzyle.

6. Composé selon la revendication 3 dans lequel R⁴ et R⁵ sont sélectionnés chacun parmi les radicaux hydrogène, méthyle, éthyle, carboxyméthyle, méthoxyéthyle, cyanométhyle, propargyle, 4-carboxybutan-1-yle, 2-amino-2-(méthoxycarbonyl)éthyle, cyclopropylméthyle, propyle, cyclopentyle, prop-2-èn-1-yle, butyle, hexyle, isopropyle, 2-hydroxyéthyle, isoxazolylméthyle, thiazolylméthyle.

7. Composé de formule (Ia) ou un sel pharmaceutiquement acceptable ou un ester hydrolysable *in vivo*, pharmaceutiquement acceptable, de ce dernier : 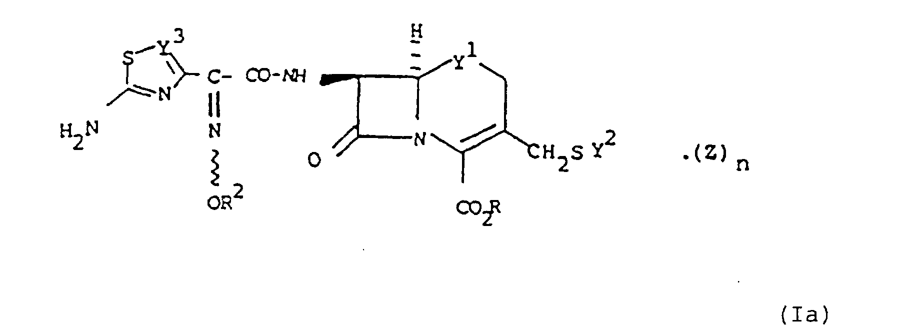 dans laquelle formule R², Y¹, Y², Y³ et n sont comme définis dans la revendication 1 ; le groupement CO₂R est un radical carboxy ou un anion carboxylate et Z est un anion inorganique ou organique pharmaceutiquement acceptable, présent dans la proportion stoechiométrique appropriée pour équilibrer la charge positive sur le cycle pyridinium du groupement Y².

8. Composé selon la revendication 1 de formule (II) : 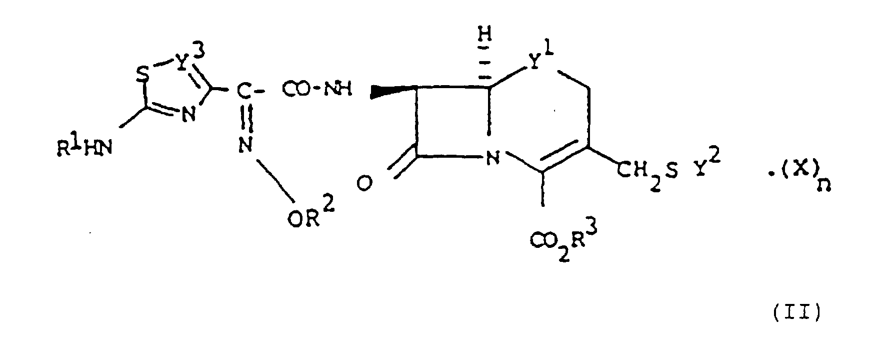 dans laquelle R¹, R², R³, X, Y¹, Y², Y³ et n sont comme définis par rapport à la formule (I) selon la revendication 1.

9. Composition pharmaceutique comprenant un composé de formule (Ia), comme défini dans la revendication 7, ou un sel pharmaceutiquement acceptable, ou un ester hydrolysable *in vivo* de ce dernier, en combinaison à un excipient ou à un diluant pharmaceutiquement acceptable.

10. Composition pharmaceutique selon la revendication 9, comprenant en outre un agent inhibiteur de la β-lactamase.

11. Composé selon la revendication 8 dans lequel R² est un radical (C₁₋₆)alkyle ou (C₃₋₇)cycloalkyle, chacun d'entre eux étant substitué à l'aide d'un groupement phényle qui peut être non substitué ou substitué jusqu'à 3 fois à l'aide d'un radical (C₁₋₆)alkoxy ou hydroxy.

12. Composé selon la revendication 11 dans lequel R² est le radical méthyle, substitué en plus, en option, par un groupement carboxy.

13. Composé selon la revendication 11 dans lequel R² est le radical carboxy(3,4-dihydroxyphényl)méthyle.

14. Composé selon la revendication 8 dans lequel R⁴ et R⁵ sont chacun sélectionnés parmi les radicaux hydrogène, méthyle, éthyle, carboxyméthyle, méthoxyéthyle, cyanométhyle, propargyle, 4-carboxybutan-1-yle, 2-amino-2-(méthoxycarbonyl)éthyle, cyclopropylméthyle, propyle, cyclopentyle, prop-2-èn-1-yle, butyle, hexyle, isopropyle, 2-hydroxyéthyle, pyridyle, isoxazolylméthyle, thiazolylméthyle, chloropyridinyle, pyrazinyle, imidazolinyle, benzopyrazidinyle, acétyle, benzoyle, 3,4-dihydroxybenzoyle, 4-nitrobenzoyle, 4-méthoxybenzoyle, 4-carboxybenzoyle, 4-aminobenzoyle, 2-furanoyle, 3,4-dihydroxycinnamoyle, carbamoyle, N-méthylcarbamoyle et t-butyloxycarbonyle.

15. Composé selon la revendication 7 qui est le composé [6R,7R]-7-[2-(2-amino-4-thiazolyl)-2-(Z)-[(R)-carboxy(3,4-dihydroxyphényl)-méthyloxyimino]-acétamido]-3-[1-(méthylamino)pyridinium-4-thiométhyl] céph-3-em-4-carboxylate ou des sels pharmaceutiquement acceptables de ce dernier.

16. Composé selon la revendication 7 qui est le composé [6R,7R]-7-[2-(2-amino-4-thiazolyl)-2-(Z)-[(R)-carboxy(3,4-dihydroxyphényl)-méthyloxyimino]-acétamido]-3-[1-(méthylamino)pyridinium-4-thiométhyl] céph-3-em-4-carboxylique ou des sels pharmaceutiquement acceptables de ce dernier.

## Revendications (Revendications pour l'(les) Etat(s) contractant(s) suivant(s): ES)

1. Procédé pour la préparation d'un composé de formule (I) ou un sel de ce dernier : 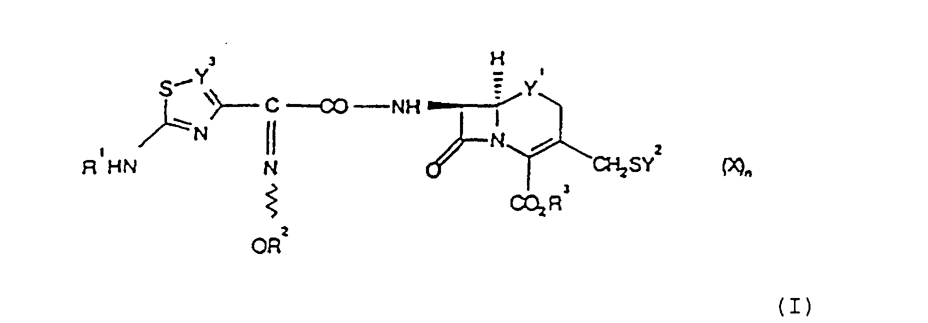 dans laquelle
Y¹ est le soufre, -SO-, ou SO₂- ;
R¹ est l'hydrogène ou un groupement de protection amino ;
R² est un radical (C₁-C₁₂)alkyle, (C₂-C₁₂)alkényle, (C₂-C₁₂)alkynyle, (C₃₋₇) cycloalkyle, (C₅₋₈) cycloalkényle, chacun d'eux étant substitué par un groupement phényle qui peut être non substitué ou substitué jusqu'à 3 fois avec le radical (C₁₋₆)alkoxy ou hydroxy ; et dans lequel chacun des substituants R² définis antérieurement est, en option, en plus substitué par le radical carboxyle ;
ou R² est le radical (méthylènedioxy)-benzyle ;
CO₂R³ est le radical carboxy ou l'anion carboxylate, ou le groupement R³ est un groupement de protection facile à éliminer ;
Y² est un groupement de formule : 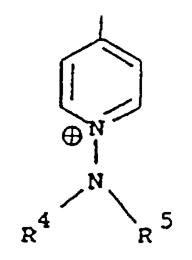 dans lequel le groupement pyridinium est non substitué ou substitué à un atome de carbone de cycle, disponible en vue d'une substitution de jusqu'à quatre substituants sélectionnés parmi les radicaux (C₁₋₆)alkyle, et (C₁₋₆)alkoxy ;
R⁴ et R⁵, qui peuvent être identiques ou différents, sont sélectionnés parmi l'hydrogène ou un groupement R⁶ ;
R⁶ est le radical (C₁₋₆)alkyle, (C₃₋₇)cycloalkyle, (C₂₋ ₆) alkényle,
(C₅₋₈) cycloalkényle, (C₂₋₆) alkynyle, chacun d'eux pouvant être non substitué ou substitué par un radical halogène, cyano, azido, nitro, carboxy, (C₁₋₆)alkoxycarbonyle, carbamoyle, mono- ou di-(C₁₋ ₆)alkylcarbamoyle, sulfono, sulfamoyle, mono- et di-(C₁₋₆)alkylsulfamoyle, amino, mono- et di-(C₁₋ ₆)alkylamino, acylamino, (C₁₋₆)alkoxycarbonylamino, hydroxy, (C₁₋₆)alkoxy, acyloxy, oxo, phénylcarbonyle, naphtylcarbonyle, hétérocyclylcarbonyle, (C₁₋₆)alkylthio, (C₁₋ ₆)alkanesulfinyle, (C₁₋₆)alkanesulfonyl, phényle, naphtyle ou hétérocyclyle ;
dans lequel le radical phényle ou naphtyle peut être non substitué ou substitué jusqu'à 5 fois par les radicaux halogène, (C₁₋₆)alkyle, phényle, (C₁₋ ₆)alkoxy, hydroxy(C₁₋₆)alkyle, mercapto(C₁₋₆)alkyle, halo(C₁₋₆)alkyle, mercapto, hydroxy, amino, mono- ou di-(C₁₋₆)alkylamino, nitro, carboxy, (C₁₋ ₆) alkoxycarbonyle, (C₁₋₆)alkoxycarbonyl-(C₁₋₆)alkyle, (C₁₋₆)alkylcarbonyloxy, formyle, ou (C₁₋ ₆)alkylcarbonyle ; dans lequel la moitié hétérocyclyle peut être non substituée ou substituée jusqu'à 3 fois par les radicaux (C₁₋ ₆) alkyle, (C₁₋₆)alkoxyl, (C₁₋₆) alkoxy(C₁₋₆) alkyle, halo(C₁₋₆)alkyle, hydroxy, amino, mono-ou di-(C₁₋ ₆)alkylamino, carboxy, carbamoyle, (C₁₋ ₆)alkoxycarbonyle, (C₁₋₆)alkoxycarbonyl (C₁₋₆)alkyle, oxo ; et la moitié hétérocyclyle est aromatique ou non aromatique, des cycles simples ou bicycliques contenant jusqu'à quatre hétéroatomes dans chaque cycle sélectionné parmi l'oxygène, l'azote, et le soufre ; ou R⁶ est le radical pyridyle ; isoxazolylméthyle ; thiazolylméthyle ; chloropyridinyle ; pyrazinyle ; imidazolinyle ; benzopyridinyle ; benzoyle ; 3,4-dihydroxybénzoyle ; 4-nitrobenzoyle ; 4-méthoxybenzoyle ; 4-carboxybenzoyle ; 4-aminobenzoyle ; 2-furanoyle ; 3,4-dihydroxycinnamoyle ; carbamoyle ; et N-méthylcarbamoyle.
Y³ est l'azote ou le radical -CH ;
X est un anion inorganique ou organique, et
n est 0 ou 1,
sous réserve que :
(i) quand CO₂R³ est le radical carboxylate, n est 0, et
(ii) quand CO₂R³ est le radical carboxy ou quand le groupement R³ est un groupement de protection facile à éliminer, alors n est 1, et l'anion X est présent dans la proportion stoechiométrique appropriée pour équilibrer la charge positive sur le groupement pyridinium ; lequel procédé comprend :
a) le traitement d'un composé de formule (V) : 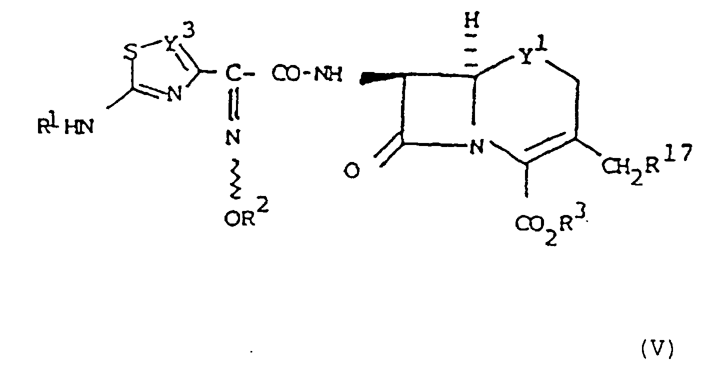 dans laquelle R¹, R², R³, Y¹, et Y³ sont comme définis jusqu'ici et R¹⁷ est un groupe partant ; et dans laquelle tout groupement réactif quelconque peut être protégé ;
avec un composé de la thiopyridone de formule (VI) : 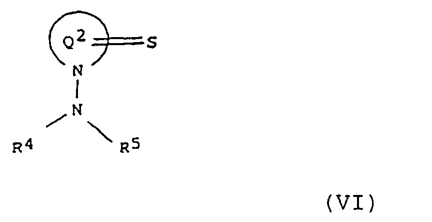 dans laquelle le noyau : 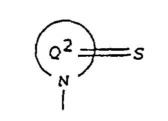 est tel qu'il est converti dans le noyau du groupement Y² (comme défini dans la revendication 1) *in situ* pendant le cours de la réaction ;
et R⁴ et R⁵ sont définis comme auparavant ;
sous réserve que, quand R¹⁷ est un groupement acyloxy, -CO₂R³ est sous la forme d'un acide libre ou sous la forme d'un sel de ce dernier ; et, dans la suite, si besoin est, en effectuant une ou plusieurs des étapes suivantes :
i) la conversion de chacun ou de l'un quelconque de ces groupes R², R³, R⁴ et R⁵ dans un groupe différent R², R³, R⁴ et R⁵ ;
ii) l'élimination de tout groupement de protection quelconque ; ou
iii) la conversion du produit en un sel ; ou
(b) la réaction d'un composé de formule (VIII) ou d'un sel de ce dernier : 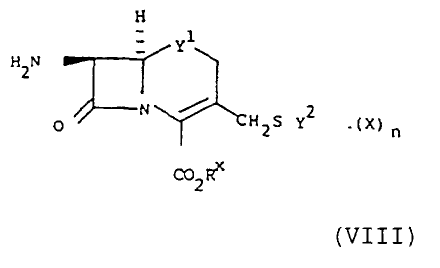
dans lequel X, Y¹, Y² et n sont définis comme auparavant, R^{x} est l'hydrogène ou un groupement de blocage carboxyle facile à éliminer et le groupement 7β-amino est, en option, substitué par un groupement qui permet de procéder à une acylation ; et tout groupement réactif quelconque peut être protégé ;
à l'aide d'un dérivé de N-acylation d'un acide de formule (IX) : 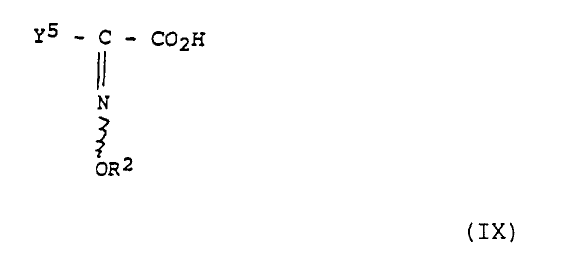 dans laquelle R² est défini comme auparavant et Y⁵ est un groupement de formule : 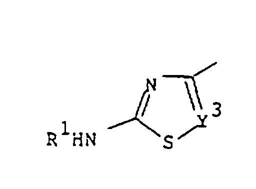 ou un groupement qui peut y être converti, et R¹ est comme défini auparavant ; et dans lequel tout groupement réactif quelconque peut être protégé ; et, ensuite, si besoin est, l'exécution d'une ou de plusieurs des étapes suivantes :
(i) l'élimination de tout groupement de protection quelconque, y compris un groupement de protection amino R¹ ;
(ii) la conversion du groupe R^{x} en un groupe R³ ;
(iii) la conversion du produit en sel ;
(iv) la conversion, en Y⁵, d'un groupe qui est capable d'une conversion en Y⁵, ou
(v) la conversion de chacun ou de l'un quelconque de ces groupes R⁴ et R⁵ dans un groupe différent R⁴ et R⁵ ;

2. Procédé selon la revendication 1 pour la préparation d'un composé de formule (la) ou d'un sel, pharmaceutiquement acceptable, ou d'un ester hydrolysable *in vivo,* pharmaceutiquement acceptable, de ce dernier : 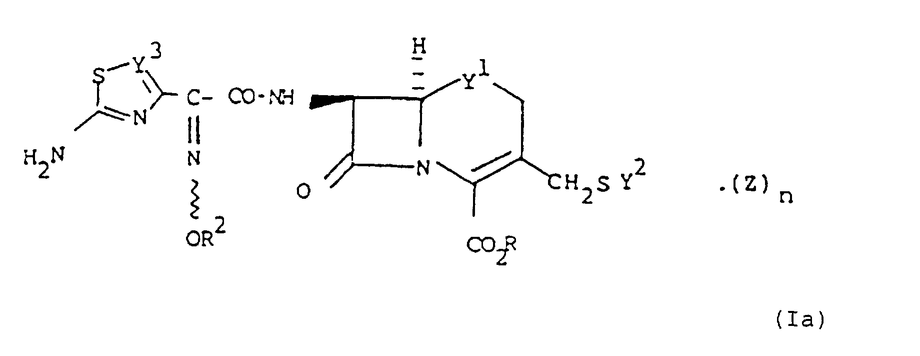 dans laquelle formule R², Y¹, Y², Y³ et n sont comme définis dans la revendication 1 ; le groupement CO₂R est un radical carboxy ou un anion carboxylate et Z est un anion inorganique ou organique pharmaceutiquement acceptable, présent dans la proportion stoechiométrique appropriée pour équilibrer la charge positive sur le cycle pyridinium du groupement Y².

3. Procédé selon la revendication 1 pour la préparation d'un composé de formule (Ib) : 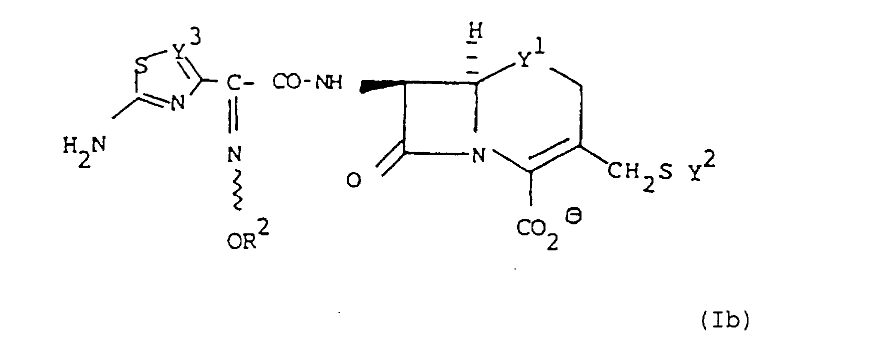 dans laquelle R², Y¹, Y², et Y³ sont définis comme auparavant par rapport à la formule (Ia).

4. Procédé selon la revendication 1 pour la préparation d'un composé de formule (Ic) : 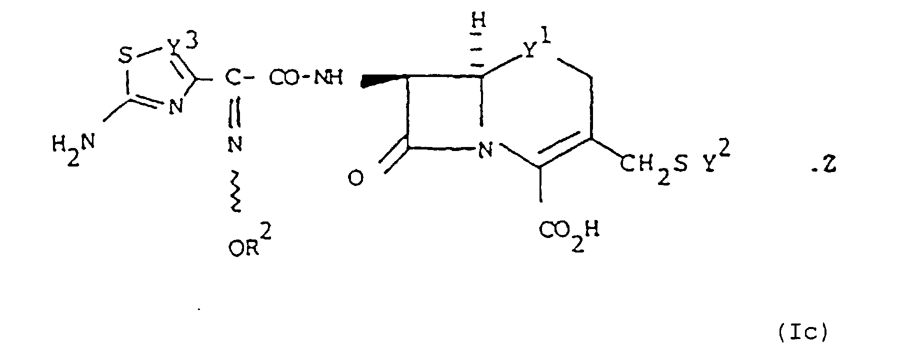 dans laquelle R², Y¹, Y², Y³ et Z sont définis comme auparavant par rapport à la formule (la).

5. Procédé selon la revendication 1 pour la préparation d'un composé de formule (II) : 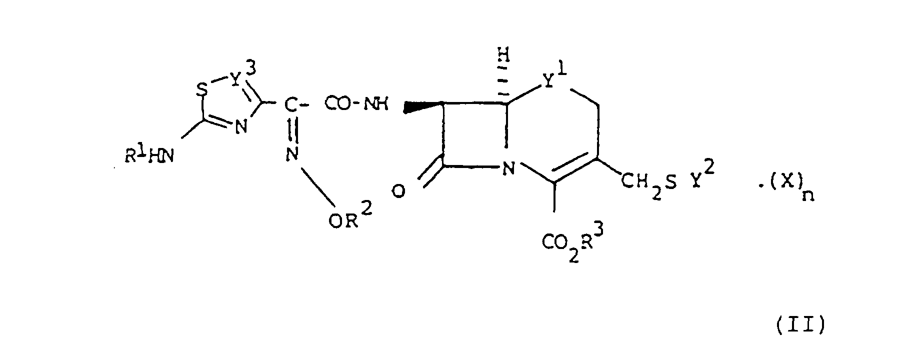 dans laquelle R¹, R², R³, X, Y¹, Y², Y³ et n sont comme définis par rapport à la formule (I).

6. Procédé selon l'une quelconque des revendications 1 à 5 dans lequel R² est un radical (C₁₋₆)alkyle ou (C₃₋ ₇)cycloalkyle, chacun d'eux étant substitué à l'aide d'un groupement phényle qui peut être non substitué ou substitué jusqu'à 3 fois à l'aide d'un groupement (C₁₋ ₆)alkoxy ou hydroxy.

7. Procédé selon la revendication 6, dans lequel R² est le radical méthyle, substitué en outre, en option, par un radical carboxy.

8. Procédé selon la revendication 7, dans lequel R² est le radical 3,4-dihydroxyphénylméthyle, substitué en outre, en option, par un radical carboxy.

9. Procédé selon la revendication 6, dans lequel R² est sélectionné parmi les radicaux carboxy (3,4-dihydroxyphényle)méthyle et (méthylènedioxy)benzyle.

10. Procédé selon la revendication 1 dans lequel R⁴ et R⁵ sont chacun sélectionnés du groupe des radicaux hydrogène, méthyle, éthyle, carboxyméthyle, méthoxyéthyle, cyanométhyle, propargyle, 4-carboxybutan-1-yle, 2-amino-2-(méthoxycarbonyl)éthyle, cyclopropylméthyle, propyle, cyclopentyle, prop-2-èn-1-yle, butyle, hexyle, isopropyle, 2-hydroxyéthyle, pyridyle, isoxazolylméthyle, thiazolylméthyle, chloropyridinyle, pyrazinyle, imidazolinyle, benzopyrazidinyle, acétyle, benzoyle, 3,4-dihydroxybenzoyle, 4-nitrobenzoyle, 4-méthoxybenzoyle, 4-carboxybenzoyle, 4-aminobenzoyle, 2-furanoyle, 3,4-dihydroxycinnamoyle, carbamoyle, N-méthyl-carbamoyle et t-butyloxycarbonyle.

11. Procédé selon l'une quelconque des revendications précédentes pour la préparation des composés sélectionnés parmi les sels pharmaceutiquement acceptables et les esters hydrolysables *in vivo* suivants de ces derniers :
[6R,7R]-7-[2-(2-Amino-4-thiazolyl)-2-(Z)-[(R,S)-carboxy(3,4-dihydroxyphényl)méthyloxyimino]acétamido]-3-[1-(méthylamino)pyridinium-4-thiométhyl]céph-3-em-4-carboxylate ;
[6R,7R]-7-[2-(2-Amino-4-thiazolyl)-2-(Z)-[(S)-carboxy(3,4-dihydroxyphényl)méthyloxyimino]acétamido]-3-[1-(méthylamino)pyridinium-4-thiométhyl]céph-3-em-4-carboxylate ;
[6R,7R]-7-[2-(2-Amino-4-thiazolyl)-2-(Z)-[(R)-carboxy(3,4-dihydroxyphényl)méthyloxyimino]acétamido]-3-[1-(méthylamino)pyridinium-4-thiométhyl]céph-3-em-4-carboxylate ;
[6R,7R]-3-[1-Aminopyridinium-4-thiométhyl]-7-[2-(2-amino-4-thiazolyl)-2-(Z)-[(R,S)-carboxy(3,4-dihydroxyphényl)méthyloxyimino]acétamido]céph-3-em-4-carboxylate ;
[6R,7R]-3-[1-Aminopyridinium-4-thiométhyl]-7-[2-(2-amino-4-thiazolyl)-2-(Z)-[(S)-carboxy(3,4-dihydroxyphényl)méthyloxyimino]acétamido]céph-3-em-4-carboxylate ;
[6R,7R]-3-[1-Aminopyridinium-4-thiométhyl]-7-[2-(2-amino-4-thiazolyl)-2-(Z)-[(R)-carboxy(3,4-dihydroxyphényl)méthyloxyimino]acétamido]céph-3-em-4-carboxylate ;
[6R,7R]-7-[2-(2-Amino-4-thiazolyl)-2-(Z)-[3,4-(méthylènedioxy)benzyloxyimino]acétamido]-3-[1-(méthylamino)pyridinium-4-thiométhyl]céph-3-em-4-carboxylate.

12. Procédé selon l'une quelconque des revendications précédentes dans lequel R¹⁷ est le radical chloro ou iodo.

13. Procédé pour la préparation d'une composition pharmaceutique, lequel procédé comprend le mélange d'un composé pharmaceutiquement acceptable, comme défini auparavant dans l'une quelconque des revendications 1 à 11, à un excipient ou à un agent vecteur pharmaceutiquement acceptable.

14. Procédé selon la revendication 13, comprenant en outre le mélange d'un agent inhibiteur de la β-lactamase.

15. Composé de formule (Ia), comme défini dans la revendication 2, ou un sel pharmaceutiquement acceptable, ou un ester hydrolysable *in vivo* de ce dernier, en vue de l'utilisation dans la fabrication d'un médicament pour le traitement des infections bactériennes.
